# FASCICULE DE BREVET EUROPEEN

(11) **EP 0 760 811 B1**
(45) Date de publication et mention de la délivrance du brevet: **18.11.2009**
(21) Numéro de dépôt: 96908172.8
(22) Date de dépôt: 21.03.1996
(51) Int. Cl.: C07D 233/64, C07D 409/12, C07D 401/12, C07D 405/12, C07D 411/12, C07D 413/12, A61K 31/415

(54) **DERIVES D'IMIDAZOLE MODULATEURS DU RECEPTEUR H3 DE L'HISTAMINE**
IMIDAZOLDERIVATE ALS HISTAMIN-H3-MODULATOREN
IMIDAZOLE DERIVATIVES AS HISTAMINE RECEPTOR H3 MODULATORS

(30) Priorité: 21.03.1995 FR 9503267
(43) Date de publication de la demande: 12.03.1997
(73) Titulaire: INSTITUT NATIONAL DE LA SANTE ET DE LA RECHERCHE MEDICALE (INSERM), 75654 Paris Cédex 13 (FR); SOCIETE CIVILE BIOPROJET, F-75003 Paris (FR)
(72) Inventeur: SCHWARTZ, Jean-Charles, F-75014 Paris (FR); ARRANG, Jean-Michel, F-91190 Gif-sur-Yvette (FR); GARBARG, Monique, F-75017 Paris (FR); QUEMENER, Agnès, F-75013 Paris (FR); LECOMTE, Jeanne-Marie, F-75003 Paris (FR); LIGNEAU, Xavier, F-75013 Paris (FR); SCHUNACK, Walter, G., D-14129 Berlin (DE); STARK, Holger, D-12157 Berlin (DE); PURAND, Katja, D-12249 Berlin (DE); HÜLS, Annette, D-12163 Berlin (DE); REIDEMEISTER, Sybille, D-12205 Berlin (DE); ATHMANI, Salah, Glasgow GY3 2PS (GB); GANELLIN, Charon, Robin, Welwyn, Hertfordshire AL6 0TD (GB); PELLOUX-LEON, Nadia, F-38240 Meylan (FR); TERTIUX, Wasyl, Hertfordshire AL7 4RZ (GB); KRAUSE, Michael, C., O., D-10717 Berlin (DE); BASSEM, Sadek, D-12167 Berlin (DE)
(74) Mandataire: Bernasconi, Jean Raymond
(86) Numéro de dépôt international: PCT/FR1996/000432
(87) Numéro de publication internationale: WO 1996/029315

(56) Documents cités:
- EP-A- 0 034 474
- EP-A- 0 058 047
- EP-A- 0 194 984
- WO-A-93/12107
- WO-A-93/12108
- WO-A-93/14070
- WO-A-95/06037
- CHEMICAL ABSTRACTS, vol. 81, no. 15, 14 Octobre 1974 Columbus, Ohio, US; abstract no. 91426e, W. SCHUNACK ET AL.: "Structure-activity relationship of histamine analogs. 4. Ethers ans esters of 4-(2-hydroxyethyl)imidazole and esters of 4-imidazolepropionic acid" page 456; XP002006246 & ARCH. PHARM., vol. 307, no. 7, WEINHEIM, DE, pages 517-23,
- JOURNAL OF PHARMACEUTICAL SCIENCES, vol. 70, no. 3, WASHINGTON DC, US, pages 341-3, XP002006240 J.L. KELLEY ET AL.: "Inhibition of histidine decarboxylase by imidazole derivatives"
- JOURNAL OF THE AMERICAN CHEMICAL SOCIETY, vol. 94, no. 4, 23 Février 1972, WASHINGTON DC, US, pages 1316-1323, XP002006241 T.H. FIFE ET AL.: "Atypical deacylation of tha acyl enzymes formed in the reaction of alpha-chymotrypsin with bis (4-nitrophenyl) carbonate and o-(4-nitrophenyl) carbonate"
- JOURNAL OF HETEROCYCLIC CHEMISTRY, vol. 25, no. 3, PROVO , US, pages 915-6, XP002006242 J. ALTMAN ET AL.: "4(5)-Vinylimidazole by dehydrobromination of 1-triphenylmethyl-4-(2-bromoethyl) imidazole"
- JOURNAL OF THE CHEMICAL SOCIETY, PERKIN TRANSACTIONS 1, LONDON, GB, pages 43-51, XP002006243 A.R. BATTERSBY ET AL.: "Studies of enzyme-mediated reactions. Part 13. Stereochemical course of the formation of histamine by decarboxylation of (2S)-histidine with enzymes from Clostrium welchii and Lactobacillus 30a"
- RECUEIL DES TRAVAUX CHIMIQUES DES PAYS-BAS, vol. 89, no. 11, AMSTERDAM, NL, pages 1181-4, XP002006244 W. BLOEMHOFF ET AL.: "An improved synthesis of 4-imidazoleethanol"
- JOURNAL OF THE AMERICAN CHEMICAL SOCIETY, vol. 71, no. 12, 24 Décembre 1949, WASHINGTON DC, US, pages 3942-4, XP002006245 C.F. HUEBNER ET AL.: "Studies of imidazole compounds. IV. Derivatives of 4-ethylimidazole"
- CHEMICAL ABSTRACTS, vol. 60, no. 7, 30 Mars 1964 Columbus, Ohio, US; abstract no. 8121h, F. SCHNEIDER: "Relation between structure, pK value, and catalytic properties of imidazole derivatives and histidyl peptides" XP002006247 & Z. PHYSIOL. CHEM., vol. 334, pages 26-43,

## Description

La présente invention concerne de nouveaux dérivés de l'imidazole, leur préparation ainsi que leurs applications thérapeutiques en tant que composés chimiques antagonistes, agonistes ou agonistes partiels des récepteurs H₃ de l'histamine.

L'histamine, de façon connue, base aminée dérivant de l'histidine par décarboxylation, provoque chez l'homme et chez l'animal, la contraction des muscles lisses, une hypotension secondaire et des phénomènes analogues au choc anaphylactique (oèdeme, urticaire...).

L'histamine est également un médiateur chimique libéré par certaines cellules histaminergiques.

On comprend donc aisément l'intérêt qui existe, notamment dans le domaine médical, à pouvoir contrôler la libération d'histamine notamment en cas de maladie.

Cet effet peut être obtenu par stimulation des récepteurs H₃ (autorécepteurs présynaptiques) alors que le blocage de ces derniers induit, au contraire, une augmentation de libération d'histamine, notamment au niveau des neurones histaminiques cérébraux (Nature, 1983, 302:832). Par ailleurs, il est apparu plus récemment que les récepteurs H₃ ont également un rôle d'hétérorécepteurs présynaptiques et, qu'à ce titre, ils contrôlent, par exemple, la libération de peptides proinflammatoires dans certains tissus ; leur stimulation par des agonistes H₃ va permettre de réaliser des effets anti-inflammatoires, anti-asthmatiques, anti-migraineux et de lutter contre les glaucomes, les problèmes liés au sommeil, la maladie d'Alzheimer, la schizophrénie, la dépression, l'hypertension, les dysfonctionnements d'ordre sexuel, etc...

Les récepteurs H₃ ont été définis pharmacologiquement en évaluant l'effet de leur stimulation sur la libération d'histamine endogène à partir de coupes de cerveau de rat (Nature 1987, 327:117-123). En outre, d'autres modèles d'études des effecteurs du récepteur H₃ ont été proposés depuis lors (Physiol. Rev. 1991, 71: 1-51) mais tous ces modèles ne permettent pas aisément de mettre en évidence l'effet d'agonistes partiels de faible activité intrinsèque, ces derniers pouvant aisément être pris pour des antagonistes. Or, ces agonistes partiels peuvent être employés comme médicaments pour des indications semblables à celles des agonistes purs et non pour des indications auxquelles sont réservés les antagonistes. Il est donc extrêmement important pour les applications de faire cette distinction à une phase pré-clinique.

Par ailleurs, la demande de brevet WO 93/14070 décrit des composés de l'imidazole présentant des propriétés antagonistes des récepteurs H₃ de l'histamine, contrôlant la libération et la synthèse de l'histamine.

La présente invention se propose de sélectionner, dans la famille des composés de l'imidazole décrite dans ladite demande de brevet WO 93/14070, des groupes de composés ou des composés remarquables par leur activité élevée sur les récepteurs H₃ de l'histamine, tout en étant généralement particulièrement bien adaptés, par leurs. propriétés pharmacologiques, à la préparation de médicaments.

Par ailleurs, les inventeurs de la présente invention ont mis au point un essai biologique sensible permettant de différencier clairement un agoniste présentant une faible activité intrinsèque d'un antagoniste pur. Ils ont, de ce fait, identifié, parmi des composés que leur structure chimique et leur essai traditionnel laissaient prévoir être, des antagonistes, des agonistes partiels à forte activité biologique in vivo. Ils ont aussi synthétisé des dérivés de l'imidazole ayant une activité antagoniste H₃, notamment in vivo, plus forte que celle des composés connus jusqu'alors, ce qui est de nature à diminuer les effets toxiques ou même secondaires aux doses thérapeutiques et à faciliter de cette manière leur emploi clinique.

La présente invention concerne donc des composés chimiques agonistes, agonistes partiels ou antagonistes des récepteurs H₃ de l'histamine répondant à la formule générale : 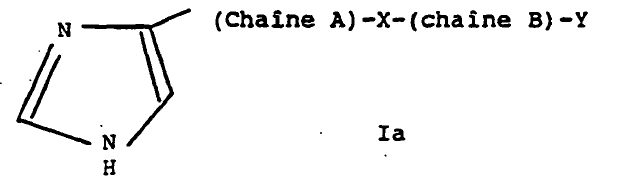 ou 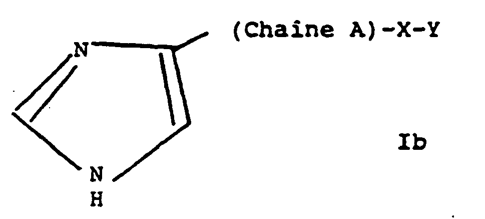 dans laquelle :
la chaîne A représente un groupement alkylène droit ou ramifié comprenant 1 à 8 atomes de carbone ; un groupement alcényle droit ou ramifié comprenant jusqu'à 8 atomes de carbone ; un groupement alcynyle droit ou ramifié comprenant jusqu'à 4 atomes de carbone ;
- le groupement X représente -O- ; -OCONH-, -OCON (alcène) - , -(OCSNH- ; -CH₂- ;
- la chaîne B représente un groupement alkylène droit ou ramifié comprenant 1 à 8 atomes de carbone ; un groupement alcényle comportant jusqu'à 8 atomes de carbone ; un groupement alcynyle comportant jusqu'à 8 atomes de carbone ; un groupement (CH₂)ₙO et n est un entier pouvant varier entre 0 et 4 ;
- le groupement Y représente un groupement phényle, mono ou polysubstitué par un ou plusieurs substituants, identiques ou différents, choisis parmi -OCF₃ , CHO, SO₂N(alkyle)₂, SO₂N(CH₃)₂, S(alkyle) , S(aryle), SCH₂(phényle), un alcène droit ou ramifié comprenant jusqu'à 8 atomes de carbone, un alcyne droit ou ramifié comprenant jusqu'à 8 atomes de carbone éventuellement substitué par un radical trialkylsilyle, O(aryle) -CH₂CN, un aldéhyde, une sulfone, un acétal, un alcool, CH=CH-CHO, -C (alkyle)=N-OH, -C(alkyle)=N-O(alkyle), -CH=NOH, -CH=NO (alkyle) , -C(alkyle)=NH-NH-CONH_{2,} un groupement -O-phényle-OCH₂(phényle) , -C(cycloalkyle)=NOH, -C(cycloalkyle)=N-O(alkyle), un hétérocycle éventuellement, substitué ; un cycle phényle accolé à un carbocycle non aromatique ou un hétérocycle portant une fonction cétone ; un alcyne droit ou ramifié comprenant jusqu'à 8 atomes de carbone et de préférence jusqu'à 5 atomes de carbone ; un alkyle linéaire ou ramifié, mono ou polyphénylique où les groupements phényles sont soit non substitués soit mono ou polysubstitués ; une phénylalkylcétone où le groupement alkyle est ramifié ou non, ou cyclique; un phénylalcool substitué ou non, dont le motif alcool est droit ou ramifié ou cyclique ; un alcène droit ou ramifié comprenant jusqu'à 8 atomes de carbone ; un groupement phénylcycloalkyle ; un groupement fluorenyle, un groupement indanyle ; un groupement phénol ; un groupement diphényle ; un groupement phénoxyphényle ; un groupement benzyloxyphényle ; ainsi que leurs sels pharmaceutiquement acceptables, leurs hydrates, leurs sels hydratés, les structures cristallines polymorphiques et les formes tautomères de ces composés ; et leurs isomères optiques, les mélanges racémiques desdits isomères et les diastéréoisomères correspondants, à l'exception des composés pour lesquels A représente un groupement alcényle droit ou ramifié comprenant jusqu'à à 8 atomes de carbone et X = -CH₂-.
ou dans laquelle :
- la chaîne A représente un groupement alkylène droit ou ramifié comprenant 1 à 8 atomes de carbone ; un groupement alcényle droit ou ramifié comprenant jusqu'à à 8 atomes de carbone ; un groupement, alcynyle droit ou ramifié comprenant, jusqu'à 4 atomes de carbone ;
- le groupement X représente -O- ; -OCONH-, -OCON(alcène)-,-OCSNH- ; -CH₂-;
- la chaîne B représente un groupement, alkylène droit ou ramifié comprenant 1 à 8 atomes de carbone ; un groupement alcényle comportant jusqu'à 8 atomes de carbone ; un groupement alcynyle et n est un entier pouvant varier entre 0 et 4 ;
- le groupement Y représente un groupement, phényle, mono ou polysubstitué par un ou plusieurs substituants, identiques ou différents, choisis parmi -OCF₃, CHO, SO₂N(alkyle)₂, SO₂N(CH₃)₂, S(alkyle), S(aryle), SCH₂ (phényle), un alcène droit ou ramifié comprenant jusqu'à 8 atomes de carbone, un alcyne droit ou ramifié comprenant, jusqu'à 8 atomes de carbone éventuellement susbstitué par un radical trialkylsilyle, O(aryle), -CH₂CN, un aldéhyde, une sulfone, un acétal, un alcool, -CH=CH-CHO, -C (alkyle)=N-OH ,-C (alkyle) =N-O(alkyle), -CH=NOH; -CH=NO(alkyle), -C(alkyle)=NH-NH-CONH₂, un groupement -O-phényle-OCH₂(phényle) , -C(cycloalkyle)=NOH, -C(cycloalkyle)-N-O(alkyle), un hétérocycle éventuellement substitué ; un cycle phényle accolé à un carbocycle non aromatique ou un hétérocycle portant une fonction cétone; un alcyne droit ou ramifié comprenant jusqu'à 8 atomes de carbone et de préférence jusqu'à 5 atomes de carbone ; un alkyle linéaire ou ramifié, mono ou polyphénylique où les groupements phényles sont soit non substitués soit mono ou polysubstitués ; une phénylalkylcétone
ou le groupement alkyle est ramifié ou non, ou cyclique; un phénylalcool substitué ou non, dont le motif alcool est droit ou ramifié ou cyclique ; un alcène droit ou ramifié comprenant jusqu'à 8 atomes de carbone ; un groupement, phénylcycloalkyle ; un groupement fluorenyle ; un groupement indanyle ; un groupement diphényle ; un groupement phénoxyphényle ; un groupement benzyloxyphényle ;
ainsi que leurs sels pharmaceutiquement acceptables, leurs hydrates, leurs sels hydratés, les structures cristallines polymorphiques et les formes tautomères de ces composés ; et leurs isomères optiques, les mélanges racémiques desdits isomères et les diastéréoisomères correspondants.

L'activité antagoniste, agoniste ou agoniste partiel de ces composés peut être facilement vérifiée par des procédés d'essai biologique, notamment ceux définis dans la présente invention.

L'invention a également pour objet, à titre de composés nouveaux, ceux des composés des formules Ia et Ib, non connus de l'art antérieur, y compris de la demande de brevet WO 93/14070.

Selon l'invention, par groupement X représentant une amine, on entend une amine secondaire ou tertiaire.

Les groupements alkyle, alcène, alcyne, cétone, aldéhyde, cycloalkyle, S-alkyle, O-alkyle, phénylalcool et phénylcycloalkyle, ci-dessus mentionnés ainsi que dans le reste de la description et les revendications du présent brevet, comprennent de 1 à 8 atomes de carbone, et de préférence 1 à 5.

De la même façon, on entend par dérivés cétoniques tout groupement oxime, alkyloxime, hydrazone, acetale, aminal, cétal, thione, carbazone, semicarbazone et les analogues thio de ces dérivés.

De même, par groupements phényles et/ou benzophénone mono ou polysubstitués, on entend que ces groupements sont substitués par un ou plusieurs substituants identiques ou différents, choisis parmi les atomes d'halogéne, OCF₃, CHO, CF₃, SO₂N(alkyl)₂, SO₂N(CH₃)₂, NO_{2,} S(alkyl), S(aryl), SCH₂(phényl), un alcène droit ou ramifié, un alcyne droit ou ramifié éventuellement substitué par un radical trialkylsilyle, -O(alkyl),-O(aryl), -CH₂CN, une cétone, un aldéhyde, une sulfone, un acétal, un alcool, un alkyle inférieur, -CH=CH-CHO, - C(alkyl)=N-OH, -C(alkyl)=N-C(alkyl) et autres dérivés cétoniques, -CH=NOH, -CH=NO(alkyl), et autres dérivés aldéhydes. -C(alkyl)=NH-NH-CONH₂, un groupement O-phényl ou -OCH₂(phényl), -C(cycloalkyl)=NOH, -C(cycloalkyl)=N-O(alkyl), un hétérocycle éventuellement substitué;

Le substituant cétone est choisi de préférence parmi une cétone aliphatique à chaîne linéaire ou ramifiée, ladite chaîne pouvant comprendre de 1 à 8 atomes de carbone et porter éventuellement un groupe hydroxy, une cycloalkylcétone, une arylalkylcétone ou arylalkénylcétone ou le groupe aryle est non-substitué ou mono- ou polysubstitué, ou une hétéroarylcétone où le motif hétéroaryle est de préférence monocyclique.

Le substituant acétal consiste de préférence en un acétal aliphatique comprenant de 1 à 8 atomes de carbone et portant éventuellement un radical hydroxy.

Par groupement Y représentant une cétone, on entend notamment une cétone substituée par un groupe alkyle ou aryle, ces groupes pouvant être substitués ou non substitués.

Quant aux hétérocycles, ils comprennent de 1 à 3 hétéroatomes, de préférence des atomes de soufre, d'oxygène ou d'azote.

Le substituant hétérocycle est choisi de préférence parmi un oxadiazole ou un imidazole.

La présente invention concerne également les sels d'addition que forment les composés de formule Ia et Ib avec des acides pharmaceutiquement acceptables. Les sels pharmaceutiquement acceptables comprennent les sels non toxiques d'acides minéraux ou organiques tels que le chlorhydrate, le bromhydrate ou le maléate.

La présente invention englobe également les hydrates des composés de formule Ia ou Ib, les sels hydratés de ces composés et les structures cristallines polymorphiques. Il faut, par ailleurs, noter que la structure des composés conformes à l'invention, telle qu'elle est illustrée par les formules Ia et Ib ne représente que l'une des formes tautomères possibles de ces composés et que ceux-ci peuvent se présenter sous d'autres formes tautomères. La présente invention englobe donc également toutes les formes tautomères possibles des composés en question, que ces tautomères se présentent sous forme isolée ou sous forme de mélanges.

Les composés de formule Ia ou Ib peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisoméres et/ou des isomères optiques peut s'effectuer selon des méthodes connues en soi.

Des groupes de composés préfères selon l'invention sont formés par des composés selon les modes de réalisation suivants ou chacune de leurs combinaisons :
- X représente un oxygène ;
- A représente -(CH₂)ₙ-, n variant de 1 à 4 ;
- A représente- (CH₂)₃ ;
- A est un groupement -(CH₂)₃ ; X est un groupement O ou OCONH; la chaîne B est un groupement (CH₂)ₙ où n = 0, 2 ou 3 ; et Y est un groupement -CH(phényle)₂.
- A est un groupement (CH₂)ₙ où n est un nombre entier pouvant varier entre 1 et 8 ou CH₂(CH)CH₃- ; X est un groupement O ou OCONH; et Y représente un alkyle ramifié ou droit mono- ou polyphénolique ;
- A est un groupement (CH₂)ₙ, où n = 2, 3 ou 4 ; X est un groupement O ou OCONH; et Y représente un groupement CH(phényle)₂,
ainsi que leurs sels pharmaceutiquement acceptables, leurs hydrates, leurs sels hydratés, les structures cristallines polymorphiques et les formes tautomères de ces composés.

Les composés selon l'invention peuvent être utilisés comme agoniste ou agoniste partiel des récepteurs H₃, permettant de par son action d'inhiber la synthèse et/ou la libération de l'histamine ou d'autres neurotransmetteurs tels que neuropeptides ou noradrénaline dans les tissus humains ou animaux.

Les composés agonistes partiels présentent un intérêt particulier en ce qu'ils assurent une normalisation des transmissions sans activation maximale ou blocage complet du récepteur H₃ comme le font respectivement les agonistes complets et les antagonistes.

Lesdits composés peuvent également être utilisés sous forme de composition pharmaceutique comprenant une quantité thérapeutiquement efficace d'un tel composé chimique (ou de plusieurs combinés ou non), dans un excipient pharmaceutiquement acceptable, destinée à une action agoniste ou agoniste partielle sur lesdits récepteurs de l'histamine.

Parmi ceux-ci, des composés agonistes partiels correspondent notamment aux exemples 1, 3, 5, 6, 7, 8, 10, 11, 12, 13, 14 , 16, 17, 110, 111, 154 et 157 décrits ci-après dans la présente description.

Le composé de l'exemple 6 est particulièrement préféré.

D'autres composés agonistes correspondent notamment aux exemples 154 et 156.

Les composés selon l'invention peuvent être utilisés comme antagoniste des récepteurs H₃ de l'histamine, permettant de par son action de favoriser la synthèse et/ou la libération de l'histanine ou d'autres neurotransmetteurs tels que neuropeptides ou noradrénaline dans les tissus humains ou animaux.

Lesdits composés peuvent également être utilisés sous forme des composition pharmaceutique comprenant une quantité thérapeutiquement efficace d'un tel composé chimique (ou de plusieurs combinés ou non), dans un excipient pharmaceutiquement acceptable, destinée à une action antagoniste sur lesdits récepteurs de l'histamine.

Des composés antagonistes correspondent notamment aux exemples 30, 68, 78, 81, 82, 85, 88, 92, 93, 95, 112, 115, 122, 125, 126, 127, 128, 129, 134, 135, 136, 138, 139, 141, 142,143, 144, 145,146, 147, 149, 160, 163, 166, 174, 175, 176.

Le composé de l'exemple 81 est particulièrement préféré.

Les exemples qui sont donnés dans le tableau I ci-après, à titre non limitatif, illustrent la présente invention.

Les composés des exemples 1 à 20, 110, 111 et 154 à 159 sont des agonistes ou agonistes partiels desdits récepteurs, les exemples 21 à 109, 112 à 153 et 160 à 183 sont des antagonistes desdits récepteurs.

**TABLEAU I**

| Exemple | Chaîne A | x | chaîne B | Y |
|---|---|---|---|---|
| 1 | (CH₂)₃ | OCONH | / | -C(CH₃)₃ |
| 2 | (CH₂)₃ | OCONH | / | 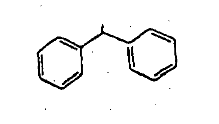 |
| 3 | (CH₂)₃ | OCONH | / | 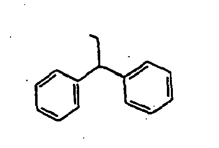 |
| 4 | (CH₂)₂ | OCONH | / | 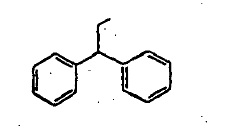 |
| 5 | (CH₂)₃ | O | / | -(CH₂)₂-CH(CH₃)₂ |
| 6 | (CH₂)₃ | O | / | -(CH₂)₂-C(CH₃)₃ |
| 7 | (CH₂)₃ | O | / | -(CH₂)₃-CH(CH₃)₂ |
| 8 | (CH₂)₃ | O | / | 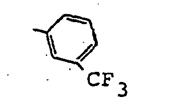 |
| 9 | (CH₂)₃ | O | / | 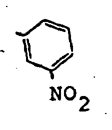 |
| 10 | (CH₂)₂ | S | / | 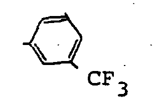 |
| 11 | (CH₂)₃ | O | / | 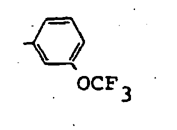 |
| 12 | (CH₂)₃ | O | / | 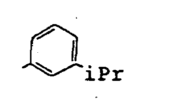 |
| 13 | (CH₂)₃ | O | / | 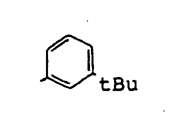 |
| 14 | (CH₂)₃ | O | / | 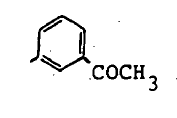 |
| 15 | (CH₂)₃ | O | / | 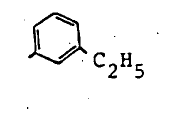 |
| 16 | (CH₂)₄ | O | / | 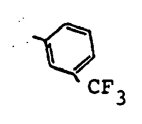 |
| 17 | (CH₂)₄ | O | / | 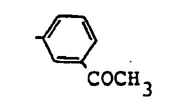 |
| 18 | (CH₂)₃ | O | / | 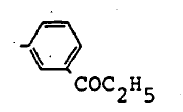 |
| 19 | (CH₂)₃ | O | / | 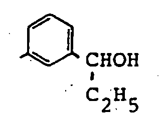 |
| 20 | (CH₂)₃ | O | / | 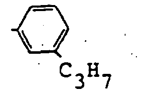 |
| 21 | (CH₂)₃ | O | / | 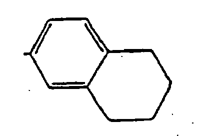 |
| 22 | (CH₂)₃ | O | / | 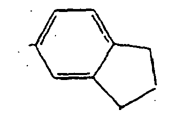 |
| 23 | (CH₂)₃ | O | / | 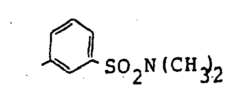 |
| 24 | (CH₂)₃ | O | / | 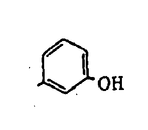 |
| 25 | (CH₂)₃ | O | / | 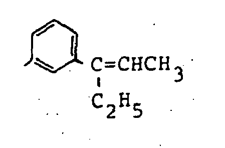 |
| 26 | (CH₂)₃ | O | / | 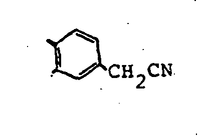 |
| 27 | (CH₂)₃ | O | / | 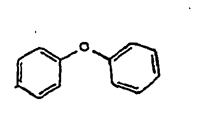 |
| 28 | (CH₂)₃ | OCONH | / | -(CH₂)₂CH₃ |
| 29 | (CH₂)₃ | OCONH | / | -(CH₂)₃CH₃ |
| 30 | (CH₂)₃ | OCONH | / | -(CH₂)₄CH₃ |
| 31 | (CH₂)₃ | OCONH | / | -(CH₂)₅CH₃ |
| 32 | (CH₂)₃ | OCONH | / | -(CH₂)₆CH₃ |
| 33 | (CH₂)₃ | OCONH | / | -(CH₂)₇CH₃ |
| 34 | (CH₂)₃ | OCONH | / | 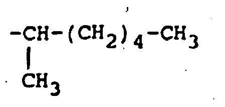 |
| 35 | (CH₂)₃ | OCONH | / | 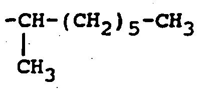 |
| 36 | (CH₂)₃ | OCONH | / | -(CH₂)₂CH(CH₃)₂ |
| 37 | (CH₂)₃ | OCONH | / | 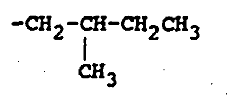 |
| 38 | (CH₂)₃ | OCONH | / | 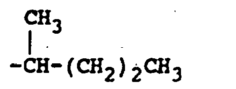 |
| 39 | (CH₂)₃ | 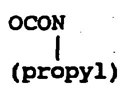 | / | -(CH₂)₂CH₃ |
| 40 | (CH₂)₃ | 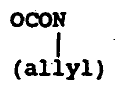 | / | -CH₂-CH=CH₂ |
| 41 | (CH₂)₃ | OCO | / | 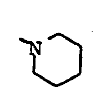 |
| 42 | (CH₂)₃ | OCONH | / | 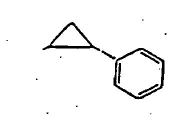 |
| 43 | (CH₂)₂ | OCONH | CH₂ | 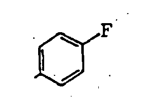 |
| 44 | (CH₂)₃ | OCONH | CH₂-CH-CH₃ | 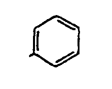 |
| 45 | (CH₂)₃ | OCONH | CH₂ | 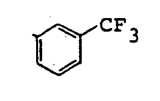 |
| 46 | (CH₂)₃ | OCONH | / | 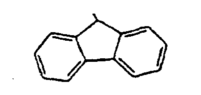 |
| 47 | (CH₂)₃ | OCONH | / | 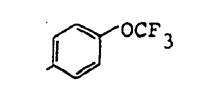 |
| 48 | (CH₂)₃ | OCONH | CH₂ | 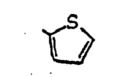 |
| 49 | (CH₂)₃ | OCSNH | / | 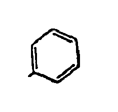 |
| 50 | (CH₂)₂ | CO | / | 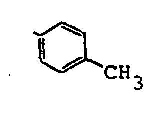 |
| 51 | CH=CH-CH₂ | CH₂ | / | 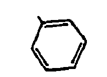 |
| 52 | (CH₂)₂ | CH₂ | CH₂ | 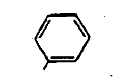 |
| 53 | CH₂ | O | CH₂ | 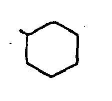 |
| 54 | CH₂ | O | CH₂ | 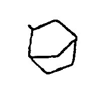 |
| 55 | (CH₂)₃ | O | (CH₂)₃ | 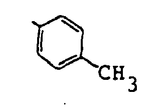 |
| 56 | (CH₂)₃ | O | CH₂ | 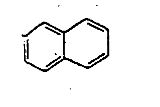 |
| 57 | (CH₂)₃ | O | CH₂ | 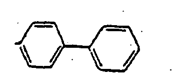 |
| 58 | (CH₂)₃ | O | (CH₂)₃ | 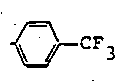 |
| 59 | (CH₂)₃ | O | CH₂ | 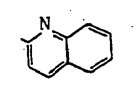 |
| 60 | (CH₂)₃ | O | (CH₂)₃ | 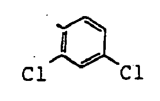 |
| 61 | (CH₂)₃ | O | (CH₂)₂ | 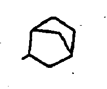 |
| 62 | (CH₂)₃ | O | (CH₂)₃ | 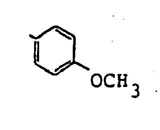 |
| 63 | (CH₂)₃ | O | (CH₂)₂ | 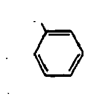 |
| 64 | (CH₂)₃ | O | / | (CH₂)₆-CH₃ |
| 65 | (CH₂)₃ | O | / | CH₂-CH(CH₃)₂ |
| 66 | (CH₂)₃ | O | (CH₂)₂ | 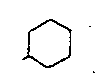 |
| 67 | (CH₂)₃ | O | / | (CH₂)₃-C≡CH |
| 68 | (CH₂)₃ | O | (CH₂)₂O | 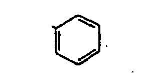 |
| 69 | (CH₂)₃ | O | CH₂ | 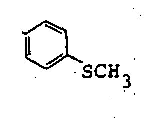 |
| 70 | CH=CHCH₂ | O | (CH₂)₃ | 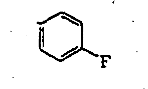 |
| 71 | (CHN₂)₃ | O | / | 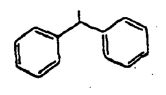 |
| 72 | (CH₂)₃ | O | / | 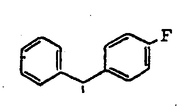 |
| 73 | (CHN₂)₃ | O | / | 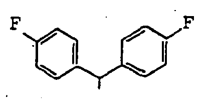 |
| 74 | (CH₂)₃ | OCH₂CO | / | 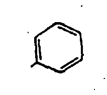 |
| 75 | (CH₂)₃ | OCH₂CO | / | 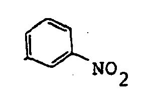 |
| 76 | (CH₂)₃ | O | / | 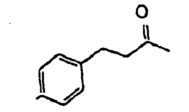 |
| 77 | (CH₂)₃ | O | / | 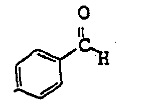 |
| 78 | (CH₂)₃ | O | / | 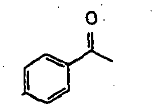 |
| 79 | (CH₂)₃ | O | / | 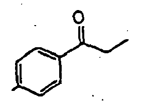 |
| 80 | (CH₂)₃ | O | / | 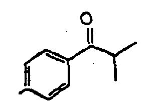 |
| 81 | (CH₂)₃ | O | / | 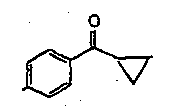 |
| 82 | (CH₂)₃ | O | / | 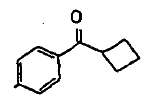 |
| 83 | (CH₂)₃ | O | / | 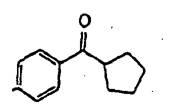 |
| 84 | (CH₂)₃ | O | / | 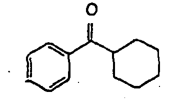 |
| 85 | (CH₂)₃ | O | / | 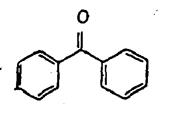 |
| 86 | (CH₂)₃ | O | / | 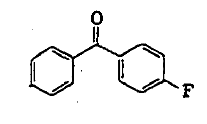 |
| 87 | (CH₂)₃ | O | / | 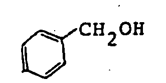 |
| 88 | (CH₂)₃ | O | / | 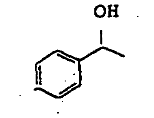 |
| 89 | (CH₂)₃ | O | / | 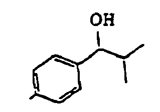 |
| 90 | (CH₂)₃ | O | / | 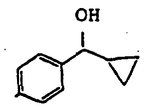 |
| 91 | (CH₂)₃ | O | / | 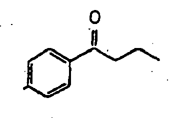 |
| 92 | (CH₂)₃ | O | / | 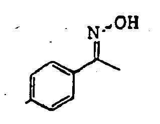 |
| 93 | (CH₂)₃ | O | / | 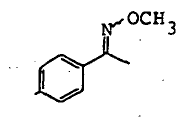 |
| 94 | (CH₂)₃ | O | / | 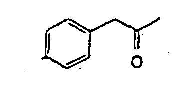 |
| 95 | (CH₂)₃ | O | / | 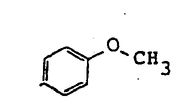 |
| 96 | (CH₂)₃ | OCONH | / | 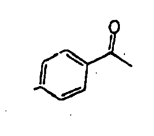 |
| 97 | (CH₂)₃ | OCONH | / | 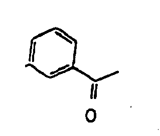 |
| 98 | (CH₂)₃ | O | / | -(CH₂)₂COCH₃ |
| 99 | (CH₂)₃ | O | (CH₂)₂ | CH₃ |
| 100 | (CH₂)₃ | O | (CH₂)₃ | 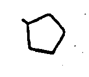 |
| 101 | (CH₂)₃ | OCONH | / | -CH(CH₃)₂ |
| 102 | (CH₂)₃ | OCONH | / | 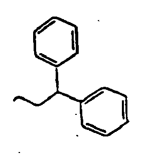 |
| 103 | (CH₂)₂ | CH₂ | (CH₂)₅ | CH₃ |
| 104 | (CH₂)₃ | O | / | 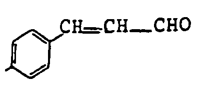 |
| 105 | (CH₂)₃ | O | / | 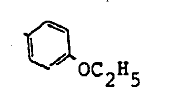 |
| 106 | (CH₂)₂ | O | / | 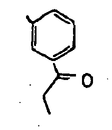 |
| 107 | (CH₂)₃ | O | / | 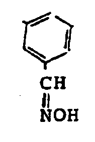 |
| 108 | (CH₂)₃ | O | / | 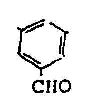 |
| 109 | (CH₂)₃ | O | / | 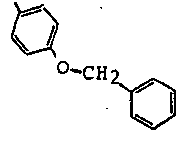 |
| 110 | (CH₂)₃ | OCONH | / | -C(CH₃)₂-C₂H₅ |
| 111 | (CH₂)₃ | OCONH | CH₂ | -C(CH₃)₃ |
| 112 | (CH₂)₃ | OCONH | CH₂ | -CH=CH₂ |
| 113 | (CH₂)₃ | OCONH | / | 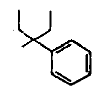 |
| 114 | (CH₂)₃ | OCONH | / | 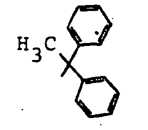 |
| 115 | (CH₂)₃ | OCONH | / | 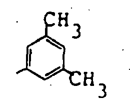 |
| 116 | (CH₂)₂ | O | / | -C(CH₃)₃ |
| 117 | (CH₂)₃ | O | / | -C(CH₉)₃ |
| 118 | (CH₂)₃ | O | CH₂ | -CH=CH₂ |
| 119 | (CH₂)₃ | O | (CH₂)₃ | -CH=CH₂ |
| 120 | (CH₂)₃ | O | CH₂ | -C-CH |
| 121 | (CH₂)₃ | O | / | 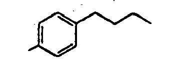 |
| 122 | (CH₂)₃ | O | / | 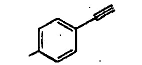 |
| 123 | (CH₂)₃ | O | / | 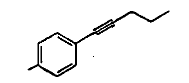 |
| 124 | (CH₂)₃ | O | / | 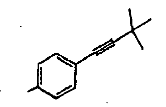 |
| 125 | (CH₂)₃ | O | (CH₂)₃ | 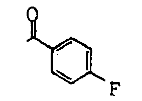 |
| 126 | (CH₂)₂ | O | / | 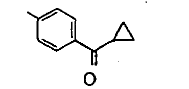 |
| 127 | (CH₂)₃ | O | / | 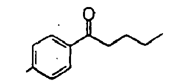 |
| 128 | (CH₂)₃ | O | / | 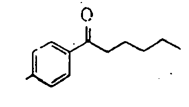 |
| 129 | (CH₂)₃ | O | / | 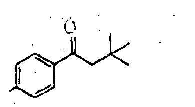 |
| 130 | (CH₂)₃ | O | / | 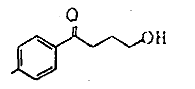 |
| 131 | (CH₂)₃ | O | / | 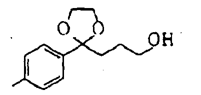 |
| 132 | (CH₂)₃ | O | / | 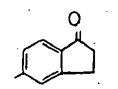 |
| 133 | (CH₂)₃ | O | / | 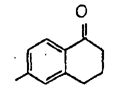 |
| 134 | (CH₂)₃ | O | / | 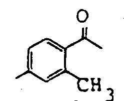 |
| 135 | (CH₂)₃ | O | / | 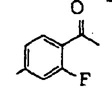 |
| 136 | (CH₂)₃ | O | / | 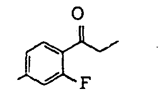 |
| 137 | (CH₂)₃ | O | / | 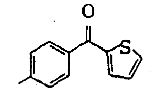 |
| 138 | (CH₂)₃ | O | / | 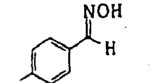 |
| 139 | (CH₂)₃ | O | / | 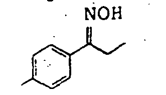 |
| 140 | (CH₂)₃ | O | / | 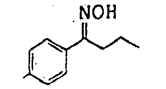 |
| 141 | (CH₂)₃ | O | / | 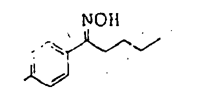 |
| 142 | (CH₂)₃ | O | / | 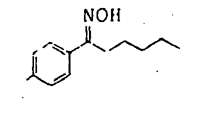 |
| 143 | (CH₂)₃ | O | / | 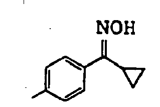 |
| 144 | (CH₂)₃ | O | / | 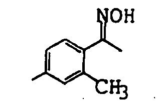 |
| 145 | (CH₂)₃ | O | / | 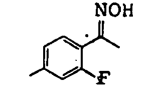 |
| 146 | (CH₂)₃ | O | / | 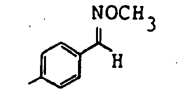 |
| 147 | (CH₂)₃ | O | / | 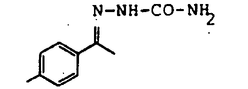 |
| 148 | (CH₂)₃ | O | / | 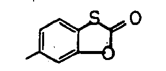 |
| 149 | (CH₂)₃ | O | / | 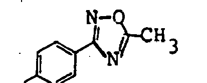 |
| 150 | (CH₂)₃ | O | / | 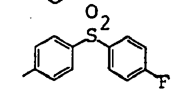 |
| 151 | (CH₂)₃ | O | / | 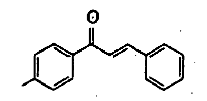 |
| 152 | (CH₂)₃ | O | / | 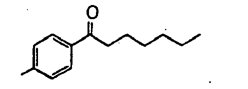 |
| 153 | (CH₂)₃ | O | / | 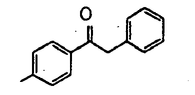 |

| Exemple | Chaîne A | X | Chaîne B | 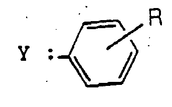 |
|---|---|---|---|---|
| | | | | avec R: |
| 154 | (CH₂)₃ | NH | / | 3-CF₃ |
| 155 | (CH₂)₃ | NH | / | 3-COCH₃ |
| 156 | (CH₂)₃ | NH | / | 3-C₂H₅ |
| 157 | (CH₂)₂ | S | / | 3-COCH₃ |
| 158 | (CH₂)₃ | O | / | 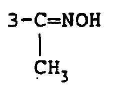 |
| 159 | 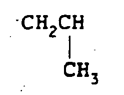 | S | / | 3-CF₃ |
| 160 | (CH₂)₂ | O | / | 4-CH₃ |
| 161 | (CH₂)₂ | O | / | 4-COC₂H₅ |
| 162 | (CH₂)₃ | O | / | 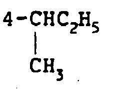 |
| 163 | (CH₂)₃ | O | / | 4-C₂H₅ |
| 164 | (CH₂)₃ | O | / | 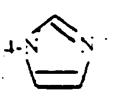 |
| 165 | (CH₂)₃ | O | / | 4-SO₂N(CH₃)₂ |
| 166 | (CH₂)₃ | O | / | 4-SCH₃ |
| 167 | (CH₂)₃ | O | / | 4-SCH₂Ph |
| 168 | (CH₂)₃ | S | / | 3-COCH₃ |
| 169 | (CH₂)₃ | NH | / | 4-C₂H₅ |
| 170 | (CH₂)₃ | NH | / | 4-Cl |

| Exemple | Chaîne A | X | Chaîne B | Y |
|---|---|---|---|---|
| 171 | (CH₂)₃ | O | / | 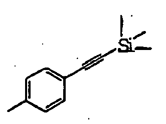 |
| 172 | (CH₂)₃ | O | / | 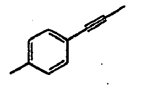 |
| 173 | (CH₂)₃ | O | / | 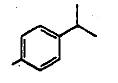 |
| 174 | (CH₂)₃ | O | / | CH₃ |
| 175 | (CH₂)₃ | O | CH₂ | CH₃ |
| 176 | (CH₂)₃ | O | (CH₂)₂ | CH₃ |
| 177 | (CH₂)₃ | O | / | 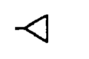 |
| 178 | (CH₂)₃ | O | CH₂ | 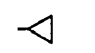 |
| 179 | (CH₂)₃ | CH₂ | (CH₂)₂ | 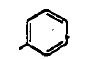 |
| 180 | (CH₂)₃ | OCONH | / | 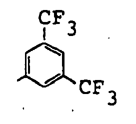 |
| 181 | (CH₂)₃ | OCONH | CHC(CH₃)₂-CH₂ | 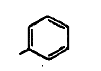 |
| 182 | (CH₂)₃ | OCONH | CH | (C₂H₅)₂ |
| 183 | (CH₂)₂ | CH=CH | (CH₂)₂ | 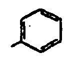 |

Les différentes voies ou méthode de synthèse des composés du tableau I sont détaillées ci-après :

### * Méthodes de synthèse des composés ayant un élément structurel:

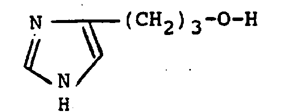

### - Synthèse du 1-(triphénylméthyl)-4-(3-hydroxypropyl)-imidazole

Dans une fiole réactionnelle équipée pour une hydrogénation 10 g d'acide urocanique (72,4 mmol) sont dissouts dans 200 ml d'eau. 1 g de Pd/C (10 %) sont ajoutés et l'hydrogénation est effectuée à 50°C pendant 4 heures. Le catalyseur est filtré et l'eau évaporée, donnant l'acide 3-(imidazol-4-yl) propionique qui apparaît sous forme de poudre blanche (8,8 g ; 86 %) ; P.F. : 209-211°C.

L'acide (3-imidazol-4-yl)propionique (6g, 42 mmol) est dissout dans de l'éthanol absolu (204 ml) et une quantité catalytique d'acide sulfurique concentré (2 ml) est ajoutée. Le mélange en résultant est chauffé au reflux pendant 16 heures. Le solvant est évaporé, ce qui donne un résidu huileux qui est dissout dans 45 ml d'eau. La solution est neutralisée par du carbonate de sodium hydrogène, et le 4-(3-carboéthoxypropyl)-1H-imidazole est extrait avec de l'acétate d'éthyle ; on obtient une huile (5,1 g ; 72 %). Le 4-(3-carboéthoxypropyl)-1H-imidazole (5,4 g, 32 mmol) est dissout dans 4 ml de diméthylformamide anhydre. 3,4 g de triéthylamine (33,6 mmol) et 9,3 g de chlorure de triphényle méthyle (33,6 mmol) sont ajoutés et le mélange est agité à température ambiante, sous azote, pendant 4 heures. Le mélange est versé sur de la glace pillée (60 g), ce qui donne un précipité blanc qui est recristallisé depuis le diéthyléther (9,2 g ; 70 %) en donnant le 1-(triphénylméthyl)-4-(3-carboéthoxypropyl)-1H-imidazole ; P.F. 134°C.

L'ester précédent (7,5 g ; 18,3 mmol), dissout dans du THF (75 ml) récemment distillé, est ajouté goutte à goutte à une solution de 0,8 g d'hydride de lithium aluminium (21 mmol) dans du THF (45 ml) récemment distillé, au froid et sous-agitation. L'agitation est continue à température ambiante pendant 12 heures, puis l'hydride de lithium aluminium est décomposé par l'ajout goutte à goutte d'une solution saturée de sulfate de sodium. Le complexe en résultant est filtré, le THF est séché sur sulphate de magnésium en donnant une huile qui est réduite dans l'acétate d'éthyle pour donner 1-(triphénylméthyl)-4-(3-hydroxypropyl)-1H-imidazole sous forme de poudre blanche (5,8 g ; 87 %) ; PF : 130°C.

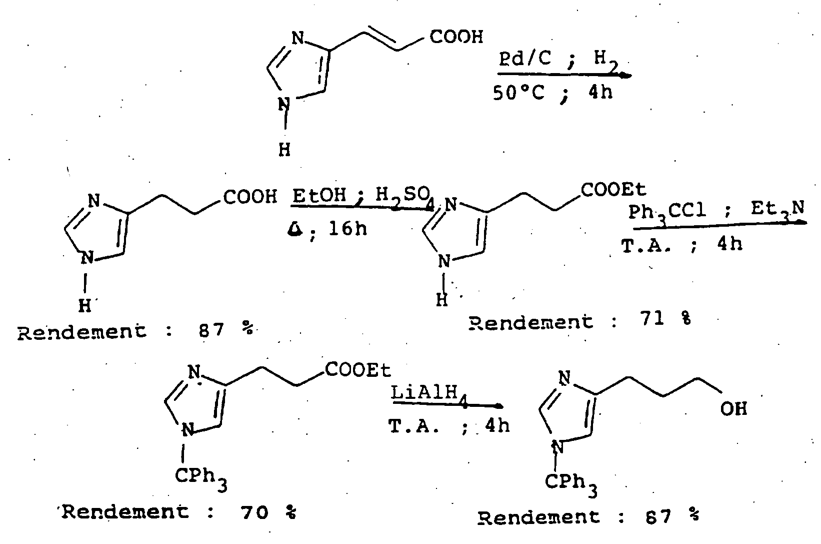

### - Méthode A

### - 4-(3-(3-Trifluorométhylphénoxy)-propyl)-1H-imidazole, oxalate

300 mg de 1-(triphénylméthyl)-4-(3-hydroxypropyl)-1H-imidazole (0,81 mmol) sont dissouts dans 8 ml de THF récemment distillé. On y ajoute 277 mg de triphénylphosphine (1,06 mmol) et 145 mg m-trifluorométhylphénol, et le mélange résultant est refroidi et agité pendant 5 minutes sous azote. Du diéthylazodicarboxylate (184 mg ; 1,06 mmol), dissout dans du THF (4 ml) récemment distillé, est ajouté doucement au mélange réactionnel et sous agitation continue à température ambiante pendant 12 heures. Après élimination du solvant sous vide, une chromatographie en colonne (SiO₂ ; premier éluant : essence de pétrole ; second éluant : essence de pétrole/diéthyléther (50/50) effectuée sur le mélange réactionnel brut donne une poudre blanche qui est réduite dans de l'essence de pétrole pour donner une huile de 1-(triphénylméthyl)-4-[3-(trifluorométhylphénoxy)-propyl]-1H-imidazole. Celle-ci (210 mg ; 0,41 mmol) est chauffée à 70°C pendant 3 heures dans le THF (5 ml) et le HCl 2N (12 ml). Le THF est éliminé sous pression réduite et le Ph₃COH est extrait avec du diéthyléther. La phase acqueuse est neutralisée avec du carbonate de potassium et le produit est extrait dans du diéthyléther ou du chloroforme. Cette solution est séchée et évaporée tout en donnant une huile qui est dissoute dans le 2-propanol. De l'acide oxalique (1,5 équivalents) est ajouté et le produit précité (par addition de diéthyléther) sous forme d'oxalate P.F. : 204-208°C.

Les phénols substitués appropriés à utiliser dans la méthode A sont obtenus dans le commerce à l'exception des composés suivants qui sont synthétisés (mais qui sont des composés connus) :
- 3-propanoylphénol (P.F. : 78-80°C) pour exemple 18 (lit. T. Geoffrey, P. Bruneau, G.C. Crawley, M.P. Edwards, S.J. Foster, J.M. Girodeau, J.F. Kingston et R.M. McMillan, J. Med. Chem. 1991, 34, 2176)
- 3-(1 hydroxypropyl)phénol (P.F. : 106-108) pour exemple 19
   (lit. Geoffrey et al., vide supra)
- 3-propylphénol (P.E. : 125°C à 24 mm Hg) pour exemple 20
   (lit. C.F. Carvalho et M.V. Sargent, J. Chem. Soc. Perk. Trans. 1. 1984, 1621-1627)

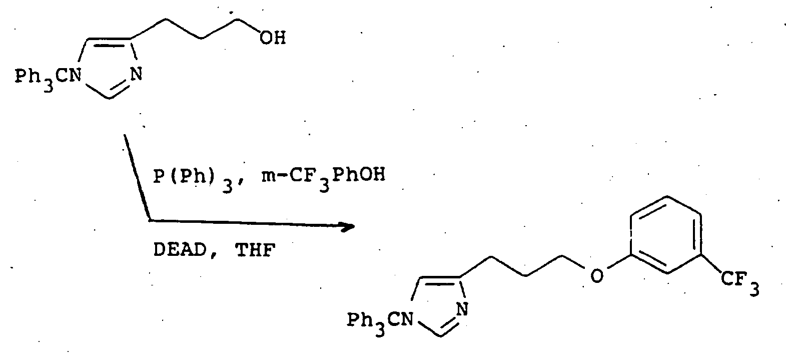

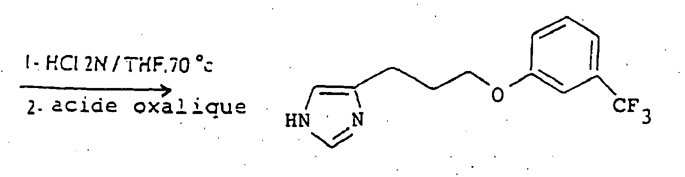

### - Méthode B

### - 4-[2-(3-Trifluorométhylphényl)-thioéthyl]-1H-imidazole, oxalate

A une solution froide de 3-trifluorométhylthiophénol (1,6 ; 8,9 mmol), dans le diméthylformamide, on ajoute doucement 0,18 g (4,5 mmol) d'hydride de sodium (60 % en suspension dans l'huile minérale). Le mélange est agité sous azote à température ambiante pendant 1 heure. 0,15 g de 4-(2-chloroéthyl)-1H-imidazole (0,89 mmol) et 0,010 g de iodure de tétrabutylammonium sont ajoutés et le mélange agité à 80°C pendant 1 jour. Le solvant est évaporé et le résidu huileux est réduit par du diéthyléther puis filtré. Le produit est alors extrait du filtrat avec du HCl dilué. La phase acqueuse est relavée avec du diéthyléther puis basifiée avec du carbonate de potassium, et le produit est extrait avec du chloroforme pour donner une huile qui subit une chromatographie en colonne (SiO₂ ; premier éluant : chloroforme ; second éluant : chloroforme/méthanol (97:3)) puis le produit est converti en un sel d'oxalate ; PF : 158-160°C.

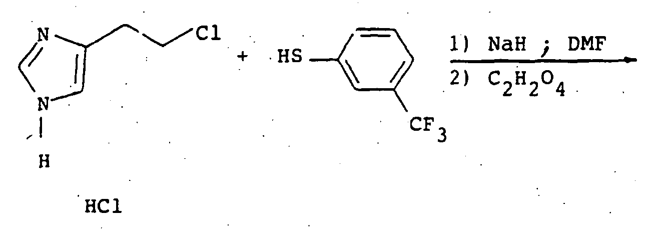

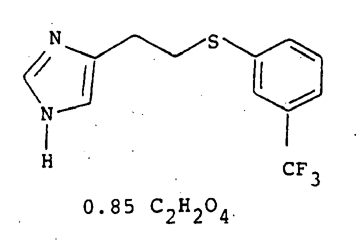

### - Méthode C

### - 4-[4-(3-Ethanoylphénoxy)butyl]-1H-imidazole oxalate

2,87 g de 3-Ethanoylphénol (21 mmol) dans du DMF anhydre (30 ml) est refroidit à 5°C (bain glacé) sous atmosphère argon, puis 0,37 g d'hydride de sodium (60 % en suspension dans l'huile minérale ; 9,25 mmol) sont ajoutés. Le mélange est agité à 5°C pendant 10 min et ensuite à 20°C pendant 2 heures. 0,70 g de 2-(t-butyldiméthylsilyl)-5-(4-chlorobutyl)-1-(diméthylsulfamoyl)-imidazole (1,85 mmol) (synthétisé comme décrit par R.C. Vollinga, W.M.P.B. Menge et H. Timmerman, Rec., Trav., Chem. Pays-Bas, 1993, 112, 123-125) et 45 mg de iodure de tétrabutylammonium sont ajoutés et le mélange est chauffé à 80°C sous argon pendant 3 jours, puis refroidi à 20°C et dilué avec du diéthylether jusqu'à ce que la solution devienne nuageuse. Le solide résultant est filtré et le filtrat évaporé sous vide jusqu'à assèchement en donnant une huile orange foncé. Cette dernière subit une chromatographie en colonne (SiO₂ ; en utilisant un mélange acétate d'éthyle : méthanol en proportion 9 : 1) pour donner le 1-(N,N-diméthylsulphamoyl)-5-[4(3-éthanoylphénoxy)butyl]-imidazole sous forme d'huile jaune.

Cette huile (0,408 g, 1,17 mmol) est chauffée sous reflux dans 30 ml de HCl 2 M pendant 12 heures, puis refroidie, lavée avec du diéthyléther, basifiée avec du carbonate de potassium et extraite (3 x 50 ml) avec du chloroforme. Les extraits chloroformiques réunis sont séchés (MgSO₄) et évaporés en donnant la base libre sous forme d'huile jaune (0,28 g) qui est convertie en sel d'oxalate dans l'éthanol (10 ml) en utilisant 1,5 équivalents molaires d'acide oxalique (dans 10 ml éthanol). Le solide en résultant est collecté, réduit avec EtOH et recristallisé depuis EtOH en donnant le produit désiré. P.F. : 168-170°C.

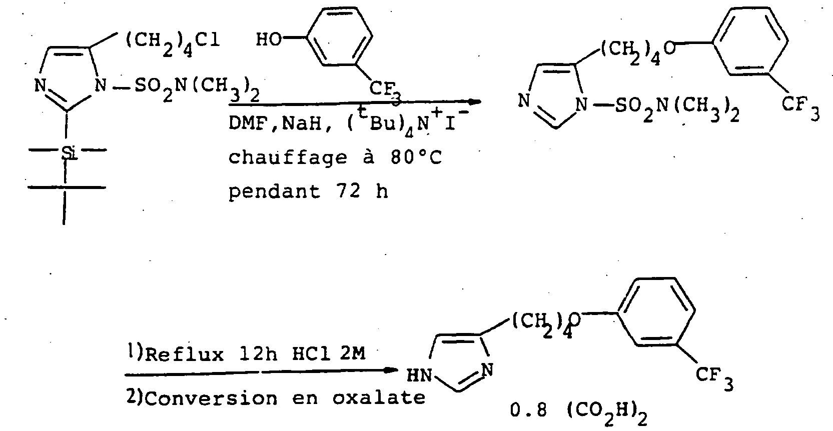

### -Méthode D

### - Préparation du 4-[3-(3-hydroxy-phénoxy)propyl]-1H-imidazole oxalate

Un mélange de monoacétate de résorcinol (0,41 g, 2,71 mmol), de 1-(triphénylméthyl-4(3-hydroxypropyl)-1H-imidazole (1 g ; 2,71 mmol) et de triphénylphosphine (1,06 g, 4,07 mmol, 1,5 équivalents) dans du THF anhydre (30 ml) est refroidi et agité pendant 10 minutes sous azote.

0,71 g de diéthylazodicarboxylate (4,07 mmol, 1,5 équiv.), dissout dans 10 ml THF récemment distillé, est ajouté doucement au mélange réactionnel, et une agitation continue est maintenue pendant 16 heures à 20°C. Après élimination du solvant sous pression réduite, l'huile résultante est soumise à une chromatographie en colonne (SiO₂ ; acetate d'éthyle : hexane, 3:7) en donnant une huile jaune. Cette huile (0,5 g ; 9,96 mmol) dans l'éthanol (15 ml) et 5 % d'hydroxyde de potassium (10 ml) sont chauffés sous reflux pendant 30 min. Le mélange est acidifie avec HCl 2N (refroidi sur bain de glace) et extrait avec du chloroforme : l'extrait organique est séché et le solvant éliminé sous pression réduite en laissant une huile. Cette huile est purifiée par chromatographie en colonne (SiO₂ ; chloroforme) puis dissoute dans 8 ml de THF et chauffée à 80°C pendant 5 heures dans du HCl 2N (12 ml). Après refroidissement, le THF est éliminé sous pression réduite, et Ph₃COH est extrait sous diéthyléther. La phase acqueuse est neutralisée avec une solution de carbonate de potassium et le produit est extrait avec du chloroforme (3 x 100 ml). Les extraits chloroformiques réunis sont séchés (Mg SO₄) et évaporés sous pression réduite pour donner une huile qui est dissoute dans du 2-propanol (2 ml) et convertie en oxalate en utilisant 1,5 équivalent d'acide oxalique et en faisant précipiter le produit par addition de diéthyléther ; P.F. : 138-140°C.

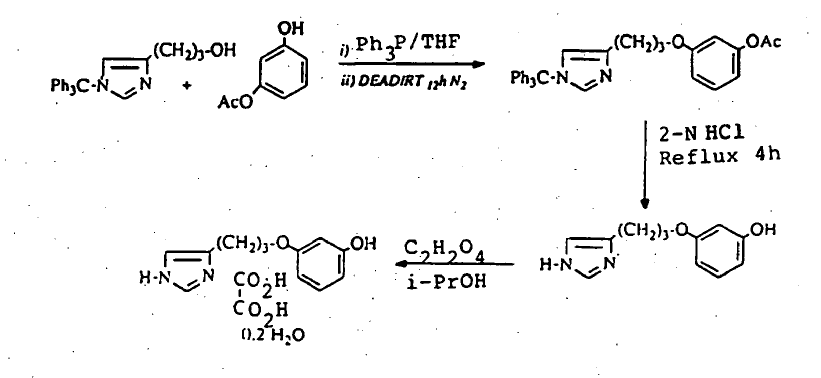

### - Méthode E

### - 4-{3-[3-(Pent-2-ène-3-yl)phénoxy]propyl)-1H-imidazole trifluoroacétate

Le 3-(3-hydroxypentan-3-yl)phénol, PF : 78-80°C est synthétisé comme décrit par M. Satomura dans le brevet japonnais n° 04 82867 A2 (Chem. Abstr., 1992, 117, 1309111).

Un mélange de ce phénol (0,3 g, 1,66 mmol), de 1-(triphénylméthyl)-4-(3-hydroxypropyl)imidazole (0,61 g, 1,66 mmol) et de triphénylphosphine (0,65 g ; 2,5 mmol ; 1,5 équiv.) dans 30 ml de THF anhydre est refroidi et agité pendant 10 min sous azote. 0,57 g de diéthylazodicarboxylate (2,5 mmol ; 1,5 équiv.), dissout dans 10 ml de THF récemment distillé, est ajouté lentement au mélange réactionnel et agité de façon continue à température ambiante pendant 16 heures. Après élimination du solvant sous pression réduite l'huile qui en résulte est purifiée par chromatographie en colonne (SiO₂ ; éluant : éthylacétate : hexane 3 : 7).

L'huile purifiée est dissoute dans du THF (8 ml) et chauffée avec du HCl 2N à 80°C pendant 5 heures.

Après refroidissement, le THF est éliminé sous pression réduite et Ph₃COH est extrait avec du diéthyléther. La phase acqueuse est neutralisée avec une solution de carbonate de potassium et le produit est extrait dans du chloroforme (3 x 100 ml). Les extraits chloroformiques réunis sont séchés et évaporés sous pression réduite pour donner une huile. Cette huile dans 2 ml de 2-propanol est convertie en oxalate et précipitée avec du diéthyléther. Le produit solide est collecté et purifié par HPLC préparative pour donner le sel de trifluoroacétate sous forme de cristaux blancs, P.F. : 169-171°C depuis le 2-propanol.

La RMN ¹H et la HPLC montrent que ce composé contient 2 isomères (E/Z) en proportion 4 : 1.

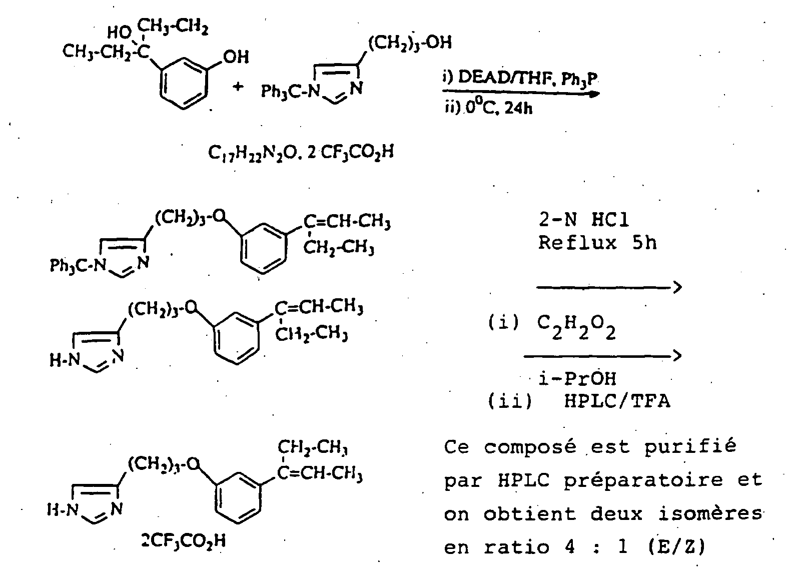

### - Méthode F

A 2 g d'une solution de 2-(tert.-butyldiméthylsilyl)-1-(diméthylsulfamoyl)imidazole (6,92 mmol) dans 20 ml de THF anhydre, refroidie et agitée sous azote à -78°C, on ajoute lentement 6 ml de n-butyllithium 1,5 M dans du THF à -78°C. Puis, on laisse le mélange se réchauffer jusqu'à 0°C. Une solution d'oxyde d'éthylène (3g ; 0,068 mol ; 10 équiv) dans 10 ml de THF anhydre est ajoutée lentement à 0°C, et le mélange est agité pendant une nuit à la température de la pièce. Le mélange réactionnel est versé dans 50 ml d'eau et le THF est évacué sous pression réduite. Le produit est extrait avec du CHCl₃ (3 x 150 ml), séché (Na₂ SO₄) et concentré sous vide en laissant une huile brune. Cette dernière est purifiée par colonne de chromatographie (gel silice) en utilisant du diéthyléther : éther de pétrole 40-60 °C (1 : 1) en tant qu'éluant, en donnant le composé hydroxyéthyle biprotégé (1,2 g ; 52% de rendement).

Une solution de 2-(tert.-butyldiméthylsilyl)-1-(diméthylsulphamoyl)-5-(2-hydroxyéthyl)-imidazole (0,7 g, 2,10 mmol), de triphénylphosphine (0,82 g, 3,15 mmol) et de 3-propanoylphénol (0,32 g, 2,10 mmol) dans 20 ml de THF anhydre est refroidie et agitée sous azote à 0°C. Puis, une solution de diéthylazodicarboxylate (DEAD) (0,55 g, 3,15 mmol) dans 10 ml de THF anhydre est ajoutée goutte à goutte à 0°C et le mélange est agité à 0°C pendant 10 min, puis éliminé sous pression réduite et l'huile résultante est purifiée sur colonne de chromatographie (gel de silice) en utilisant un éluant éthylacétate : hexane (3 : 7) pour donner le 2-(tertbutyldiméthylsilyl)-1-(diméthylsulphamoyl)-5[3-(3-propanoylphénoxyéthyl]-imidazole.

Le composé précédent (0,3 g ; 6,72 x 10⁻⁴) est chauffé à 80°C avec du 2N HCl (12 ml) pendant 4 h. Après refroidissement, le THF est éliminé sous pression réduite. La phase aqueuse est lavée avec du diéthyléther (3 x 100 ml), puis basifiée avec du carbonate de potassium et extraite (3 x) avec du chloroforme. Les extraits chloroformiques combinés sont séchés (Mg SO₄) puis évaporés en donnant une huile qui est dissoute dans du 2-propanol (2 ml) et traitée avec un excès d'acide oxalique (1,5 équiv.) dans du 2-propanol (2 ml). Le produit est précipité par addition de diéthyléther, récolté par filtration et lavé avec du diéthyléther. La cristallisation depuis l'éthanol donne du 4-[2-(3-propanoylphénoxy)éthyl]-1H-imidazole oxale pur. P.F. : 148-150°C.

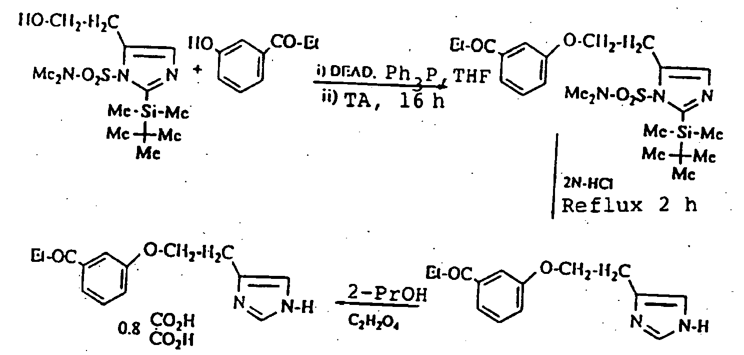

### * Méthode de synthèse des composés ayant un élément structurel :

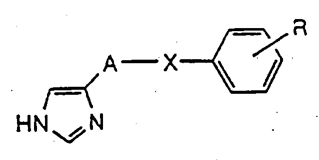

avec A = -(CH₂)₃- et X = -NH-

### - Méthode G

### Oxalate de 4-[3-(3-trifluorométhylphénylamino)propyl]-1H-imidazole

Un mélange de 1 g (2,72 mmol) de 1-(triphénylméthyl)-4-[3-hydroxypropyllimidazole, 0,55 g (4,07 mmol ; 1,5 équivalent) d'oxyde de morpholine et 1,36 g de tamis moléculaire de 4 Å en poudre dans un mélange anhydre d'acétonitrile et de dichlorométhane (10/4) est agité à la température ordinaire sous azote. 0,047 g (0,135 mmol ; 5 % molaires) de perruthénate(VII) de tétrapropylammonium est ajouté en une seule portion et le mélange est agité à la température ordinaire pendant 48 heures. Le mélange réactionnel est filtré sur gel de silice (préchargé avec de l'acétate d'éthyle) et le filtrat est évaporé sous pression réduite. L'huile obtenue est purifiée par chromatographie sur une colonne de gel de silice avec de l'éther diéthylique comme éluant pour fournir le 3-(1-triphényl-méthylimidazol-4-yl)-propionaldéhyde.

0,5 g (1,36 mmol) de l'aldéhyde ci-dessus est chauffé avec 0,22 g (1,36 mmol) de 3-trifluorométhylaniline dans 50 ml de toluène anhydre à 50°C pendant 30 minutes. Le solvant est chassé sous pression réduite pour laisser 0,6 g (86 %) d'une huile qui est dissoute dans le méthanol, refroidie à 0°C, puis traitée avec 1,06 g (0,027 mole ; 20 équivalents) de borohydrure de sodium ajouté lentement à 0°C. Le mélange est agité à la température ordinaire pendant une nuit, puis le solvant est chassé sous pression réduite, 20 ml d'eau sont ajoutés et le mélange est extrait avec du chloroforme. Les extraits chloroformiques sont séchés (MgSO₄) et le solvant est évaporé sous pression réduite pour laisser une huile qui est purifiée par chromatographie sur une colonne de gel de silice (éluant : éther diéthylique) pour fournir 0,4 g de 1-triphénylméthyl-4-(3-(3-trifluorométhylphénylamino)propyl]imidazole sous forme d'une huile incolore. Cette dernière (0,35 g ; 6,85 mmol) dans 8 ml de tétrahydrofuranne et 12 ml d'HCl 2 M est chauffée à 80°C pendant 5 heures. Le tétrahydrofuranne est évaporé sous pression réduite et le Ph₃COH est extrait avec de l'éther diéthylique. La couche aqueuse est neutralisée avec du carbonate de potassium et le produit est extrait dans du chloroforme. La solution chloroformique est séchée et évaporée pour fournir une huile brune qui est purifiée par chromatographie sur une colonne de gel de silice avec comme éluant un mélange d'acétate d'éthyle/méthanol (5/1). L'huile obtenue est dissoute dans 4 ml de 2-propanol, traitée avec une solution de 1,5 équivalent d'acide oxalique dans 3 ml de 2-propanol et le mélange est refroidi pendant 4 heures. Le précipité qui est formé par addition d'éther diéthylique, est recueilli et lavé avec de l'éther pour fournir l'oxalate désiré sous forme d'un solide blanc,P.F. 150-151° C.

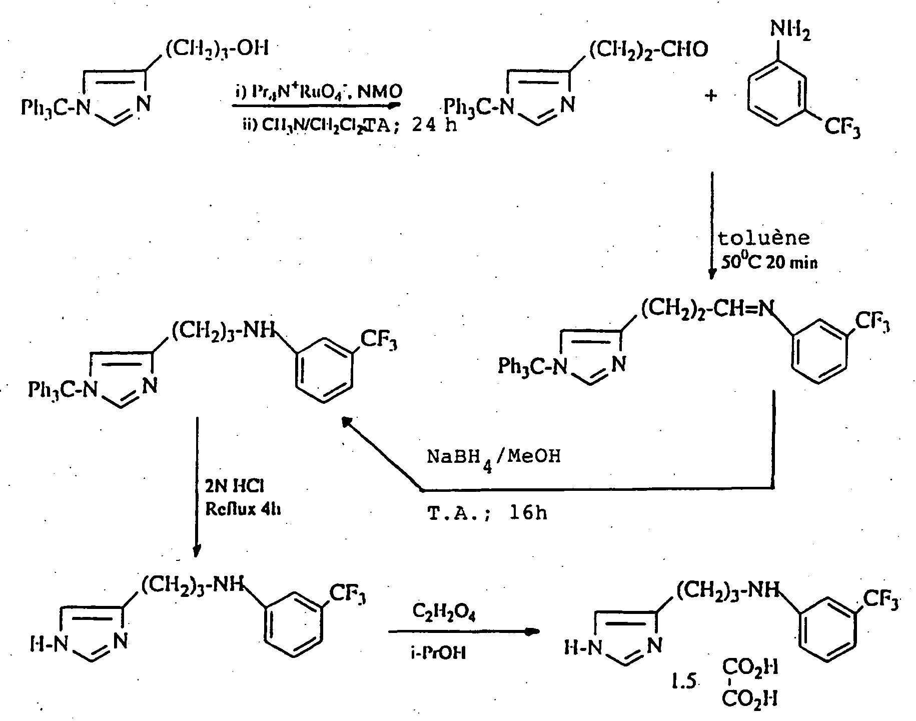

### * Méthode de synthèse des composés ayant un élément structurel :

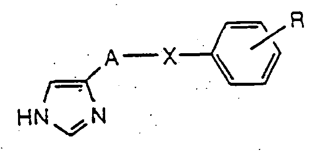

avec A = -CH₂-CH(CH₃)- et X = -S-

### - Méthode H

### Oxalate de 4-[2-(3-trifluorométhylphénylthio)propyl]-1H-imidazole

5,426 g (18,7 mmol) de 1-(N,N-diméthylsulfamoyl)-2-tert-butyldiméthyisilyl-imidazole sont dissous dans 100 ml de THF fraîchement distillé sous azote, refroidis à -78°C et une solution de n-butyllithium dans l'hexane (2,5 M : 15 ml ; 37,5 mmol) est ajoutée goutte à goutte en une période de 10 min. Le mélange est agité pendant 30 min à -78°C. La solution est réchauffée à 0°C avec agitation rapide et une solution de 3,0 ml (2,49 g ; 42,9 mmol) d'oxyde de propylène dans 20 ml de THF fraîchement distillé est ajoutée goutte à goutte en une période de 15 min. Le mélange est agité pendant 18 heures avec chauffage à 20°C, puis le mélange est hydrolysé par addition de 100 ml d'une solution saturée de NH₄Cl. Le THF est éliminé sous pression réduite et le mélange obtenu est extrait trois fois avec 100 ml de dichlorométhane. Les couches organiques sont combinées, séchées (MgSO₄) et évaporées sous pression réduite pour fournir une huile qui est soumise à une chromatographie sur colonne avec de l'éther diéthylique comme éluant pour fournir le 1-(N,N-diméthylsulfamoyl)-2-tert-butyldiméthylsilyl-5-(2-hydroxypropyl)imidazole sous forme d'une huile jaune visqueuse.

L'huile ci-dessus (11,28 g ; 32,5 mmol) est dissoute dans 50 ml de tétrachlorure de carbone anhydre et 9,18 g (35,0 mmol) de triphénylphosphine anhydre dans 50 ml de tétrachlorure de carbone anhydre sont ajoutés. Le mélange est agité sous une atmosphère d'azote à 50°C, puis porté à reflux pendant 16 heures. Le solvant est évaporé sous vide et le solide obtenu est soumis à une chromatographie sur colonne avec du dichlorométhane sur du gel de silice pour fournir le 1-(N,N-diméthylsulfamoyl)-2-tert-butyl-diméthylsilyl-5-(2-chloropropyl)imidazole sous forme d'une huile jaune pâle qui se solidifie, P.F. 51-53°C.

Du 3-trifluorométhyl-thiophénol (0,298 g ; 1,67 mmol) est dissous dans 20 ml de DMF anhydre et refroidi à 0°C sous une atmosphère d'azote et du NaH (dispersion à 60 % dans l'huile minérale ; 0,0393 g ; 1,638 mmol) est ajouté par petites portions. Le mélange réactionnel est agité à 0°C pendant 15 min, puis à 20°C pendant encore 1,5 h et 0,293 g (0,80 mmol) de 1-(N,N-diméthyl-: sulfamoyl)-2-tert-butyldiméthylsilyl-s-(2-chloropropyl)imidazole dissous dans 5 ml de DMF et 10 mg de n-Bu₄ NI sont ajoutés et le mélange est chauffé à 80°C pendant 3 jours. Le solvant est chassé sous pression réduite pour fournir une huile brune qui est traitée avec 100 ml d'eau et extraite 3 fois avec 40 ml de dichlorométhane. Les extraits sont séchés (MgSO₄) et concentrés et l'huile obtenue est soumise à une chromatographie sur colonne en utilisant du white-spirit/acétate d'éthyle 2/1 et 1/1 puis dissoute dans 10 ml d'HCl 2 M et chauffée à 100°C à reflux pendant 3 heures. Le mélange réactionnel est ensuite alcalinisé par addition de NaOH à 10 % (pH environ 11) et est extrait 3 fois avec 40 ml de dichlorométhane. Les extraits sont séchés (MgSO₄) et évaporés pour former une huile limpide qui est soumise à une chromatographie sur colonne avec de l'acétate d'éthyle comme éluant et transformée en-l'oxalate du produit désiré dans le 2-propanol, P.F. 166-168°C.

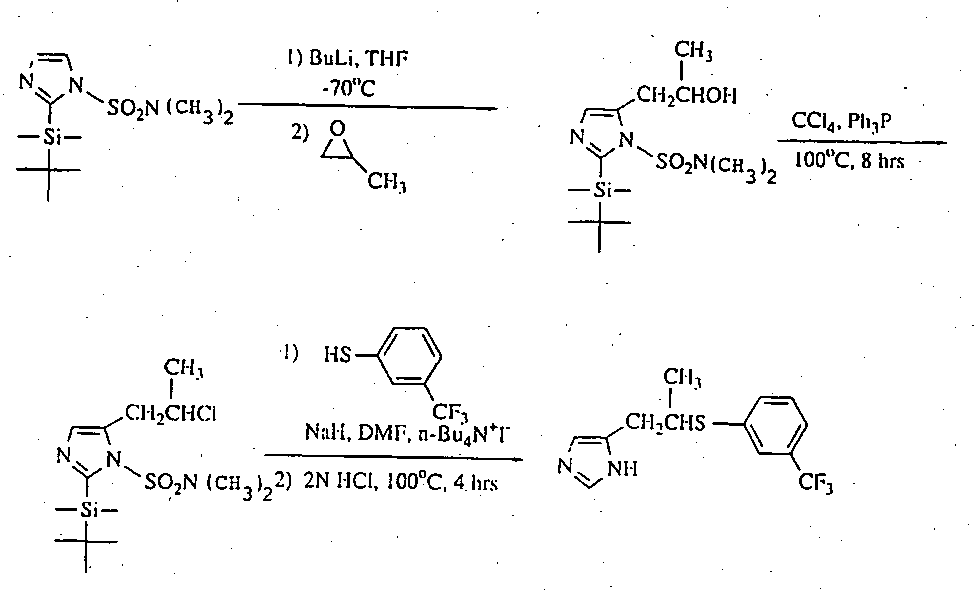

### * Synthèse des composés 1 à 7 et 28 à 183 et récapitulatif pour les composés 8 à 27

### - Exemples de composés selon la présente invention

+ Les composés 1 à 20, 110, 111 et 154 à 159 sont des agonistes ou agonistes partiels.
+ Les composés 21-109, 112-153 et 160-182 sont des antagonistes.

### Exemple 1

### N-t-Butyl-3-(1H-imidazol-4-yl)propyl carbamate

5 mmol de 3-(1H-Imidazol-4-yl)propanol.HCl et de 5 mmol t-butyl isocyanate dans 10 ml d'acétonitrile anhydre sont mis au reflux pendant 1 à 3 h. Le solvant est évaporé et ensuite le résidu purifié par chromatographie rotatoire (éluant : chloroforme/méthanol (99/1- 90/10), atmosphère ammoniaquée). Après élimination du solvant sous pression réduite le résidu est cristallisé en tant que maléate d'hydrogène depuis le diéthyléther et l'éthanol.
FS : C₁₁H₁₉N₃O₂.C₄H₄O₄.O,25H₂O (345.9)

| | | | | | | | |
|---|---|---|---|---|---|---|---|
| Analyse CHN | Calculé | C | 52.1 | H | 6.85 | N | 12.2 |
| | Trouvé | C | 52.0 | H | 6.78 | N | 12.0 |
| Rendement : 40 % | | | | P.F. : 106-107.5°C | | | |

### Exemple 2

### 3-(1H-Imidazol-4-yl)propyl-N-(diphénylméthyl)carbamate

5 mmol de 3-(1H-Imidazol-4-yl)propanol.HCl et de 5 mmol d'isocyanate de diphénylméthyle sont traités comme décrit dans l'exemple 1.
FS : C₂₀H₂₁N₃O₂.C₄H₄O₄.O,25H₂O (456.0)

| | | | | | | | |
|---|---|---|---|---|---|---|---|
| Analyse CHN | Calculé | C | 63.2 | H | 5.64 | N | 9.22 |
| | Trouvé | C | 63.5 | H | 5.64 | N | 9.19 |
| Rendement : 85 % | | | | P.F. : 126-127°C | | | |

### Exemple 3

### 3-(1H-Imidazol-4-yl)propyl-N-(2,2-diphényléthyl)carbamate

5 mmol de 3-(1H-Imidazol-4-yl)propanol.HCl et de 5 mmol d'isocyanate de 2,2-diphényléthyle sont traités comme décrit dans l'exemple 1.
FS : C₂₁H₂₃N₃O₂.C₄H₄O₄ (465.5)

| | | | | | | | |
|---|---|---|---|---|---|---|---|
| Analyse CHN | Calculé | C | 64.5 | H | 5.85 | N | 9.03 |
| | Trouvé | C | 64.5 | H | 5.75 | N | 8.94 |
| Rendement : 60 % | | | | P.F. : 103-104°C | | | |

### Exemple 4

### 2-(1H-Imidazol-4-yl)éthyl-N-(2,2-diphényléthyl)carbamate

5 mmol de 2-(1H-Imidazol-4-yl)éthanol.HCl et de 5 mmol d'isocyanate de 2,2-diphényléthyle sont traités comme décrit dans l'exemple 1.
FS : C₂₀H₂₁N₃O₂.C₄H₄O₄ (451.5)

| | | | | | | | |
|---|---|---|---|---|---|---|---|
| Analyse CHN | Calculé | C | 63.9 | H | 5.58 | N | 9.31 |
| | Trouvé | C | 63.7 | H | 5.77 | N | 9.13 |
| Rendement : 40 % | | | | P.F. : 150-152°c | | | |

### Exemple 5

### 3-(1H-Imidazol-4-yl)propyl-(3-méthylbutyl)éther

5 mmol de 3-(1-Triphénylméthyl-1H-imidazol-4-yl)propanolate de sodium, 5 mmol de bromure de 3-méthylbutane et 0,5 mmol de 15-CROWN-15 (1, 4, 7, 10, 13-pentaoxacyclopentadécane) dissouts dans 10 ml de toluène anhydre sont mis au reflux pendant 24 heures. Le solvant est ensuite évaporé, le résidu dissout dans 10 ml THF et 30 ml HCl 2N puis chauffé à 70°C pendant 2 heures. Le THF est évaporé sous pression réduite et le triphénylméthanol est extrait avec du diéthyléther. La phase acqueuse est neutralisée avec du carbonate de potassium et le produit est extrait avec du diéthyléther. L'extrait organique est séché et évaporé en laissant apparaître une huile qui est cristallisée en tant que maléate d'hydrogène depuis le diéthyléther et l'éthanol.
FS : C₁₁H₂₀N₂O.C₄H₄O₄ (312.4)

| | | | | | | | |
|---|---|---|---|---|---|---|---|
| Analyse CHN | Calculé | C | 57.7 | H | 7.74 | N | 8.97 |
| | Trouvé | C | 57.5 | H | 7.66 | N | 8.89 |
| Rendement : 55 % | | | | P.F. : 74°C | | | |

### Exemple 6

### 3-(1H-Imidazol-4-yl)propyl-(3,3-diméthylbutyl)éther

5 mmol de 3-(1H-Imidazol-4-yl)propanolate de sodium et 5 mmol de chlorure de 3,3-diméthylbutane sont traités comme décrit dans l'exemple 5.
FS : C₁₂H₂₂N₂O.C₄H₄O₄ (326.4)

| | | | | | | | |
|---|---|---|---|---|---|---|---|
| Analyse CHN | Calculé | C | 58.9 | H | 8.03 | N | 8.58 |
| | Trouvé | C | 58.5 | H | 7.80 | N | 8.37 |
| Rendement : 60 % | | | | P.F. : 91°C | | | |

### Exemple 7

### 3-(1H-Imidazol-4-yl)propyl-(4-méthylpentyl)éther

5 mmol de 3-(1H-Imidazol-4-yl)propanolate de sodium et 5 mmol de chlorure de 4-méthylpentane sont traités comme décrit dans l'exemple 5.
FS : C₁₂H₂₂N₂O.C₄H₄O₄ (326.4).

| | | | | | | | |
|---|---|---|---|---|---|---|---|
| Analyse CHN | Calculé | C | 58.9 | H | 8.03 | N | 8.58 |
| | Trouvé | C | 58.8 | H | 7.77 | N | 8.41 |
| Rendement : 50 % | | | | P.F. : 86°C | | | |

### Exemple 8

### 4-[3-(3-Trifluorométhylphénoxy)propyl]-1H-imidazole

On procède comme décrit dans la voie de synthèse A.
Forme saline: OXALATE
Solvant de cristallisation : depuis 2-PrOH par précipitation avec Et₂O.
FS : C₁₃H₁₃F₃N₂O.C₂H₂O₄

| | | | | | | | |
|---|---|---|---|---|---|---|---|
| Analyse CHN | Calculé | C | 50.0 | H | 4.20 | N | 7.78 |
| | Trouvé | C | 50.2 | H | 4.19 | N | 8.26 |
| | | | | P.F. : 204-208°C | | | |

### Exemple 9

### 4-[3-(3-Nitrophénoxy)propyl]-1H-imidazole

On procède comme décrit dans la voie de synthèse A.
Forme saline : OXALATE
Solvant de cristallisation : depuis 2-PrOH par précipitation avec Et₂O.
FS : C₁₂H₁₃N₃O₃.0,9C₂H₂O₄

| | | | | | | | |
|---|---|---|---|---|---|---|---|
| Analyse CHN | Calculé | C | 50.4 | H | 4.54 | N | 12.8 |
| | Trouvé | C | 50.4 | H | 4.43 | N | 12.6 |
| | | | | P.F. : 189-191°C | | | |

### Exemple 10

### 4-[2-(3-Trifluorométhylphénoxy)thioethyl]-1H-imidazole

On procède comme décrit dans la voie de synthèse B.
Forme saline : OXALATE
Solvant de cristallisation : depuis 2-PrOH par précipitation avec Et₂O.
FS : C₁₂H₁₁F₃N₂S.0,85 C₂H₂O₄

| | | | | | | | |
|---|---|---|---|---|---|---|---|
| Analyse CHN | Calculé | C | 47.0 | H | 3.66 | N | 8.01 |
| | Trouvé | C | 47.1 | H | 3.70 | N | 8.35 |
| | | | | P.F. : 158-160°C | | | |

### Exemple 11

### 4-[3-(3-Trifluorométhoxyphénoxy)propyl]-1H-imidazole

On procède comme décrit dans la voie de synthèse A.
Forme saline : OXALATE
Solvant de cristallisation : depuis 2-PrOH par précipitation avec Et₂O.
FS. : C₁₃H₁₃F₃N₂O₂.0,9 C₂H₂O₄

| | | | | | | | |
|---|---|---|---|---|---|---|---|
| Analyse CHN | Calculé | C | 48.4 | H | 4.06 | N | 7.63 |
| | Trouvé | C | 48.5 | H | 4.15 | N | 7.64 |
| | | | | P.F. : 177-179°C | | | |

### Exemple 12

### 4-[3-(3-Isopropylphénoxy)propyl]-1H-imidazole

On procède comme décrit dans la voie de synthèse A.
Forme saline : OXALATE
Solvant de cristallisation : 2-PrOH.
FS : C₁₅H₂₀N₂O.0,8 C₂H₂O₄

| | | | | | | | |
|---|---|---|---|---|---|---|---|
| Analyse CHN | Calculé | C | 63.0 | H | 6.88 | N | 8.85 |
| | Trouvé | C | 63.0 | H | 6.89 | N | 8.86 |
| | | | | P.F. : 180-182°C | | | |

### Exemple 13

### 1-[3-(3-Tert.-butylphénoxy)propyl]-1H-imidazole

On procède comme décrit dans la voie de synthèse A.
Forme saline : OXALATE
Solvant de cristallisation : depuis 2-PrOH par précipitation avec Et₂O.
FS : C₁₆H₂₂N₂O.0,8 C₂H₂O₄

| | | | | | | | |
|---|---|---|---|---|---|---|---|
| Analyse CHN | Calculé | C | 63.9 | H | 7.26 | N | 8.47 |
| | Trouvé | C | 64.2 | H | 7.00 | N | 8.57 |
| | | | | P.F. : 183-185°C | | | |

### Exemple 14

### 4-[3-(3-Ethanoylphénoxy)propyl]-1H-imidazole

On procède comme décrit dans la voie de synthèse A.
Forme saline : OXALATE
Solvant de cristallisation : MeOH.
FS : C₁₄H₁₆N₂O₂.0,8 C₂H₂O₄

| | | | | | | | |
|---|---|---|---|---|---|---|---|
| Analyse CHN | Calculé | C | 59.2 | H | 5.61 | N | 8.86 |
| | Trouvé | C | 59.1 | H | 5.87 | N | 8.80 |
| | | | | P.F. : 156-158°C | | | |

### Exemple 15

### 4-[3-(3-Ethylphénoxy)propyl]-1H-imidazole

On procède comme décrit dans la voie de synthèse A.
Forme saline : OXALATE
Solvant de cristallisation : MeOH.
FS : C₁₄H₁₈N₂O.0,85C₂H₂O₄

| | | | | | | | |
|---|---|---|---|---|---|---|---|
| Analyse CHN | Calculé | C | 61.5 | H | 6.47 | N | 9.13 |
| | Trouvé | C | 61.7 | H | 6.36 | N | 9.39 |
| | | | | P.F. : 173-175°C | | | |

### Exemple 16

### 4-[4-(3-Trifluorométhylphénoxy)butyl]-1H-imidazole

On procède comme décrit dans la voie de synthèse C.
Forme saline : OXALATE
Solvant de cristallisation : EtOH
FS : C₁₄H₁₅F₃N₂O.0,8 C₂H₂O₄

| | | | | | | | |
|---|---|---|---|---|---|---|---|
| Analyse CHN | Calculé | C | 52.6 | H | 4.70 | N | 7.86 |
| | Trouvé | C | 52.5 | H | 4.72 | N | 7.72 |
| | | | | P.F. : 175-176°C | | | |

### Exemple 17

### 4-4-(3-Ethanoylphénoxy)butyl]-1H-imidazole

On procède comme décrit dans la voie de synthèse C.
Forme saline : OXALATE
Solvant de cristallisation : EtOH
FS : C₁₅H₁₈N₂O₂.0,75 C₂H₂O₄

| | | | | | | | |
|---|---|---|---|---|---|---|---|
| Analyse CHN | Calculé | C | 60.8 | H | 6.03 | N | 8.60 |
| | Trouvé | C | 60.7 | H | 6.12 | N | 8.59 |
| | | | | P.F. : 168-170°C | | | |

### Exemple 18

### 4-[3-(3-Propanoylphénoxy)propyl]-1H-imidazole

On procède comme décrit dans la voie de synthèse A.
Forme saline : OXALATE
Solvant de cristallisation : 2-PrOH
FS : C₁₅H₁₈N₂O₂·0,8 C₂H₂O₄

| | | | | | | | |
|---|---|---|---|---|---|---|---|
| Analyse CHN | Calculé | C | 60.4 | H | 5.98 | N | 8.48 |
| | Trouvé | C | 60.6 | H | 5.73 | N | 8.26 |
| | | | | P.F. : 156-158°C | | | |

### Exemple 19

### 4-[3-(3-(1-Hydroxypropyl)phénoxy)propyl]-1H-imidazole

On procède comme décrit dans la voie de synthèse A.
Forme saline : OXALATE
Solvant de cristallisation : 2-PrOH
FS : C₁₅H₂₀N₂O₂.0,8 C₂H₂O₄

| | | | | | | | |
|---|---|---|---|---|---|---|---|
| Analyse CHN | Calculé | C | 60.0 | H | 6.55 | N | 8.43 |
| | Trouvé | C | 60.2 | H | 6.61 | N | 8.49 |
| | | | | P.F. : 141-142°C | | | |

### Exemple 20

### 1-[3-(3-Propylphénoxy)propyl]-1H-imidazole

On procède comme décrit dans la voie de synthèse A.
Forme saline : OXALATE
Solvant de cristallisation : 2-PrOH
FS : C₁₅H₂₀N₂O.1,1 C₂H₂O₄

| | | | | | | | |
|---|---|---|---|---|---|---|---|
| Analyse CHN | Calculé | C | 60.3 | H | 6.03 | N | 9.20 |
| | Trouvé | C | 60.6 | H | 6.15 | N | 9.35 |
| | | | | P.F. : 166-168°C | | | |

### Exemple 21

### 4-[3-(1,2,3,4-Tétrahydronaphth-6-yloxy)propyl]-1H-imidazole

On procède comme décrit dans la voie de synthèse A.
Forme saline : OXALATE
Solvant de cristallisation : MeOH
FS : C₁₆H₂₀N₂O.0,85 C₂H₂O₄

| | | | | | | | |
|---|---|---|---|---|---|---|---|
| Analyse CHN | Calculé | C | 63.9 | H | 6.56 | N | 8.42 |
| | Trouvé | C | 63.9 | H | 6.42 | N | 8.50 |
| | | | | P.F. : 171-173°C | | | |

### Exemple 22

### 4-[3-(Indan-5-yloxy)propyl]-1H-imidazole

On procède comme décrit dans la voie de synthèse A.
Forme saline : OXALATE
Solvant de cristallisation : MeOH
FS : C₁₅H₁₈N₂O.0,8 C₂H₂O₄

| | | | | | | | |
|---|---|---|---|---|---|---|---|
| Analyse CHN | Calculé | C | 63.4 | H | 6.28 | N | 8.91 |
| | Trouvé | C | 63.4 | H | 6.05 | N | 8.89 |
| | | | | P.F. : 188-190°C | | | |

### Exemple 23

### 4-[3-(3-N,N-Diméthylsulphonamidophénoxy)propyl]-1H-imidazole

On procède comme décrit dans la voie de synthèse A.
Forme saline : OXALATE
Solvant de cristallisation : EtOH
FS : C₁₄H₁₉N₃O₃S.C₂H₂O₄

| | | | | | | | |
|---|---|---|---|---|---|---|---|
| Analyse CHN | Calculé | C | 48.1 | H | 5.30 | N | 10.5 |
| | Trouvé | C | 48.3 | H | 4.94 | N | 10.4 |
| | | | | P.F. : 142-144°C | | | |

### Exemple 24

### 4-[3-(3-Hydroxyphénoxy)propyl]-1H-imidazole

On procède comme décrit dans la voie de synthèse D.
Forme saline : OXALATE
Solvant de cristallisation : 2-PrOH
FS : C₁₂H₁₄N₂O₂.C₂H₂O₄.0,2 H₂O

| | | | | | | | |
|---|---|---|---|---|---|---|---|
| Analyse CHN | Calculé | C | 53.9 | H | 5.30 | N | 8.98 |
| | Trouvé | C | 54.0 | H | 5.31 | N | 8.78 |
| | | | | P.F. : 138-140°C | | | |

### Exemple 25

### 4-{3-[3-(Pent-2-ene-3-yl)phénoxy]propyl}-1H-imidazole

On procède comme décrit dans la voie de synthèse E.
Forme saline : Ditrifluoroacétate
Solvant de cristallisation : 2-PrOH
FS : C₁₇H₂₂N₂O.2CF₃CO₂H

| | | | | | | | |
|---|---|---|---|---|---|---|---|
| Analyse CHN | Calculé | C | 50.6 | H | 4.85 | N | 5.62 |
| | Trouvé | C | 50.5 | H | 4.82 | N | 5.40 |
| | | | | P.F. : 169-171°C | | | |

### Exemple 26

### 4-[3-(4-cyanométhylphénoxy)propyl]-1H-imidazole

On procède comme décrit dans la voie de synthèse A.
Forme saline : base
Solvant de cristallisation :
FS : C₁₄H₁₅N₃O.0,5 H₂O

| | | | | | | | |
|---|---|---|---|---|---|---|---|
| Analyse CHN | Calculé | C | 67.2 | H | 6.44 | N | 16.8 |
| | Trouvé | C | 67.4 | H | 6.44 | N | 16.5 |
| | | | | P.F. : 125-127°C | | | |

### Exemple 27

### 4-[3-(4-Phénoxyphénoxy)propyl]-1H-imidazole

On procède comme décrit dans la voie de synthèse A.
Forme saline : OXALATE
Solvant de cristallisation : EtOH
FS : C₁₈H₁₈N₂O₂.0,9 C₂H₂O₄

| | | | | | | | |
|---|---|---|---|---|---|---|---|
| Analyse CHN | Calculé | C | 63.4 | H | 5.32 | N | 7.46 |
| | Trouvé | C | 63.6 | H | 5.35 | N | 7.48 |
| | | | | P.F. : 186-188°C | | | |

### Exemple 28

### 3-(1H-Imidazol-4-yl)propyl-N-propyl carbamate

5 mmol de 3-(1H-Imidazol-4-yl)propanol.HCl et 5 mmol d'isocyanate de propyle sont traités comme décrit dans l'exemple 1.
FS : C₁₀H₁₇N₃O₂.C₄H₄O₄ (327.3)

| | | | | | | | |
|---|---|---|---|---|---|---|---|
| Analyse CHN | Calculé | C | 51.4 | H | 6.47 | N | 12.8 |
| | Trouvé | C | 51.4 | H | 6.55 | N | 12.7 |
| Rendement : 20 % | | | | P.F. : 97-99°C | | | |

### Exemple 29

### N-Butyl-3-(1H-imidazol-4-yl)propyl carbamate

5 mmol de 3-(1H-Imidazol-4-yl)propanol.HCl- et 5 mmol d'isocyanate de butyle sont traités comme décrit dans l'exemple 1.
FS : C₁₁H₁₉N₃O₂.C₄H₄O₄ (341.4)

| | | | | | | | |
|---|---|---|---|---|---|---|---|
| Analyse CHN | Calculé | C | 52.8 | H | 6.79 | N | 12.3 |
| | Trouvé | C | 52.9 | H | 6.78 | N | 12.2 |
| Rendement : 25 % | | | | P.F. : 95-96°C | | | |

### Exemple 30

### 3-(1H-Imidazol-4-yl)propyl-N-pentyl carbamate

5 mmol de 3-(1H-Imidazol-4-yl)propanol.HCl et 5 mmol d'isocyanate de pentyle sont traités comme décrit dans l'exemple 1.
FS : C₁₂H₂₁N₃O₂.C₄H₄O₄.0,25 H₂O (359.9)

| | | | | | | | |
|---|---|---|---|---|---|---|---|
| Analyse CHN | Calculé | C | 53.4 | H | 7.14 | N | 11.7 |
| | Trouvé | C | 53.7 | H | 7.24 | N | 12.0 |
| Rendement : 85 % | | | | P.F. : 91°C | | | |

### Exemple 31

### N-Hexyl-3-(1H-imidazol-4-yl)propyl carbamate

5 mmol de 3-(1H-Imidazol-4-yl)propanol.HCl et 5 mmol d'isocyanate d'hexyle sont traités comme décrit dans l'exemple 1.
FS : C₁₃H₂₃N₃O₂.C₄H₄O₄.0,25 H₂O (373.9)

| | | | | | | | |
|---|---|---|---|---|---|---|---|
| Analyse CHN | Calculé | C | 54.6 | H | 7.41 | N | 11.2 |
| | Trouvé | C | 54.4 | H | 7.70 | N | 11.0 |
| Rendement : 80 % | | | | P.F. : 86°C | | | |

### Exemple 32

### N-Heptyl-3-(1H-imidazol-4-yl)propyl carbamate

5 mmol de 3-(1H-Imidazol-4-yl)propanol.HCl et 5 mmol d'isocyanate d'heptyle sont traités comme décrit dans l'exemple 1.
FS : C₁₄H₂₅N₃O₂.C₄H₄O₄.0,25 H₂O (387.9)

| | | | | | | | |
|---|---|---|---|---|---|---|---|
| Analyse CHN | Calculé | C | 55.7 | H | 7.66 | N | 10.8 |
| | Trouvé | C | 56.0 | H | 7.68 | N | 10.8 |
| Rendement : 40 % | | | | P.F. : 103-104°C | | | |

### Exemple 33

### 3-(1H-Imidazol-4-yl)propyl-N-octyl carbamate

5 mmol de 3-(1H-Imidazol-4-yl)propanol.HCl et 5 mmol d'isocyanate d'octyle sont traités comme décrit dans l'exemple 1.
FS : C₁₅H₂₇N₃O₂.C₄H₄O₄.0,25 H₂O (402.0)

| | | | | | | | |
|---|---|---|---|---|---|---|---|
| Analyse CHN | Calculé | C | 56.8 | H | 7.90 | N | 10.5 |
| | Trouvé | C | 56.8 | H | 7.93 | N | 10.5 |
| Rendement : 40 % | | | | P.F. : 103-104°C | | | |

### Exemple 34

### 3-(1H-Imidazol-4-yl)propyl-N-(2-heptyl) carbamate

5 mmol de 3-(1H-Imidazol-4-yl)propanol.HCl et 5 mmol d'isocyanate de 2-heptyle sont traités comme décrit dans l'exemple 1.
FS: C₁₄H₂₅N₃O₂.C₄H₄O₄ (383.4)

| | | | | | | | |
|---|---|---|---|---|---|---|---|
| Analyse CHN | Calculé | C | 56.4 | H | 7.62 | N | 11.0 |
| | Trouvé | C | 56.2 | H | 7.75 | N | 10.7 |
| Rendement : 65 % | | | | P.F. : 117-119°C | | | |

### Exemple 35

### 3-(1H-Imidazol-4-yl)propyl-N-(2-octyl) carbamate

5 mmol de 3-(1H-Imidazol-4-yl)propanol.HCl et 5 mmol d'isocyanate de 2-octyle sont traités comme décrit dans l'exemple 1.
FS : C₁₅H₂₇N₃O₂.C₄H₄O₄ (397.5)

| | | | | | | | |
|---|---|---|---|---|---|---|---|
| Analyse CHN | Calculé | C | 57.4 | H | 7.86 | N | 10.6 |
| | Trouvé | C | 57.3 | H | 7.72 | N | 10.5 |
| Rendement : 80% | | | | P.F. : 118-119°C | | | |

### Exemple 36

### 3-(1H-Imidazol-4-yl)propyl-N-(3-méthylbutyl) carbamate

5 mmol de 3-(1H-Imidazol-4-yl)propanol.HCl et 5 mmol d'isocyanate de 3-méthylbutyle sont traités comme décrit dans l'exemple 1.
FS : C₁₂H₂₁N₃O₂.C₄H₄O₄.0,25H₂O (359.9)

| | | | | | | | |
|---|---|---|---|---|---|---|---|
| Analyse CHN | Calculé | C | 53.4 | H | 7.14 | N | 11.7 |
| | Trouvé | C | 53.7 | H | 7.17 | N | 11.8 |
| Rendement : 30 % | | | | P.F. : 99-100°C | | | |

### Exemple 37

### 3-(1H-Imidazol-4-yl)propyl-N-(2-méthylbutyl) carbamate

5 mmol de 3-(1H-Imidazol-4-yl)propanol.HCl et 5 mmol d'isocyanate de 2-méthylbutyle sont traités comme décrit dans l'exemple 1.
FS : C₁₂H₂₁N₃O₂.C₄H₄O₄.0,25H₂O (359.9)

| | | | | | | | |
|---|---|---|---|---|---|---|---|
| Analyse CHN | Calculé | C | 53.4 | H | 7.14 | N | 11.7 |
| | Trouvé | C | 53.5 | H | 7.08 | N | 11.4 |
| Rendement : 55.% | | | | P.F. : 98-100,5°C | | | |

### Exemple 38

### 3-(1H-Imidazol-4-yl)propyl-N-(2-pentyl) carbamate

5 mmol de 3-(1H-Imidazol-4-yl)propanol.HCl et 5 mmol d'isocyanate de 2-pentyle sont traités comme décrit dans l'exemple 1.
FS : C₁₂H₂₁N₃O₂.C₄H₄O₄.0,25H₂O (359.9)

| | | | | | | | |
|---|---|---|---|---|---|---|---|
| Analyse CHN | Calculé | C | 53.4 | H | 7.14 | N | 11.7 |
| | Trouvé | C | 53.7 | H | 7.09 | N | 11.6 |
| Rendement : 35 % | | | | P.F. : 114-116°C | | | |

### Exemple 39

### 3-(1H-Imidazol-4-yl)propyl-N,N-dipropyl carbamate

5 mmol de 3-(1H-Imidazol-4-yl)propanol.HCl et 5 mmol de chlorure de N,N-dipropylcarbamoyle sont traités comme décrit dans l'exemple 1 et cristallisés en tant que hydrogénoxalate depuis le diéthyléther et l'éthanol.
FS : C₁₃H₂₃N₃O₂.C₂H₂O₄ (343.4)

| | | | | | | | |
|---|---|---|---|---|---|---|---|
| Analyse CHN | Calculé | C | 52.5 | H | 7.34 | N | 12.2 |
| | Trouvé | C | 52.4 | H | 7.33 | N | 12.2 |
| Rendement : 35 % | | | | P.F. : 145-147°C | | | |

### Exemple 40

### N,N-Diallyl-3-(1H-imidazol-4-yl)propyl carbamate

5 mmol de 3-(1H-Imidazol-4-yl)propanol.HCl et 5 mmol de chlorure de N,N-diallylcarbamoyle sont traités comme décrit dans l'exemple 1.
FS : C₁₃H₁₉N₃0₂.C₄H₄O₄.0,25H₂O (369.9)

| | | | | | | | |
|---|---|---|---|---|---|---|---|
| analyse CHN | Calculé | C | 55.2 | H | 6.40 | N | 11.4 |
| | Trouvé | C | 55.2 | H | 6.13 | N | 11.6 |
| Rendement : 20 % | | | | P.F. : 56-57°C | | | |

### Exemple 41

### N-(3-(1H-Imidazol-4-yl)propyloxycarbonyl)pipéridine

5 mmol de 3-(1H-Imidazol-4-yl)propanol.HCl et 5 mmol de chlorure de pipéridinecarbamoyle sont traités comme décrit dans l'exemple 1.
FS : C₁₂H₁₉N₃O₂.C₄H₄O₄.0,25H₂O (357.9)

| | | | | | | | |
|---|---|---|---|---|---|---|---|
| Analyse CHN | Calculé | C | 53.7 | H | 6.62 | N | 11.7 |
| | Trouvé | C | 54.0 | H | 6.61 | N | 11.7 |
| Rendement : 70 % | | | | P.F. : 97-99°C | | | |

### Exemple 42

### 3-(1H-Imidazol-4-yl)propyl-N-trans-(2-phénylcyclopropyl) carbamate

5 mmol de 3-(1H-Imidazol-4-yl)propanol.HCl et 5 mmol de isocyanate de trans-2-phénylcyclopropyle sont traités comme décrit dans l'exemple 1.
FS : C₁₆H₁₉N₃O₂.C₄H₄O₄.0,25H₂O (405.9)

| | | | | | | | |
|---|---|---|---|---|---|---|---|
| Analyse CHN | Calculé | C | 59.2 | H | 5.84 | N | 10.4 |
| | Trouvé | C | 59.4 | H | 5.96 | N | 10.2 |
| Rendement : 45 % | | | | P.F. : 107-109°C | | | |

### Exemple 43

### N-(4-Fluorophénylméthyl)-2-(1H-imidazol-4-yl)éthyl carbamate

5 mmol de 3-(1H-Imidazol-4-yl)éthanol.HCl et 5 mmol de isocyanate de 4-fluorophénylméthyle sont traités comme décrit dans l'exemple 1.
FS : C₁₃H₁₄N₃O₂F.C₄H₄O₄ (379.3)

| | | | | | | | |
|---|---|---|---|---|---|---|---|
| Analyse CHN | Calculé | C | 53.8 | H | 4.78 | N | 11.1 |
| | Trouvé | C | 53.8 | H | 4.80 | N | 11.0 |
| Rendement : 60 % | | | | P.F. : 139°C | | | |

### Exemple 44

### 3·(1H-Imidazol-4-yl)propyl N-(2-phenylpropyl) carbamate

5 mmol de 3-(1H-imidazol-4-yl)propanol.HCl et 5 mmol d'isocyanate de 2-phénylpropyle (fraîchement préparé à partir de 2-phénylpropylamine et de diphosgène dans l'acétate d'éthyle (Japan Kokai Tokkyo Koho JP. 60, 162, 262 (05.07.1985): Chem. Abstr. 103.215012) sont traités comme décrit à l'exemple 1.
C₁₆H₂₁N₃O₂.C₄H₄O₄.0.25 H₂O (407.9)

| Analyse CHN : | | | | | | | |
|---|---|---|---|---|---|---|---|
| | calculé : | C | 58.9 | H | 6.30 | N | 10.3 |
| | trouvé: | C | 58.6 | H | 6.14 | N | 10.3 |
| Rendement : 40 % | | | | | P.F. : 90° C. | | |

### Exemple 45

### N-(3-(Trifluorométhyl)phénylméthyl)-3-(1H-imidazol-4-yl)propyl carbamate

5 mmol de 3-(1H-Imidazol-4-yl)propanol.HCl et 5 mmol d'isocyanate de 3-(trifluorométhyl)phénylméthyle sont traités comme décrit dans l'exemple 1.
FS : C₁₅H₁₆N₃O₂F₃.C₄H₄O₄ (443.4)

| | | | | | | | |
|---|---|---|---|---|---|---|---|
| Analyse CHN | Calculé | C | 51.5 | H | 4.55 | N | 9.48 |
| | Trouvé | C | 51.6 | H | 4.46 | N | 9.36 |
| Rendement : 20 % | | | | P.F. : 97-98°C | | | |

### Exemple 46

### N-Fluorén-9-yl-3-(1H-imidazol-4-yl)propyl carbamate

5 mmol de 3-(1H-Imidazol-4-yl)propanol.HCl et 5 mmol d'isocyanate de 9-fluorényle sont traités comme décrit dans l'exemple 1.
FS : C₂₀H₁₉N₃O₂.C₄H₄O₄.0,25H₂O (454.0)

| | | | | | | | |
|---|---|---|---|---|---|---|---|
| Analyse CHN | Calculé | C | 63.5 | H | 5.22 | N | 9.26 |
| | Trouvé | C | 63.6 | H | 5.23 | N | 9.38 |
| Rendement : 50 % | | | | P.F. : 174-175°C | | | |

### Exemple 47

### N-(4-(Trifluorométhoxy)phényl)-3-(1H-imidazol-4-yl)propyl carbamate

5 mmol de 3-(1H-Imidazol-4-yl)propanol.HCl et 5 mmol d'isocyanate de 4-(trifluoromethoxy)phényle sont traités comme décrit dans l'exemple 1.
FS : C₁₄H₁₄N₃O₃F₃.C₄H₄O₄.0,25H₂O (449.9)

| | | | | | | | |
|---|---|---|---|---|---|---|---|
| Analyse CHN | Calculé | C | 48.1 | H | 4.14 | N | 9.34 |
| | Trouvé | C | 48.3 | H | 4.19 | N | 9.32 |
| Rendement : 95 % | | | | P.F. : 119-121°C | | | |

### Exemple 48

### 3-(1H-Imidazol-4-yl)propyl-N-(2-thényl) carbamate

5 mmol de 3-(1H-Imidazol-4-yl)propanol.HCl et 5 mmol d'isocyanate de 2-thényle sont traités comme décrit dans l'exemple 1.
FS : C₁₂H₁₅N₃O₂S.C₄H₄O₄.0,25H₂O (385.9)

| | | | | | | | |
|---|---|---|---|---|---|---|---|
| Analyse CHN | Calculé | C | 49.8 | H | 5.04 | N | 10.9 |
| | Trouvé | C | 50.2 | H | 5.09 | N | 10.9 |
| Rendement : 80 % | | | | P.F. : 103-105°C | | | |

### Exemple 49

### 3-(1H-Imidazol-4-yl)propyl-N-phényl thioncarbamate

5 mmol de 3-(1H-Imidazol-4-yl)propanolate de sodium et 5 mmol de thiocyanate de phényle sont traités comme décrit dans l'exemple 1.
FS : C₁₃H₁₅N₃OS.C₄H₄O₄ (377.4)

| | | | | | | | |
|---|---|---|---|---|---|---|---|
| Analyse CHN | Calcule | C | 54.1 | H | 5.07 | N | 11.1 |
| | Trouvé | C | 53.7 | H | 5.12 | N | 11.1 |
| Rendement : 55 % | | | | P.F. : 129-131°C | | | |

### Exemple 50

### 3-(1H-Imidazol-4-yl)-1-(4-méthylphényl)propanone

5 mmol d'ester de méthyle de l'acide. 3-(1-triphénylméthyl-1H-imidazol-4-yl)propanoïque sont dissouts à 0°C dans 10 ml de chlorure de thionyle. Après 1 heure d'agitation à température ambiante, le solvant est évacué sous pression réduite. Le résidu est dissout dans du toluène et ajouté à une solution de 15 mmol de AlCl₃ dans 20 ml de toluène; Après 30 minutes à 0°C, la réaction est mise au reflux pendant 5 heures. Le toluène est évaporé et le résidu est hydrolysé avec de l'eau. Une extraction au diéthyléther suivie d'une concentration donne une huile, qui est chauffée dans 30ml HCl 2N et '10 ml de THF.

Le THF est évaporé sous pression réduite et le triphénylméthanol est extrait au diéthyléther. La phase acqueuse est alcalnisée à l'aide d'ammoniac, le produit brut est extrait avec du diéthyléther et purifié par chromatographie rotatoire. (éluant : chloroforme/méthanol (99/1-90/10), atmosphère ammoniaquée). Après évacuation du solvant sous pression réduite, le produit est recristallisé en tant que maléate d'hydrogène depuis le diéthyléther et l'éthanol.
FS : C₁₃H₁₄N₂.C₄H₄O₄ (330.4)

| | | | | | | | |
|---|---|---|---|---|---|---|---|
| Analyse CHN | Calculé | C | 61.8 | H | 5.49 | N | 8.48 |
| | Trouvé | C | 61.8 | H | 5.52 | N | 8.50 |
| Rendement : 35 % | | | | P.F. : 121°C | | | |

### Exemple 51

### 3-(1H-Imidazol-4-yl)-4-phénylbut-1-ène

10 mmol de bromure de 3-phénylpropyltriphénylphosphonium, 10 mmol de (1-triphénylméthyl-1H-imidazol-4-yl)méthanal (J.L. Kelley, C.A. Miller, E.W. Mc Lean, J. Med. Chem. 1977, 20, 721) et 12 mmol de potassium de t-butanolate sont agités pendant 24 heures dans 50 ml THF. Le solvant est évaporé sous pression réduite, hydrolysé avec de l'eau et extrait à l'aide de chloroforme. L'extrait organique concentré est chauffé du reflux dans 30 ml HCl 2N et 30 ml d'acétone pendant 1 heure. Le solvant est évaporé sous pression réduite et le triphénylméthanol est extrait à l'aide de diéthyléther. La phase acqueuse est alcalinisée à l'aide d'ammoniac, le produit brut est extrait à l'aide de diéthyléther. La phase acqueuse est alcalinisée à l'aide de diéthyléther et cristallisé en tant que maléate d'hydrogène depuis le diéthyléther et l'éthanol.
FS : C₁₃H₁₄N₂.C₄H₄O₄ (314.3)

| | | | | | | | |
|---|---|---|---|---|---|---|---|
| Analyse CHN | Calculé | C | 65.0 | H | 5.77 | N | 8.91 |
| | Trouvé | C | 65.0 | H | 5.74 | N | 8.81 |
| Rendement : 40 % | | | | P.F. : 114°C | | | |

### Exemple 52

### 3-(1H-Imidazol-4-yl)-4-phénylbutane

3 mmol de (1H-Imidazol-4-yl)-4-phénylbut-1-ène (exemple 51) sont dissouts dans 50 ml de méthanol. On ajoute 70 mg Pd/C (10 %) et une réduction est opérée pendant 12 heures à une pression de 10 bar sous hydrogène.

La solution est filtrée et purifiée par chromatographie rotatoire. Le produit est cristallisé en tant que maléate d'hydrogène depuis le diéthyléther et l'éthanol.
FS : C₁₃H₁₆N₂.C₄H₄O₄ (316.4)

| | | | | | | | |
|---|---|---|---|---|---|---|---|
| Analyse CHN | Calculé | C | 64.5 | H | 6.37 | N | 8.86 |
| | Trouvé | C | 64.7 | H | 6.36 | N | 8.73 |
| Rendement : 95 % | | | | P.F. : 128-129°C | | | |

### Exemple 53

### cyclohéxylméthyl-(1H-imidazol-4-yl)méthyl éther

5 mmol de (1-Triphénylméthyl-1H-imidazol-4-yl)méthanolate de sodium et 5 mmol de chlorure de cyclohexylméthane sont traités comme décrit dans l'exemple 5.
FS : C₁₁H₁₈N₂O.C₄H₄O₄.0,5H₂O (319.4)

| | | | | | | | |
|---|---|---|---|---|---|---|---|
| Analyse CHN | Calculé | C | 56.4 | H | 7.26 | N | 8.77 |
| | Trouvé | C | 56.3 | H | 7.19 | N | 8.94 |
| Rendement : 40 % | | | | P.F. : 104°C | | | |

### Exemple 54

### (Bicyclo[2.2.1]hept-2-yl)méthyl-(1H-imidazol-4-y1)méthyl éther

5 mmol de (1-Triphénylméthyl-1H-imidazol-4-yl)méthanolate de sodium et 5 mmol de chlorure de (bicyclo[2.2.1]hept-2-yl)méthane sont traités comme décrit dans l'exemple 5.
FS : C₁₂H₁₈N₂O.C₄H₄O₄ (322.4)

| | | | | | | | |
|---|---|---|---|---|---|---|---|
| Analyse CHN | Calculé | C | 59.6 | H | 6.88 | N | 8.69 |
| | Trouvé | C | 59.2 | H | 6.86 | N | 8.66 |
| Rendement : 50 % | | | | P.F. : 114°C | | | |

### Exemple 55

### 3-(1H-Imidazol-4-yl)propyl-3-(4-méthylphényl)propyl éther

5 mmol de 3-(1-Triphénylméthyl-1H-imidazol-4-yl)propanolate de sodium et 5 mmol de chlorure de 3-(4-méthylphényl) propane sont traités comme décrit dans l'exemple 5.
FS: C₁₆H₂₂N₂O.C₄H₄O₄ (374.4)

| | | | | | | | |
|---|---|---|---|---|---|---|---|
| Analyse CHN | Calculé | C | 64.2 | H | 7.00 | N | 7.48 |
| | Trouvé | C | 64.0 | H | 7.25 | N | 7.70 |
| Rendement : 20 % | | | | P.F. : 125-126°C | | | |

### Exemple 56

### 3-(1H-Imidazol-4₋yl)propyl-2-naphthylméthyl éther

5 mmol de 3-(1-Triphénylméthyl-1H-imidazol-4-yl)propanolate de sodium et 5 mmol de chlorure de 2-naphthylméthane sont traités comme décrit dans l'exemple 5.
FS : C₁₇H₁₈N₂O.C₄H₄O₄ (382.4)

| | | | | | | | |
|---|---|---|---|---|---|---|---|
| Analyse CHN | Calculé | C | 66.0 | H | 5.80 | N | 7.33 |
| | Trouvé | C | 65.8 | H | 5.76 | N | 7.06 |
| Rendement : 40 % | | | | P.F. : 93°C | | | |

### Exemple 57

### 3-(1H-Imidazol-4-yl)propyl-(4-biphényl)méthyl éther

5 mmol de 3-(1-Triphénylméthyl-1H-imidazol-4-yl)propanolate de sodium et 5 mmol de chlorure de 4-biphénylméthane sont traités comme décrit dans l'exemple 5.
FS : C₁₉H₂₀N₂O.C₄H₄O₄.0,5H₂O (417.5)

| | | | | | | | |
|---|---|---|---|---|---|---|---|
| Analyse CHN | Calculé | C | 66.2 | H | 6.04 | N | 6.71 |
| | Trouvé | C | 66.0 | H | 5.88 | N | 6.74 |
| Rendement : 30 % | | | | P.F. : 122°C | | | |

### Exemple 58

### (3-(4-Trifluorométhyl)phényl)propyl-3-(1H-imidazol-4-yl)propyl éther

5 mmol de 3-(1-Triphénylméthyl-1H-imidazol-4-yl)propanolate de sodium et 5 mmol de chlorure de 3-(4-trifluorométhyl)phénylpropane sont traités comme décrit dans l'exemple 5.
FS : C₁₆H₁₉N₂OF₃.C₄H₄O₄ (428.4)

| | | | | | | | |
|---|---|---|---|---|---|---|---|
| Analyse CHN | Calculé | C | 56.1 | H | 5.41 | N | 6.54 |
| | Trouvé | C | 56.2 | H | 5.62 | N | 6.61 |
| Rendement : 35 % | | | | P.F. : 104°C | | | |

### Exemple 59

### 3-(1H-Imidazol-4-yl)propyl-2-quinolylméthyl éther

5 mmol de 3-(1-Triphénylméthyl-1H-imidazol-4-yl)propanolate de sodium et 5 mmol de chlorure de 2-quinolylméthane sont traités comme décrit dans l'exemple 5.
FS : C₁₆H₁₇N₃O_{.}C₄H₄O₄ (383.4)

| | | | | | | | |
|---|---|---|---|---|---|---|---|
| Analyse CHN | Calculé | C | 62.7 | H | 5.52 | N | 11.0 |
| | Trouvé | C | 62.4 | H | 5.71 | N | 10.7 |
| Rendement : 35 % | | | | P.F. : 97°C | | | |

### Exemple 60

### 3-(2,4-Dichlorophényl)propyl-3-(1H-imidazol-4-yl)propyl éther

5 mmol de 3-(1-Triphénylméthyl-1H-imidazol-4-yl)propanolate de sodium et 5 mmol de chlorure de 3-(2,4-dichlorophényl)propane sont traités comme décrit dans l'exemple 5.
FS : C₁₅H₁₈N₂OCl₂.C₄H₄O₄ (429.3)

| | | | | | | | |
|---|---|---|---|---|---|---|---|
| Analyse CHN | Calcule C | | 53.2 | H | 5.17 | N | 6.53 |
| | Trouvé | C | 53.2 | H | 5.33 | N | 6.23 |
| Rendement : 30 % | | | | P.F. : 119-120°C | | | |

### Exemple 61

### 2-(Bicyclo[2.2.1]hept-2-yl)éthyl-3-(1H-imidazol-4-yl)propyl éther

5 mmol de 3-(1-Triphénylméthyl-1H-imidazol-4-yl)propanolate de sodium et 5 mmol de chlorure de 2-(bicyclo[2.2.1]hept-2-yl)éthane sont traités comme décrit dans l'exemple 5.
FS : C₁₅H₂₄N₂O.C₄H₄O₄.0,25H₂O (368.9)

| | | | | | | | |
|---|---|---|---|---|---|---|---|
| Analyse CHN | Calculé | C | 61.9 | H | 7.79 | N | 7.59 |
| | Trouvé | C | 61.6 | H | 7.72 | N | 7.72 |
| Rendement : 55 % | | | | P.F. : 94°C | | | |

### Exemple 62

### 3-(1H-Imidazol-4-yl)propyl-3-(4-méthoxyphényl)propyl éther

5 mmol de 3-(1-Triphénylméthyl-1H-imidazol-4-yl)propanolate de sodium et 5 mmol de chlorure de 3-(4-méthoxyphényl)propane sont traités comme décrit dans l'exemple 5.
FS : C₁₆H₂₂N₂O₂.C₄H₄O₄.0,25H₂O (394.9)

| | | | | | | | |
|---|---|---|---|---|---|---|---|
| Analyse CHN | Calculé | C | 60.8 | H | 6.76 | N | 7.09 |
| | Trouvé | C | 60.6 | H | 6.69 | N | 7.09 |
| Rendement : 50 % | | | | P.F. : 116°C | | | |

### Exemple 63

### 3-(1H-Imidazol-4-yl)propyl-2-phényléthyl éther

5 mmol de Chlorure de 3-(1-triphénylméthyl-1H-imidazol-4-yl)propane préparés à partir de 3-(1-triphénylméthyl-1H-imidazol-4-yl)propanol avec du chlorure de thionyle, et 15 mmol de 2-phényléthanolate de sodium sont traités comme décrit dans l'exemple 5.
FS : C₁₄H₁₈N₂O.C₄H₄O₄ (346.4)

| | | | | | | | |
|---|---|---|---|---|---|---|---|
| Analyse CHN | Calculé | C | 62.4 | H | 6.40 | N | 8.09 |
| | Trouvé | C | 62.2 | H | 6.47 | N | 8.06 |
| Rendement : 15 % | | | | P.F. : 89-91°C | | | |

### Exemple 64

### 3-(1H-Imidazol-4-yl)propyl-heptyl éther

5 mmol de 3-(1-Triphénylméthyl-1H-imidazol-4-yl)propanolate de sodium et 5 mmol de chlorure d'heptane sont traités comme décrit dans l'exemple 5.
FS : C₁₃H₂₄N₂O.C₄H₄O₄ (340.4)

| | | | | | | | |
|---|---|---|---|---|---|---|---|
| Analyse CHN | Calculé | C | 60.0 | H | 8.29 | N | 8.23 |
| | Trouvé | C | 59.6 | H | 8.23 | N | 8.18 |
| Rendement : 55 % | | | | P.F. : 88°C | | | |

### Exemple 65

### 3-(1H-Imidazol-4-yl)propyl-(2-méthylpropyl)éther

5 mmol de 3-(1-Triphénylméthyl-1H-imidazol-4-yl)propanolate de sodium et 5 mmol de chlorure de 2-méthylpropane sont traités comme décrit dans l'exemple 5.
FS : C₁₀H₁₈N₂O.C₄H₄O₄ (298.3)

| | | | | | | | |
|---|---|---|---|---|---|---|---|
| Analyse CHN | Calculé | C | 56.4 | H | 7.43 | N | 9.39 |
| | Trouvé | C | 56.6 | H | 7.23 | N | 9.21 |
| Rendement : 30 % | | | | P.F. : 82°C | | | |

### Exemple 66

### 2-(Cyclohexyléthyl)-3-(1H-imidazol-4-yl)propyl éther

5 mmol de Chlorure de 3-(1-triphénylméthyl-1H-imidazol-4-yl)propane (voir exemple 63) et 15 mmol de 2-cyclohéxyléthanolate de sodium sont traités comme décrit dans l'exemple 5.
FS : C₁₄H₂₄N₂O.C₄H₄O₄ (352.4)

| | | | | | | | |
|---|---|---|---|---|---|---|---|
| Analyse CHN | Calculé | C | 61.4 | H | 8.01 | N | 7.95 |
| | Trouvé | C | 61.2 | H | 8.05 | N | 7.95 |
| Rendement : 45 % | | | | P.F. : 96°C | | | |

### Exemple 67

### 3-(1H-Imidazol-4-yl)propyl-(pent-4-inyl)éther

5 mmol de 3-(1-Triphénylméthyl-1H-imidazol-4-yl)propanolate de sodium et 5 mmol de chlorure de pent-4-ine sont traités comme décrit dans l'exemple 5.
FS : C₁₁H₁₆N₂O.C₄H₄O₄.0,5H₂O (317.3)

| | | | | | | | |
|---|---|---|---|---|---|---|---|
| Analyse CHN | Calculé | C | 56.8 | H | 6.67 | N | 8.83 |
| | Trouvé | C | 56.9 | H | 6.42 | N | 8.77 |
| Rendement : 40 % | | | | P.F. : 74°C | | | |

### Exemple 68

### 3-(1H-Imidazol-4-yl)propyl-2-(phénoxy)éthyl éther

5 mmol de 3-(1-Triphénylméthyl-1H-imidazol-4-yl)propanolate de sodium et 5 mmol de chlorure 2-phénoxyéthane sont traités comme décrit dans l'exemple 5.
FS : C₁₄H₁₈N₂O₂.C₄H₄O₄.0,25H₂O (366.9)

| | | | | | | | |
|---|---|---|---|---|---|---|---|
| Analyse CHN | Calculé | C | 58.9 | H | 6.18 | N | 7.64 |
| | Trouvé | C | 58.7 | H | 6.15 | N | 7.64 |
| Rendement : 40 % | | | | P.F. : 96°C | | | |

### Exemple 69

### 3-(1H-Imidazol-4-yl)propyl-4-(méthylthio)phénylméthyl éther

5 mmol de 3-(1-Triphénylméthyl-1H-imidazol-4-yl)propanolate de sodium et 5 mmol de chlorure 4-(méthylthio)phénylméthane sont traités comme décrit dans l'exemple 5.
FS : C₁₄H₁₈N₂OS.C₄H₄O₄ (378.5)

| | | | | | | | |
|---|---|---|---|---|---|---|---|
| Analyse CHN | Calculé | C | 57.1 | H | 5.86 | N | 7.40 |
| | Trouvé | C | 57.2 | H | 5.84 | N | 7.25 |
| Rendement : 50 % | | | | P.F. : 108°C | | | |

### Exemple 70

### 3-(4-Fluorophényl)propyl-3-(1H-imidazol-4-yl)prop-2-ényl. éther

5 mmol de 3-(1-Triphénylméthyl-1H-imidazol-4-yl)prop-2-énolate de sodium et de chlorure de 3-(4-fluorophényl)propane sont traités comme décrit dans l'exemple 5.
FS : C₁₅H₁₇N₂OF.C₄H₄O₄ (376.4)

| | | | | | | | |
|---|---|---|---|---|---|---|---|
| Analyse CHN | Calculé | C | 60.6 | H | 5.62 | N | 7.44 |
| | Trouvé | C | 60.4 | H | 5.55 | N | 7.69 |
| Rendement : 20 % | | | | P.F. : 130-132°C | | | |

### Exemple 71

### 3-(1H-Imidazol-4-yl)propyl-(diphénylméthyl) éther

5 mmol de 3-(1H-Imidazol-4-yl)propanol.HCl et 5 mmol de chlorure de diphénylméthane dans 50 ml d'acétonitrile sont mis au reflux pendant 4-5 heures, le solvant est évaporé et le résidu purifié par chromatographie rotatoire (éluant : chloroforme/méthanol (90-99/10-1), atmosphère ammoniaquée). Après évacuation du solvant sous pression réduite, le résidu est cristallisé en tant que maléate d'hydrogène depuis le diéthyl éther et l'éthanol.
FS : C₁₉H₂₀N₂O.C₄H₄O₄ (408.5)

| | | | | | | | |
|---|---|---|---|---|---|---|---|
| Analyse CHN | Calculé | C | 67.6 | H | 5.92 | N | 6.85 |
| | Trouvé | C | 67.4 | H | 5.92 | N | 6.86 |
| Rendement : 55 % | | | | P.F. : 105-107°C | | | |

### Exemple 72

### ((4-Fluorophényl)phénylméthyl)-3-(1H-imidazol-4-yl)propyl éther

5 mmol de 3-(1H-Imidazol-4-yl)propanol.HCl. et 5 mmol de chlorure de (4-fluorophényl)phénylméthane sont traités comme décrit dans l'exemple 71.
FS : C₁₉H₁₉N₂OF.C₄H₄O₄ (426.4)

| | | | | | | | |
|---|---|---|---|---|---|---|---|
| Analyse CHN | Calculé | C | 64.8 | H | 5.44 | N | 6.57 |
| | Trouvé | C | 64.7 | H | 5.34 | N | 6.76 |
| Rendement : 65 % | | | | P.F. : 90°C | | | |

### Exemple 73

### Bis(4-fluorophényl)méthyl-3-(1H-imidazol-4-yl)propyl éther

5 mmol de 3-(1H-Imidazol-4-yl)propanol.HCl et 5 mmol de chlorure de bis(4-fluorophényl)méthane sont traités comme décrit dans l'exemple 71.
FS : C₁₉H₁₈N₂OF₂.C₄H₄O₄ (444.4)

| | | | | | | | |
|---|---|---|---|---|---|---|---|
| Analyse CHN | Calculé | C | 62.2 | H | 4.98 | N | 6.30 |
| | Trouvé | C | 61.9 | H | 4.98 | N | 6.29 |
| Rendement : 40 % | | | | P.F. : 107-109°C | | | |

### Exemple 74

### 2-(3-(1H-Imidazol-4-yl)propyloxy)-1-phényléthanone

5 mmol de 3-(1-Triphénylméthyl-1H-imidazol-4-yl)propanol et 5 mmol de 2-bromo-1-phényléthanone sont mis à agitation pendant 72 heures dans le chlorure de méthylène. Le solvant est évaporé sous pression réduite et le résidu mis au reflux pendant 1 heure dans 30 ml HCl 2N et 30 ml d'acétone. Le solvant est évaporé sous pression réduite et le triphénylméthanol est extrait à l'aide de diéthyléther. La phase acqueuse est alcalinisé avec de l'ammoniac, le produit brut est extrait au diéthyléther, purifié par chromatographie rotatoire et cristallisé en tant que maléate d'hydrogène depuis le diéthyléther et l'éthanol.
FS : C₁₄H₁₆N₂O₂.C₄H₄O₄ (360.4)

| | | | | | | | |
|---|---|---|---|---|---|---|---|
| Analyse CHN | Calculé | C | 60.0 | H | 5.59 | N | 7.77 |
| | Trouvé | C | 60.0 | H | 5.73 | N | 7.77 |
| Rendement : 80 % | | | | P.F. : 85-86°C | | | |

### Exemple 75

### 2-(3-(1H-Imidazol-4-yl)propyloxy)-1-(3-nitrophényl) éthanone

5 mmol de 3-(1-Triphénylméthyl-1H-imidazol-4-yl)propanol et 5 mmol de 2-bromo-1-(3-nitrophényl)éthanone sont traités comme décrit dans l'exemple 74.
FS : C₁₄H₁₅N₃O₄.C₄H₄O₄.H₂O (423.4)

| | | | | | | | |
|---|---|---|---|---|---|---|---|
| Analyse CHN | Calculé | C | 51.1 | H | 5.00 | N | 9.92 |
| | Trouvé | C | 51.4 | H | 4.73 | N | 10.1 |
| Rendement : 35 % | | | | P.F. : 117-118°C | | | |

### Exemple 76

### 4-(4-(3-(1H-Imidazol-4-yl)propyloxy)phényl)butan-2-one

5 mmol de 3-(1-Triphénylméthyl-1H-imidazol-4-yl)propanol, 6 mmol de triphénylphosphine, et 5 mmol de 4-(4-hydroxyphényl)butan-2-one sont dissouts sous azote au froid. 6 mmol diéthylazodicarboxylate, dissouts dans 4 ml THF, sont ajoutés et le mélange réactionnel est agité à température ambiante pendant 48 heures. Après évacuation du solvant sous pression réduite et une chromatographie en colonne (éluant : éthylacétate), le rédidu est dissout dans 10 ml THF et 30 ml de HCl 2N, et chauffé à 70°C pendant. 2 heures. Le solvant est évaporé sous presion réduite et le triphénylméthanol est extrait à l'aide de diéthyléther. La phase acqueuse est neutralisée avec du carbonate de potassium et le produit extrait avec du diéthyléther. La solution d'éther est séchée et évaporée pour obtenir une huile qui est cristallisée en tant que maléate d'hydrogène depuis le diéthyléther et l'éthanol.
FS : C₁₆H₂₀N₂O₂.C₄H₄O₄.0,5H₂O (397.4)

| | | | | | | | |
|---|---|---|---|---|---|---|---|
| Analyse CHN | Calculé | C | 60.4 | H | 6.34 | N | 7.05 |
| | Trouvé | C | 60.5 | H | 6.28 | N | 7.43 |
| Rendement : 75 % | | | | P.F. : 104°C | | | |

### Exemple 77

### (4-(3-(1H-Imidazol-4-yl)propyloxy)phényl)carbaldéhyde

5 mmol de 3-(1-Triphénylméthyl-1H-imidazol-4-yl)propanol et 6 mmol de 4-hydroxyphénylcarbaldéhyde sont traités comme décrit dans l'exemple 76.
FS : C₁₃H₁₄N₂O₂.C₄H₄O₄ (346.3)

| | | | | | | | |
|---|---|---|---|---|---|---|---|
| Analyse CHN | Calculé | C | 59.0 | H | 5.24 | N | 8.08 |
| | Trouvé | C | 58.9 | H | 5.51 | N | 8.24 |
| Rendement : 85 % | | | | P.F. : 120°C | | | |

### Exemple 78

### (4-(3-(1H-Imidazol-4-yl)propyloxy)phényl)éthanone

5 mmol de 3-(1-Triphénylméthyl-1H-imidazol-4-yl)propanol et 6 mmol de (4-hydroxyphényl)éthanone sont traités comme décrit dans l'exemple 76.
FS : C₁₄H₁₆N₂O₂.C₄H₄O₄ (360.4)

| | | | | | | | |
|---|---|---|---|---|---|---|---|
| Analyse CHN | Calculé | C | 60.0 | H | 5.59 | N | 7.77 |
| | Trouvé | C | 59.8 | H | 5.86 | N | 7.56 |
| Rendement : 80 % | | | | P.F. : 118°C | | | |

### Exemple 79

### (4-(3-(1H-Imidazol-4-yl)propyloxy)phényl)propanone

5 mmol de 3-(1-Triphénylméthyl-1H-imidazol-4-yl)propanol et 6 mmol de 4-hydroxyphénylpropanone sont traités comme décrit dans l'exemple 76.
FS : C₁₅H₁₈N₂O₂.C₄H₄O₄ (374.4)

| | | | | | | | |
|---|---|---|---|---|---|---|---|
| Analyse CHN | Calculé | C | 61.0 | H | 5.92 | N | 7.46 |
| | Trouvé | C | 61.0 | H | 5.88 | N | 7.42 |
| Rendement : 80 % | | | | P.F. : 136°C | | | |

### Exemple 80

### (4-(3-(1H-Imidazol-4-yl)propyloxy)phényl)-2-méthylpropanone

5 mmol de 3-(1-Triphénylméthyl-1H-imidazol-4-yl)propanol et 6 mmol de (4-hydroxyphényl)-2-méthylpropanone sont traités comme décrit dans l'exemple 76.
FS : C₁₆H₂₀N₂O₂.C₄H₄O₄.0,5H₂O (397.4)

| | | | | | | | |
|---|---|---|---|---|---|---|---|
| Analyse CHN | Calculé | C | 60.4 | H | 6.34 | N | 7.05 |
| | Trouvé | C | 60.5 | H | 6.03 | N | 7.03 |
| Rendement : 85 % | | | | P.F. : 95°C | | | |

### Exemple 81

### cyclopropyl-(4-(3-(1H-imidazol-4-yl)propyloxy)phényl) cétone

Méthode 1 : 5 mmol de 3-(1-triphénylméthyl-1H-imidazol-4-yl)propanolate de sodium, 10 mmol de 4-chloro-4'-fluorobutyrophénone et 30 mmol de NaH (60 % en suspension dans l'huile minérale) sont chauffés pedant 48 h dans du toluène au reflux. Le mélange réactionnel est traité comme décrit à l'exemple 5. Rendement : 40 %.
Méthode 2 : 5 mmol de 3-(1-triphénylméthyl-1H-imidazol-4-yl)propanol et 6 mmol de cyclopropyl-(4-hydroxyphényl)cétone sont traités comme décrit dans l'exemple 76. Rendement 80 %.
Méthode 3 : 5 mmol de 3-(1-Triphénylméthyl-1H-imidazol-4-yl)propanolate de sodium, 10 mmol de cyclopropyl-4-fluorophénylcétone et 10 mmol NaH (60 % en suspension dans l'huile minérale) sont chauffés pendant 4 heures dans du toluène sous reflux. Le mélange réactionnel est alors traité comme décrit dans l'exemple 5. Rendement 40 %.
FS : C₁₆H₁₈N₂O₂.C₄H₄O₄ (386,4)

| | | | | | | | |
|---|---|---|---|---|---|---|---|
| Analyse CHN | Calculé | C | 62.2 | H | 5.74 | N | 7.25 |
| | Trouvé | C | 62.5 | H | 5.81 | N | 7.20 |
| | | | | P.F. : 118-125°C. | | | |

### Exemple 82

### cyclobutyl-(4-(3-(1H-imidazol-4-yl)propyloxy)phényl) cétone

5 mmol de 3-(1-Triphénylméthyl-1H-imidazol-4-yl)propanol et 6 mmol de cyclobutyl-(4-hydroxyphényl)cétone sont traités comme décrit dans l'exemple 76.
FS : C₁₇H₂₀N₂O₂.C₄H₄O₄.0,25H₂O (404.9)

| | | | | | | | |
|---|---|---|---|---|---|---|---|
| Analyse CHN | Calculé | C | 62.3 | H | 6.10 | N | 6.92 |
| | Trouvé | C | 62.0 | H | 6.02 | N | 7.18 |
| Rendement : 80 % | | | | P.F. : 130°C | | | |

### Exemple 83

### Cyclopentyl-(4-(3-(1H-imidazol-4-yl)propyloxy)phényl) cétone

5 mmol de 3-(1-Triphénylméthyl-1H-imidazol-4-yl)propanol et 6 mmol de cyclopentyl-(4-hydroxyphényl)cétone sont traités comme décrit dans l'exemple 76.
FS : C₁₈H₂₂N₂O₂.C₄H₄O₄.0,25H₂O (419.0)

| | | | | | | | |
|---|---|---|---|---|---|---|---|
| Analyse CHN | Calculé | C | 63.1 | H | 6.38 | N | 6.69 |
| | Trouvé | C | 63.3 | H | 6.46 | N | 6.94 |
| Rendement : 80 % | | | | P.F. : 141°C | | | |

### Exemple 84

### cyclohéxyl-(4-(3-(1H-imidazol-4-yl)propyloxy)phényl) cétone

5 mmol de 3-(1-Triphénylméthyl-1H-imidazol-4-yl)propanol et 6 mmol de cyclohéxyl-(4-hydroxyphényl)cétone sont traités comme décrit dans l'exemple 76.
FS : C₁₉H₂₄N₂O₂.C₄H₄O₄ (428.5)

| | | | | | | | |
|---|---|---|---|---|---|---|---|
| Analyse CHN | Calculé | C | 64.5 | H | 6.59 | N | 6.54 |
| | Trouvé | C | 64.6 | H | 6.32 | N | 6.82 |
| Rendement : 80 % | | | | P.F. : 103°C | | | |

### Exemple 85

### (4-(3-(1H-Imidazol-4-yl)propyloxy)phényl)phényl cétone

5 mmol de 3-(1-Triphénylméthyl-1H-imidazol-4-yl)propanol et 6 mmol de 4-hydroxybenzophénone sont traités comme décrit dans l'exemple 76.
FS : C₁₉H₁₈N₂O₂.C₄H₄O₄.0,25H₂O (426.9)

| | | | | | | | |
|---|---|---|---|---|---|---|---|
| Analyse CHN | Calculé | C | 64.7 | H | 5.31 | N | 6.56 |
| | Trouvé | C | 64.7 | H | 5.23 | N | 6.59 |
| Rendement : 70 % | | | | P.F. : 126°C | | | |

### Exemple 86

### (4-(3-(1H-Imidazol-4-yl)propyloxy)phényl)-4-fluorophényl cétone

5 mmol de 3-(1-Triphénylméthyl-1H-imidazol-4-yl)propanol et 5 mmol de 4-fluoro-4'-hydroxybenzophénone sont traités comme décrit dans l'exemple 76.
FS : C₁₉H₁₇N₂O₂F.C₄H₄O₄ (440.4)

| | | | | | | | |
|---|---|---|---|---|---|---|---|
| Analyse CHN | Calculé | C | 62.7 | H | 4.81 | N | 6.36 |
| | Trouvé | C | 62.6 | H | 4.83 | N | 6.43 |
| Rendement : 80 % | | | | P.F. : 125-127°C | | | |

### Exemple 87

### (4-(3-(1H-Imidazol-4-yl)propyloxy)phényl)méthanol

2 mmol de (4-(3-(1H-Imidazol-4-yl)propyloxy)phényl) carbaldéhyde (exemple 77) sont mis dans une suspension de 0,5 mmol LiAlH₄ dans 10 ml THF anhydre puis le mélange réactionnel est mis au reflux pendant 1 heure. 5 ml de NaOH 2N sont ajoutés, la phase organique séparée, lavée à l'eau et séchée avec du carbonate de sodium.

Après élimination du solvant sous pression réduite, le résidu est cristallisé en tant que maléate d'hydrogène depuis le diéthyléther et l'éthanol.
FS : C₁₃H₁₆N₂O₂.C₄H₄O₄.0,25H₂O (352.9)

| | | | | | | | |
|---|---|---|---|---|---|---|---|
| Analyse CHN | Calculé | C | 57.9 | H | 5.86 | N | 7.94 |
| | Trouvé | C | 58.0 | H | 5.83 | N | 7.77 |
| Rendement : 50 % | | | | P.F. : 134°C | | | |

### Exemple 88

### 1-(4-(3-(1H-Imidazol-4-yl)propyloxy)phényl)éthanol

2 mmol de (4-(3-(1H-Imidazol-4-yl)propyloxy)phényl) éthanone (exemple 78) sont traités comme décrit dans l'exemple 87.
FS : C₁₄H₁₈N₂O₂.C₄H₄O₄.0,25H₂O (366.9)

| | | | | | | | |
|---|---|---|---|---|---|---|---|
| Analyse CHN | Calculé | C | 58.9 | H | 6.18 | N | 7.64 |
| | Trouvé | C | 58.8 | H | 6.22 | N | 7.62 |
| Rendement : 55 % | | | | P.F. : 95°C | | | |

### Exemple 89

### 1-(4-(3-(1H-Imidazol)4-yl)propyloxy)phényl-2-méthylpropanol

2 mmol de (4-(3-(1H-Imidazol-4-yl)propyloxy)phényl)-2-méthylpropanone (exemple 80) sont traités comme décrit dans l'exemple 87.
FS : C₁₆H₂₂N₂O₂.C₄H₄O₄ (390.4)

| | | | | | | | |
|---|---|---|---|---|---|---|---|
| Analyse CHN | Calculé | C | 61.5 | H | 6.71 | N | 7.71 |
| | Trouvé | C | 61.2 | H | 6.63 | N | 7.42 |
| Rendement : 55 % | | | | P.F. : 139°C | | | |

### Exemple 90

### Cyclopropyl-(4-(3-(1H-imidazol-4-yl)propyloxy)phényl) méthanol

2 mmol de Cyclopropyl-(4-(3-(1H-imidazol-4-yl)propyloxy) phényl)cétone (exemple 81) sont traités comme décrit dans l'exemple 87.
FS : C₁₆H₂₀N₂O₂.C₄H₄O₄ (388.4)

| | | | | | | | |
|---|---|---|---|---|---|---|---|
| Analyse CHN | Calculé | C | 61.9 | H | 6.23 | N | 7.21 |
| | Trouvé | C | 61.9 | H | 6.29 | N | 7.12 |
| Rendement : 70 % | | | | P.F. : 84-85°C | | | |

### Exemple 91

### (4-(3-(1H-Imidazol₋4-yl)propyloxy)phényl)butanone

5 mmol de 3-(1-Triphénylméthyl-1H-imidazol-4-yl)propanol et 6 mmol (4-hydroxyphényl)butanone sont traités comme décrit dans l'exemple 76.
FS : C₁₆H₂₀N₂O.C₄H₄O₄.H₂O (406.4)

| | | | | | | | |
|---|---|---|---|---|---|---|---|
| Analyse CHN | Calculé | C | 59.1 | H | 6.45 | N | 6.89 |
| | Trouvé | C | 59.2 | H | 6.23 | N | 7.30 |
| Rendement : 78 % | | | | P.F. : 87°C | | | |

### Exemple 92

### (4-(3-(1H-Imidazol-4-yl)propyloxy)phényl)éthanone oxime

1,2 mmol de (4-(3-(1H-Imidazol-4-yl)propyloxy)phényl) éthanone (exemple 78), 2,4 mmol d'hydrochlorure d'hydroxylamine et 4,8 mmol de NaOH sont chauffés sous reflux dans 10 ml d'eau et 10 ml d'éthanol pendant 7 heures. Le mélange est concentré sous pression réduite, alcalinisé avec une solution saturée de K₂CO₃, et le produit brut est filtré et lavé avec de l'eau. Le produit est cristallisé en tant que maléate d'hydrogène depuis le diéthyléther et l'éthanol.
FS : C₁₄H₁₇N₃O₂.C₄H₄O₄.0,5H₂O (384.4)

| | | | | | | | |
|---|---|---|---|---|---|---|---|
| Analyse CHN | Calculé | C | 56.2 | H | 5.76 | N | 10.9 |
| | Trouvé | C | 56.4 | H | 5.60 | N | 10.9 |
| Rendement : 31 % | | | | P.F. : 141-144°C | | | |

### Exemple 93

### (4-(3-(1H-Imidazol-4-yl)propyloxy)phényl)éthanone o-méthyl oxime

1,2 mmol de (4-(3-(1H-Imidazol-4-yl)propyloxy)phényl) éthanone (exemple 78) et 2,4 mmol d'hydro chlorure de 0-méthylhydroxylamine sont traités comme décrit dans l'exemple 92.
FS : C₁₅H₁₉N₃O₂.C₄H₄O₄ (389.4)

| | | | | | | | |
|---|---|---|---|---|---|---|---|
| Analyse CHN | Calculé | C | 58.6 | H | 5.95 | N | 10.8 |
| | Trouvé | C | 58.3 | H | 5.96 | N | 10.9 |
| Rendement : 42 % | | | | P.F. : 124-126°C | | | |

### Exemple 94

### (4-(3-(1H-Imidazol-4-yl)propyloxy)phényl)propan-2-one

5 mmol de 3-(1-Triphénylméthyl-1H-imidazol-4-yl)propanol et 6 mmol de (4-hydroxyphényl)propan-2-one sont traités comme décrit dans l'exemple 76.
FS : C₁₅H₁₈N₂O₂.C₄H₄O4.0,5H₂O (383.4)

| | | | | | | | |
|---|---|---|---|---|---|---|---|
| Analyse CHN | Calculé | C | 59.5 | H | 6.05 | N | 7.31 |
| | Trouvé | C | 59.3 | H | 6.00 | N | 7.66 |
| Rendement : 70 % | | | | P.F. : 89°C | | | |

### Exemple 95

### 3-(1H-Imidazol-4-yl)propyl-4-méthoxyphényl éther

5 mmol de 3-(1-Triphénylméthyl-1H-imidazol-4-yl)propanol et 6 mmol de 4-méthoxyphénol sont traités comme décrit dans l'exemple 76.
FS : C₁₃H₁₆N₂O₂.C₄H₄O₄.0,25H₂O (352.9)

| | | | | | | | |
|---|---|---|---|---|---|---|---|
| Analyse CHN | Calculé | C | 57.9 | H | 5.86 | N | 7.94 |
| | Trouvé | C | 57.7 | H | 5.71 | N | 8.01 |
| Rendement : 85 % | | | | P.F. : 126°C | | | |

### Exemple 96

### N-(4-Acétylphényl)-(3-(4H-imidazol-4-yl)propyl carbamate

5 mmol de 3-(1H-Imidazol-4-yl)propanol.HCl et 5 mmol d'isocyanate d'acétylphényl sont traités comme décrit dans l'exemple 1.
FS : C₁₅H₁₇N₃O₃.C₄H₄O₄.0,25H₂O (407.9)

| | | | | | | | |
|---|---|---|---|---|---|---|---|
| Analyse CHN | Calculé | C | 56.0 | H | 5.31 | N | 10.3 |
| | Trouvé | C | 56.0 | H | 5.20 | N | 10.3 |
| Rendement : 36 % | | | | P.F. : 158-159°C | | | |

### Exemple 97

### N-(3-Acétylphényl)-(3-(1H-imidazol-4-yl)propyl carbamate

5 mmol de 3-(1H-Imidazol-4-yl)propanol.HCl et 5 mmol d'isocyanate de 3-acétylphényl sont traités comme décrit dans l'exemple 1.
FS : C₁₅H₁₇N₃O₃.C₄H₄C₄.0,25H₂O (407.9)

| | | | | | | | |
|---|---|---|---|---|---|---|---|
| Analyse CHN | Calculé | C | 56.0 | H | 5.31 | N | 10.3 |
| | Trouvé | C | 56.2 | H | 5.29 | N | 10.2 |
| Rendement : 46 % | | | | P.F. : 136-137°C | | | |

### Exemple 98

### 4-(3-(1H-1midazol-4-yl)propyloxy)butan-2-one

5 mmol de 3-(1H-Imidazol-4-yl)propanol.HCl et 10 mmol de 3-butèn-2-one sont chauffés à 40°C pendant 1 heure dans 20 ml d'acétonitrile et une goutte de H₂SO₄ conc. Le mélange est neutralisé avec du Na₂CO₃, le solvant est évaporé et le résidu purifié par chromatographie en colonne (éluant : chlorure de méthylène/méthanol (90/10), atmosphère ammoniquée). Après élimination du solvant sous pression réduite, le produit est recristallisé en tant que maléate d'hydrogène depuis le diéthyléther et l'éthanol.
FS: C₁₀H₁₆N₂O.C₄H₄O₄.0,5H₂O (321.3)

| | | | | | | | |
|---|---|---|---|---|---|---|---|
| Analyse CHN | Calculé | C | 52.3 | H | 6.59 | N | 9.16 |
| | Trouvé | C | 51.9 | H | 6.45 | N | 9.07 |
| Rendement : 30 % | | | | P.F. : 56°C | | | |

### Exemple 99

### 3-(1H-Imidazol-4-yl)propyl-2-méthoxyéthyl éther

5 mmol de 3-(1-Triphénylméthyl-1H-imidazol-4-yl)propanolate de sodium et 7 mmol de chlorure de 2-méthoxyéthane sont traités comme décrit dans l'exemple 5.
FS : C₉H₁₆N₂O₂.C₄H₄O₄ (247.3)

| | | | | | | | |
|---|---|---|---|---|---|---|---|
| Analyse CHN | Calculé | C | 48.2 | H | 6.61 | N | 10.2 |
| | Trouvé | C | 48.6 | H | 6.98 | N | 10.6 |
| Rendement : 20 % | | | | P.F. : 130-132°C | | | |

### Exemple 100

### 3-Cyclopentylpropyl-3-(1H-imidazol-4-yl)propyl éther

5 mmol de 3-(1-Triphénylméthyl-1H-imidazol-4-yl)propanolate de sodium et du chlorure de 3-cyclopentylphénylpropane sont traités comme décrit dans l'exemple 5.
FS : C₁₄H₂₄N₂O.C₄H₄O₄ (352.4)

| | | | | | | | |
|---|---|---|---|---|---|---|---|
| Analyse CHN | Calculé | C | 61.4 | H | 8.01 | N | 7.95 |
| | Trouvé | C | 61.2 | H | 8.05 | N | 7.98 |
| Rendement : 70 % | | | | P.F. : 101°C | | | |

### Exemple 101

### 3-(1H-Imidazol-4-yl)propyl-N-isopropyl carbamate

5 mmol de 3-(1H-Imidazol-4-yl)propanol.HCl et 5 mmol d'isocyanate d'isopropyle sont traités comme décrit dans l'exemple 1.
FS : C₁₀H₁₇N₃O₂.C₄H₄O₄.0,25H₂O (331.8)

| | | | | | | | |
|---|---|---|---|---|---|---|---|
| Analyse CHN | Calculé | C | 50.7 | H | 6.53 | N | 12.7 |
| | Trouvé | C | 50.8 | H | 6.29 | N | 12.6 |
| Rendement : 45 % | | | | P.F. : 118-119°C | | | |

### Exemple 102

### N-(3,3-Diphénylpropyl)-3-(1H-imidazol-4-yl)propyl carbamate

5 mmol de 3-(1H-Imidazol-4-yl)propanol.HCl et 5 mmol d'isocyanate de 3,3-diphénylpropyle sont traités comme décrit dans l'exemple
FS : C₂₂H₂₅N₃O₂.C₄H₄O₄ (479.5)

| | | | | | | | |
|---|---|---|---|---|---|---|---|
| Analyse CHN | Calculé | C | 65.1 | H | 6.10 | N | 8.76 |
| | Trouvé | C | 65.0 | H | 6.18 | N | 8.63 |
| Rendement : 61 % | | | | P.F. : 126-128C | | | |

### Exemple 103

### (1H-Imidazol-4-yl)nonane

10 mmol de bromure de nonyltriphénylphosphonium, 10 mmol de (1-triphénylméthyl-1H-imidazol-4-yl)méthanal (voir exemple 51) sont traités comme décrit dans l'exemple 51.
Le produit de cette réaction ((1H-imidazol-4-yl)non-1-ène) est traité comme décrit dans l'exemple 52.
FS : C₁₂H₂₂N₂.C₂H₂O₄ (284.4)

| | | | | | | | |
|---|---|---|---|---|---|---|---|
| Analyse CHN | Calculé | C | 59.1 | H | 8.51 | N | 9.85 |
| | Trouvé | C | 59.1 | H | 8.39 | N | 9.79 |
| Rendement : 10 % | | | | P.F. : 137-138°C | | | |

### Exemple 104

### 3-(4-(3-(1H-Imidazol-4-yl)propyloxy)phényl)propénal

5 mmol de (4-(3-(1-Triphénylméthyl-1H-imidazol-4-yl)propyloxy)phényl)carbaldéhyde. (intermédiaire de l'exemple 77) et 5 mmol de bromure d'éthinylmagnésium sont dissouts dans 20 ml THF et mis au reflux pendant 1 h. Le solvant est évaporé et le résidu chauffé pendant 2 h dans 50 ml de HCl 2N. Les sous-produits lipophiles sont extraits avec du diéthyléther. La phase aqueuse est alcalinisée avec de l'ammoniac, le produit brut est extrait avec du diéthyléther et purifié par chromatographie rotatoire (éluant : chloroforme/méthanol (99/1 - 90/10), atmosphère ammoniaquée). Après élimination du solvant sous pression réduite, le produit est recristallisé en tant que maléate d'hydrogène depuis le diéthyléther et l'éthanol.
FS : C₁₅H₁₆N₂O₂.C₄H₄O₄.0,5H₂O (381.4)

| | | | | | | | |
|---|---|---|---|---|---|---|---|
| Analyse CHN | Calculé | C | 59.8 | H | 5.55 | N | 7.35 |
| | Trouvé | C | 59.5 | H | 5.37 | N | 7.99 |
| Rendement : 36 % | | | | P.F. : 119-121°C | | | |

### Exemple 105

### 4-[3-(4-Ethoxyphénoxy)propyl]-1H-imidazole

On procède comme décrit à la voie de synthèse A.
Forme saline : oxalate
Solvant de cristallisation : 2-PrOH : Et₂O
FS : C₁₄H₁₈N₂O₂.0,8C₂H₂O₄

| | | | | | | | |
|---|---|---|---|---|---|---|---|
| Analyse CHN | Calculé | C | 58.8 | H | 6.21 | N | 8.80 |
| | Trouvé | C | 59.0 | H | 6.35 | N | 8.94 |
| | | | | P.F. : 191-193°C | | | |

### Exemple 106

### 4-[2-(3-Propanoylphénoxy)éthyl]-1H-imidazole

On procède comme décrit à la voie de synthèse F.
Forme saline : oxalate
Solvant de cristallisation : EtOH
FS : C₁₄H₁₆N₂O₂.0,8C₂H₂O₄

| | | | | | | | |
|---|---|---|---|---|---|---|---|
| Analyse CHN | Calculé | C | 59.2 | H | 5.61 | N | 8.86 |
| | Trouvé | C | 59.5 | H | 5.62 | N | 8.85 |
| | | | | P.F. : 148-150°C | | | |

### Exemple 107

### 4-[3-(3-Hydroxyiminométhylènephénoxy)propyl]-1H-imidazole

On procède comme décrit à la voie de synthèse ci-dessous. Forme saline : oxalate
Solvant de cristallisation : 2-PrOH : Et₂O
FS : C₁₃H₁₅N₃O₂.C₂H₂O₄

| | | | | | | | |
|---|---|---|---|---|---|---|---|
| Analyse CHN | Calculé | C | 53.7 | H | 5.50 | N | 12.5 |
| | Trouvé | C | 53.7 | H | 5.11 | N | 12.5 |
| | | | | P.F. : 120-122°C | | | |

### Préparation du 4-[3-(3-Hydroximinométhylènephénoxy) propyl]-1H-imidazole oxalate

Un mélange d'hydrochlorure d'hydroxylamine (0,5 : 7,24 mmol) et d'acétate de sodium (1 g ; 0,012 mmol) dans 10 ml d'eau est agité pendant 10 min. Puis, une solution de 4-[3-(3-formylphénoxy)propyl]-1H-imidazole (0,25 g ; 1,08 mmol) dans 3 ml d'éthanol est ajoutée lentement et le mélange est chauffé à 80°C pendant 2 heures. Le solvant est éliminé sous pression réduite, et il reste un résidu blanc qui est extrait avec du chloroforme (3 x 100 ml). Les extraits chloroformiques combinés sont séchés (Mg SO₄) et évaporés sous pression réduite en laissant une huile (0,11 g). Cette dernière est dissoute dans du 2-propanol (3 ml) et traitée avec un excès d'acide oxalique (1,5 équiv.) dans 2 ml de 2-propanol. Le produit, qui est précipité par addition de diéthyléther, est filtré, lavé avec du diéthyléther et a un point de fusion de 120-122°C.

### Exemple 108

### 4-[3-(3-Formylphénoxy)propyl]-1H-imidazole

On procède comme décrit à la voie de synthèse A.
Forme saline : oxalate
Solvant de cristallisation : EtOH
FS : C₁₃H₁₄N₂O₂.0,85C₂H₂O₄.0,1H₂O

| | | | | | | | |
|---|---|---|---|---|---|---|---|
| Analyse CHN | Calculé | C | 57.6 | H | 5.22 | N | 9.31 |
| | Trouvé | C | 57.3 | H | 4.89 | N | 9.65 |
| | | | | P.F. : 158-160°C | | | |

### Exemple 109

### 4-[3-(4-Benzyloxyphénoxy)propyl]-1H-imidazole

On procède comme décrit à la voie de synthèse A.
Forme saline : base
Solvant de cristallisation : EtOH
FS : C₁₉H₂₀N₂O₂.0,85H₂O

| | | | | | | | |
|---|---|---|---|---|---|---|---|
| Analyse CHN | Calculé | C | 70.9 | H | 6.73 | N | 8.70 |
| | Trouvé | C | 70.9 | H | 6.44 | N | 8.55 |
| | | | | P.F. : 135-137°C | | | |

### Exemple 110

### 3-(1H-imidazol-4-yl)propyl-N-(2-méthylbut-2-yl)carbamate

5 mmol d'acide 2,2-diméthylbutyrique, 5 mmol de triéthylamine et 5 mmol de phosphorazidate de diphényle dans 30 ml d'acétonitrile anhydre sont agitées à 20°C pendant 45 minutes, puis sont portées à reflux pendant 30 minutes. 6 mmol de 3-(1H-imidazol-4-yl)propanol.HCl sont ajoutées et sont portées à reflux pendant 40 heures. Le mélange est évaporé et le résidu dissous dans l'éther diéthylique. La solution est lavée successivement avec 30 ml d'acide citrique aqueux à 5 %, 30 ml d'H₂O et 30 ml de NaHCO₃ aqueux saturé. La couche organique est évaporée et le résidu est purifié par chromatographie rotatoire (éluant : chloroforme/méthanol (99/1-90/10)). Après élimination du solvant sous pression réduite, le réside est cristallisé sous forme de l'hydrogénooxalate dans l'éther diéthylique et l'éthanol.
C₁₂H₂₁N₃O₂.C₂H₂O₄ (329,4)

| | | | | | | | |
|---|---|---|---|---|---|---|---|
| Analyse CHN | Calculé | C | 51,1 | H | 7,04 | N | 12,8 |
| | Trouvé | C | 51,1 | H | 6,93 | N | 13,1 |
| Rendement : 15 % | | | | | P.F. : 129-130°C | | |

### Exemple 111

### H-(2,2-diméthylpropyl)-3-(1H-imidazol-4-yl)propyl carbamate

5 mmol d'acide 3,3-diméthylbutyrique, 5 mmol de triéthylamine et 5 mmol de phosphorazidate de diphényle sont traitées comme décrit dans l'exemple 110.
C₁₂H₂N₃O₂.C₂H₂O₄.0,5H₂O (338,4)

| | | | | | | |
|---|---|---|---|---|---|---|
| Analyse CHN | Calculé | C 49,7 | H | 7,15 | N | 12,4 |
| | Trouvé | C 49,8 | H | 6,81 | N | 12,3 |
| Rendement : 20 % | | | P.F. : 131-132°C | | | |

### Exemple 112

### 3-(1H-imidazol-4-yl)propyl-N-prop-2-ényl carbamate

5 mmol de 3-(1H-imidazol-4-yl)propanol.HCl et 5 mmol d'isocyanate de prop-2-ényle sont traitées comme décrit dans l'exemple 1.
C₁₀H₁₅N₃O₂.C₄H₄O (325,3)

| | | | | | | | |
|---|---|---|---|---|---|---|---|
| Analyse CHN | Calculé | C | 51,7 | H | 5,89 | N | 12,9 |
| | Trouvé | C | 51,5 | H | 5,84 | N | 12,8 |
| Rendement : 90 % | | | | P.F. : 88°C | | | |

### Exemple 113

### 3-(1H-imidazol-4-yl)propyl-N-(3-phénylpent-3-yl) carbamate

5 mmol d'acide 2-éthyl-2-phénylbutyrique, 5 mmol de triéthylamine et 5 mmol de phosphorazidate de diphényle sont traitées comme décrit dans l'exemple 110.
C₁₈H₂₅N₃O₂.C₂H₂O₄.0,5H₂O (414,5)

| | | | | | | | |
|---|---|---|---|---|---|---|---|
| Analyse CHN | Calculé | C | 58,0 | H | 6,81 | N | 10,1 |
| | Trouvé | C | 57,7 | H | 6,87 | N | 9,77 |
| Rendement : 35 % | | | | P.F. : 147-148°C | | | |

### Exemple 114

### N-(1,1-diphényléthyl)-3-(1H-imidazol-4-yl)propyl carbamate

5 mmol d'acide 2,2-diphénylpropionique, 5 mmol de triéthylamine et 5 mmol de phosphorazidate de diphényle sont traitées comme décrit dans l'exemple 110.
C₂₁H₂₃N₃O₂.C₂H₂O₄.0,25H₂O (444,0)

| | | | | | | | |
|---|---|---|---|---|---|---|---|
| Analyse CHN | Calculé | C | 62,2 | H | 5,79 | N | 9,46 |
| | Trouvé | C | 62,4 | H | 5,80 | N | 9,46 |
| Rendement : 45 % | | | | P.F. : 131-132°C | | | |

### Exemple 115

### N-(3,5-diméthylphényl)-3-(1H-imidazol-4-yl)propyl carbamate

5 mmol de 3-(1H-imidazol-4-yl)propanol.HCl et 5 mmol d'isocyanate de 3,5-diméthylphényle sont traitées comme décrit dans l'exemple 1.
C₁₅H₁₉N₃O₂.C₄H₄O₄.0,25H₂O (393,9)

| | | | | | | | |
|---|---|---|---|---|---|---|---|
| Analyse CHN | Calcule | C | 57,9 | H | 6,01 | N | 10,7 |
| | Trouvé | C | 57,9 | H | 6,22 | N | 10,6 |
| Rendement : 40 % | | | | P.F. : 107-108°C | | | |

### Exemple 116

### (1,1-diméthyléthyl)-2-(1H-imidazol-4-yl)éthyl éther

2 mmol de 2-(1-triphénylméthyl-1H-imidazol-4-yl)éthanol et 4 mmol de trichloroacétimidate de tert-butyle sont dissoutes dans 4 ml de cyclohexane et 2 ml de dichlorométhane. Après addition de 120 µl d'éthérate de trifluorure de bore, la solution est agitée à 60-70°C pendant 18 heures. La filtration du mélange réactionnel et l'évaporation du solvant sont suivies d'une détritylation dans 2 ml d'éthanol, 2 ml d'acétone et 15 ml d'HCl 2 N à 70°C, L'éthanol et l'acétone sont éliminés sous pression réduite et le triphénylméthanol est extrait avec de l'éther diéthylique. La couche aqueuse est alcalinisée avec de l'ammoniac et extraite avec de l'éther diéthylique. L'évaporation du solvant et la purification finale par chromatographie sur colonne (éluant : dichlorométhane/méthanol : 90/10) fournissent le produit sous forme d'une huile qui est cristallisée sous forme de l'hydrogénooxalate dans de l'éthanol et de l'éther diéthylique.
C₉H₁₆N₂O.0,8C₂H₂O₄ (240,3)

| | | | | | | | |
|---|---|---|---|---|---|---|---|
| Analyse CHN | Calculé | C | 53,0 | H | 7,38 | N | 11,7 |
| | Trouvé | C | 53,1 | H | 7,38 | N | 11,6 |
| Rendement : 15 % | | | | P.F. : 168°C | | | |

### Exemple 117

### (1,1-diméthyléthyl)-3-(1H-imidazol-4-yl)propyl éther

5 mmol de 3-(1-triphénylméthyl-1H-imidazol-4-yl)propanol et 10 mmol de trichloroacétimidate de tert-butyle sont traitées comme décrit dans l'exemple 116. Le composé du titre est cristallisé sous forme de l'hydrogénomaléate dans de l'éthanol et de l'éther diéthylique.
C₁₀H₁₈N₂O.C₄H₄O₄.0,25H₂O (302,8)

| | | | | | | | |
|---|---|---|---|---|---|---|---|
| Analyse CHN | Calculé | C | 55,5 | H | 7,49 | N | 9,25 |
| | Trouvé | C | 55,8 | H | 7,30 | N | 9,12 |
| Rendement : 15 % | | | | P.F. : 132°C | | | |

### Exemple 118

### 3-(1H-imidazol-4-yl)propyl prop-2-ényl éther

5 mmol de 3-(1-triphénylméthyl-1H-imidazol-4-yl)propanol et 5 mmol de 3-bromoprop-1-ène sont traitées comme décrit dans l'exemple 5. Le composé du titre est cristallisé sous forme de l'hydrogénooxalate à partir d'éthanol et d'éther diéthylique.
C₉H₁₄N₂O.0,8C₂H₂O₄ (238,3)

| | | | | | | | |
|---|---|---|---|---|---|---|---|
| Analyse CHN | Calculé | C | 53,4 | H | 6,60 | N | 11,8 |
| | Trouvé | C | 53,3 | H | 6,61 | N | 11,6 |
| Rendement : 20 % | | | | P.F. : 158-159°C | | | |

### Exemple 119

### 3-(1H-imidazol-4-yl)propyl-pent-4-ényl éther

5 mmol de 3-(1-triphénylméthyl-1H-imidazol-4-yl)propanol et 5 mmol de 5-bromopent-1-ène sont traitées comme décrit dans l'exemple 5. Le composé du titre est cristallisé sous forme de l'hydrogénooxalate à partir d'éthanol et d'éther diéthylique.
C₁₁H₁₈N₂O.C₂H₂O₄.0,75H₂O (297,8)

| | | | | | | | |
|---|---|---|---|---|---|---|---|
| Analyse CHN | Calculé | C | 52,4 | H | 7,28 | N | 9,41 |
| | Trouvé | C | 52,6 | H | 7,03 | N | 9,56 |
| Rendement : 15 % | | | | P.F. : 156°C | | | |

### Exemple 120

### 3-(1H-imidazol-4-yl)propyl-prop-2-ynyl éther

5 mmol de 3-(1-triphénylméthyl-1H-imidazol-4-yl)propanol et 5 mmol de 3-bromoprop-1-yne sont traitées comme décrit dans l'exemple 5. Le composé du titre est cristallisé sous forme de l'hydrogénooxalate à partir d'éthanol et d'éther diéthylique.
C₉H₁₂N₂O.0,75C₂H₂O₄ (231,7)

| | | | | | | | |
|---|---|---|---|---|---|---|---|
| Analyse CHN | Calculé | C | 54,4 | H | 5,87 | N | 12,1 |
| | Trouvé | C | 54,2 | H | 5,85 | N | 12,0 |
| Rendement : 20 % | | | | P.F. : 148°C | | | |

### Exemple 121

### (4-butylphényl)-3-(1H-imidazol-4-yl)propyl éther

0,8 mmol de cyclopropyl-(4-(3-(1H-imidazol-4-yl)propyloxy) phényl)cétone (exemple 61), 4 mmol d'hydrate d'hydrazine et 3,2 mmol de KOH dans 30 ml de triéthylèneglycol sont portées à reflux pendant 2 heures. Le solvant est évaporé sous pression réduite et le produit brut est extrait avec du dichlorométhane et purifié par chromatographie rotatoire (éluant : dichlorométhane/méthanol (99/1)-(90/10), atmosphère d'ammoniac). Le produit est cristallisé sous forme d'hydrogénomaléate à partir d'éther diéthylique et d'éthanol.
C₁₆H₂₂N₂O.0,75C₂H₂O₄ (325,9)

| | | | | | | | |
|---|---|---|---|---|---|---|---|
| Analyse CHN | Calculé | C | 64,5 | H | 7,27 | N | 8,60 |
| | Trouvé | C | 64,2 | H | 7,54 | | N 8,41 |
| Rendement : 20 % | | | | P.F. : 193°C | | | |

### Exemple 122

### (4-éth-1-ynylphényl)-3-(1H-imidazol-4-yl)propyl éther

10 mmol de 3-(1H-imidazol-4-yl)propanol sont mises à réagir avec 12 mmol de dicarbonate de di-tert-butyle dans 20 ml d'acétonitrile, 5 ml de triéthylamine et 5 ml d'H₂O en présence de 1 mmol de 4-(N,N-diméthylamino)pyridine à la température ordinaire pendant 2 heures. L'évaporation du solvant et la purification finale par chromatographie sur colonne (éluant : dichlorométhane/méthanol 90/10) fournissent l'ester tert-butylique de l'acide 4-(3-hydroxypropyl)-1H-imidazole-1-carboxylique sous forme d'une huile.

Du 4-(2-(triméthylsilyl)éth-1-ynyl)phénol est préparé à partir de 4-iodoanisole selon Sonogashira K. et coll., Tetrahedron Lett. 1975, 50, 4467 et Feutrill, G.I. et coll., ibid, 1970, 16, 1327.

5 mmol de ce dernier composé sont mises à réagir avec 5 mmol du premier comme décrit dans l'exemple 56.
C₁₄H₁₄N₂O.C₄H₄O₄.0,25H₂O (346,9)

| | | | | | | | |
|---|---|---|---|---|---|---|---|
| Analyse CHN | Calculé | C | 62,3 | H | 5,38 | N | 8,08 |
| | Trouvé | C | 62,4 | H | 5,28 | N | 8,07 |
| Rendement : 70 % | | | | P.F. : 150°C | | | |

### Exemple 123

### 3-(1H-imidazol-4-yl)propyl-(4-pent-1-ynylphényl) éther

5 mmol d'ester tert-butylique de l'acide 4-(3-hydroxypropyl)-1H-imidazole-1-carboxylique et 5 mmol de 4-pent-1-ynylphénol sont traitées comme décrit dans l'exemple 122.
C₁₇H₂₀N₂O.C₄H₄O₄.0,25H₂O (388,9)

| | | | | | | | |
|---|---|---|---|---|---|---|---|
| Analyse CHN | Calculé | C | 64,9 | H | 6,35 | N | 7,20 |
| | Trouvé | C | 64,9 | H | 6,54 | N | 7,22 |
| Rendement : 85 % | | | | P.F. : 126°C | | | |

### Exemple 124

### (4-(3,3-diméthylbut-1-ynyl)phényl)-3-(1H-imidazol-4-yl)propyl éther

5 mmol d'ester tert-butylique de l'acide 4-(3-hydroxypropyl)-1H-imidazole-1-carboxylique et 5 mmol de 4-(3,3-diméthylbut-1-ynyl)phénol sont traitées comme décrit dans l'exemple 122.
C₁₈H₂₂N₂O.C₄H₄O₄.0,5H₂O (407,5)

| | | | | | | | |
|---|---|---|---|---|---|---|---|
| Analyse CHN | Calculé | C | 64,9 | H | 6,68 | N | 6,87 |
| | Trouvé | C | 64,9 | H | 6,67 | N | 6,64 |
| Rendement : 95 % | | | | P.F. : 132°C | | | |

### Exemple 125

### (4-fluorophényl)-4-(3-(1H-imidazol-4-yl)propyloxy) butanone

5 mmol de 3-(1-triphénylméthyl-1H-imidazol-4-yl)propanolate de sodium et 5 mmol de 2-(3-chloropropyl)-2-(4-fluorophényl)-1,3-dioxolanne sont traitées comme décrit dans l'exemple 5, mais le solvant de clivage est constitué de 30 ml d'HCl/H₂SO₄ (1/1) et l'huile est cristallisée sous forme d'hydrogénooxalate à partir d'éther diéthylique et d'éthanol.
C₁₆H₁₉N₂O₂F.C₂H₂O₄.0,25H₂O (384,9)

| | | | | | | | |
|---|---|---|---|---|---|---|---|
| Analyse CHN | Calculé | C | 56,2 | H | 5,63 | N | 7,28 |
| | Trouvé | C | 55,9 | H | 5,67 | N | 7,39 |
| Rendement : 30 % | | | | P.F. : 119°C | | | |

### Exemple 126

### Cyclopropyl-(4-(2-(1H-imidazol-4-yl)éthyloxy)phényl) cétone

5 mmol de 2-(1-triphénylméthyl-1H-imidazol-4-yl)éthanolate de sodium et 10 mmol de cyclopropyl-(4-fluorophényl)cétorie sont traitées comme décrit dans l'exemple 61 (procédé A).
C₁₅H₁₆N₂O₂.C₄H₄O₄ (372,4)

| | | | | | | | |
|---|---|---|---|---|---|---|---|
| Analyse CHN | Calculé | C | 61,3 | H | 5,41 | N | 7,52 |
| | Trouvé | C | 61,3 | H | 5,32 | N | 7,43 |
| Rendement : 20 % | | | | P.F. : 133°C | | | |

### Exemple 127

### (4-(3-(1H-imidazol-4-yl)propyloxy)phényl)pentanone

5 mmol de 3-(1-triphénylméthyl-1H-imidazol-4-yl)propanol et 10 mmol de cyclopropyl-(4-fluorophényl)cétone sont traitées comme décrit dans l'exemple 61 (procédé A).
C₁₇H₂₂N₂O₂.C₄H₄O₄.0,5H₂O (411,5)

| | | | | | | | |
|---|---|---|---|---|---|---|---|
| Analyse CHN | Calculé | C | 61,3 | H | 6,61 | N | 6,81 |
| | Trouvé | C | 60,9 | H | 6,40 | N | 6,55 |
| Rendement : 80 % | | | | P.F. : 85°C | | | |

### Exemple 128

### (4-(3-(1H-imidazol-4-yl)propyloxy)phényl)hexanone

5 mmol de 3-(1-triphénylméthyl-1H-imidazol-4-yl)propanol et 6 mmol de (4-hydroxyphényl)hexanone (préparée à partir d'acide hexanoïque selon des procédés standards (acylation de Friedel-Crafts)) sont traitées comme décrit dans l'exemple 56.
C₁₈H₂₄N₂O₂.C₄H₄O₄ (416,5)

| | | | | | | | |
|---|---|---|---|---|---|---|---|
| Analyse CHN | Calculé | C | 63,4 | H | 6,78 | N | 6,73 |
| | Trouvé | C | 63,2 | H | 6,76 | N | 6,70 |
| Rendement : 70 % | | | | P.F. : 114°C | | | |

### Exemple 129

### 3,3-diméthyl-(4-(3-(1H-imidazol-4-yl)propyloxy)phényl)butanone

5 mmol de 3-(1-triphénylméthyl-1H-imidazol-4-yl)propanol et 5 mmol de 4-(3,3-diméthyl-but-1-ynyl)phénol sont traitées comme décrit dans l'exemple 56. La détritylation dans de l'HCl 2 N fournit le composé du titre. C₁₈H₂₄N₂O₂.C₄H₄O₄ (416,5)

| | | | | | | | |
|---|---|---|---|---|---|---|---|
| Analyse CHN | Calculé | C | 63,5 | H | 6,78 | N | 6,73 |
| | Trouvé | C | 63,3 | H | 6,88 | N | 6,63 |
| Rendement : 60 % | | | | P.F. : 139°C | | | |

### Exemple 130

### 4-hydroxy-(4-(3-(1H-imidazol-4-yl)propyloxy)phényl)butanone

5 mmol de 3-(1-triphénylméthyl-1H-imidazol-4-yl)propanolate de sodium et 10 mmol d'ester diéthylique de l'acide 2-(4-(4-fluorophényl)-4-oxobutyl)malonique (préparé à partir du 2-(3-chloropropyl)-2-(4-fluorophényl)-1,3-dioxolanne avec l'acide malonique selon des procédés standards) sont traitées comme décrit dans l'exemple 61 (procédé A). C₁₆H₂₀N₂O₃.C₄H₄O₄ (404,4)

| | | | | | | | |
|---|---|---|---|---|---|---|---|
| Analyse,CHN | Calculé | C | 59,4 | H | 5,98 | N | 6,93 |
| | Trouvé | C | 59,1 | H | 6,08 | N | 7,01 |
| Rendement : 20 % | | | | P.F. : 107°C | | | |

### Exemple 131

### 4-hydroxy-(4-(3-(1H-imidazol-4-yl)propyloxy)phényl)butanoneéthylène-acétal

1,3 mmol de cyclopropyl-(4-(3-(1H-imidazol-4-yl)propyloxy) phényl)cétone (exemple 61) et une quantité catalytique d'acide 4-toluènesulfonique dans 15 ml d'éthylèneglycol sont portées à reflux pendant 5 heures. Le solvant est évaporé sous pression réduite, le résidu est dissous dans 10 ml d'H₂O et alcalinisé avec de l'ammoniac.
Le produit brut est extrait avec du dichlorométhane et purifié par chromatographie rotatoire (éluant : dichlorométhane/méthanol (99/1-90/10), atmosphère d'ammoniac). Le produit est cristallisé sous forme d'hydrogénomaléate à partir d'éther diéthylique et d'éthanol.
C₁₈H₂₄N₂O₄.C₄H₄O₄ (448,5)

| | | | | | | | |
|---|---|---|---|---|---|---|---|
| Analyse CHN | Calculé | C | 58,9 | H | 6,29 | N | 6,25 |
| | Trouvé | C | 59,0 | H | 6,32 | N | 6,19 |
| Rendement : 55 % | | | | P.F. : 104°C | | | |

### Exemple 132

### 5-(3-(1H-imidazol-4yl)propyloxy)indan-1-one

On traite 5 mmol de 3-(1-triphénylméthyl-1H-imidazol-4-yl) propanol et 6 mmol de 5-hydroxyindan-1-one comme décrit dans l'exemple 56.
C₁₅H₁₆N₂O₂.C₄H₄O₄.H₂O (390,4)

| | | | | | | | |
|---|---|---|---|---|---|---|---|
| Analyse CHN | Calculé | C | 58,5 | H | 5,68 | N | 7,17 |
| | Trouvé | C | 58,8 | H | 5,87 | N | 7,30 |
| Rendement : 70 % | | | | P.F. : 144°C | | | |

### Exemple 133

### 3,4-dihydro-6-(3-(1H-imidazol-4-yl)propyloxy)-2H-naphtalen-1-one

5 mmol de 3-(1-triphénylméthyl-1H-imidazol-4-yl)propanol et 6 mmol de 3,4-dihydro-6-hydroxy-2H-naphtalen-1-one sont traitées comme décrit dans l'exemple 56.
C₁₆H₁₈N₂O₂.C₄H₄O₄.0,5H₂O (395,4)

| | | | | | | | |
|---|---|---|---|---|---|---|---|
| Analyse CHN | Calculé | C | 60,8 | H | 5,86 | N | 7,08 |
| | Trouvé | C | 60,5 | H | 5,72 | N | 7,15 |
| Rendement : 40 % | | | | P.F. : 100-102°C | | | |

### Exemple 134

### (4-(3-(1H-imidazol-4-yl)propyloxy)-2-méthylphényl)éthanone

5 mmol de 3-(1-triphényiméthyl-1H-imidazol-4-yl)propanol et 6 mmol de (4-hydroxy-2-méthylphényl)éthanone sont traitées comme décrit dans l'exemple 56, mais isolées sous forme de la base libre
C₁₅H₁₈N₂O₂ (258,3)

| | | | | | | | |
|---|---|---|---|---|---|---|---|
| Analyse CHN | Calculé | C | 69,7 | H | 7,02 | N | 10,8 |
| | Trouvé | C | 69,4 | H | 7,07 | N | 10,5 |
| Rendement : 70 % | | | | P.F. : 143°C | | | |

### Exemple 135

### (2-fluoro-4-(3-(1H-imidazol-4-yl)propyloxy)phényl)éthanone

5 mmol de 3-(1-triphénylméthyl-1H-imidazol-4-yl)propanol et 6 mmol de (2-fluoro-4-hydroxyphényl)éthanone sont traitées comme décrit dans l'exemple 56.
C₁₄H₁₅N₂O₂F.C₄H₄O.0,25H₂O (382,9)

| | | | | | | | |
|---|---|---|---|---|---|---|---|
| Analyse CHN | Calculé | C | 56,5 | H | 5,13 | N | 7,32 |
| | Trouvé | C | 56,4 | H | 5,27 | N | 7,20 |
| Rendement : 70% | | | | P.F. : 115°C | | | |

### Exemple 136

### (2-fluoro-4-(3-(1H-imidazol-4-yl)propyloxy)phényl)propanone

5 mmol de 3-(1-triphénylméthyl-1H-imidazol-4-yl)propanolate de sodium et 10 mmol de (2,4-difluorophényl)propanone sont traitées comme décrit dans l'exemple 61 (procédé A).
C₁₅H₁₇N₂O₂F.C₄H₄O₄.0,25H₂O (396,9)

| | | | | | | | |
|---|---|---|---|---|---|---|---|
| Analyse CHN | Calculé | C | 57,5 | H | 5,46 | N | 7,06 |
| | Trouvé | C | 57,7 | H | 5,55 | N | 7,06 |
| Rendement : 30 % | | | | P.F. : 122°C | | | |

### Exemple 137

### (4-(3-(1H-imidazol-4-yl)propyloxy)phényl)-2-thiényl-cétone

5 mmol de 3-(1-triphénylméthyl-1H-imidazol-4-yl)propanolate de sodium et 10 mmol de (4-fluorophényl)-2-thiényl-cétone sont traitées comme décrit dans l'exemple 61 (procédé A).
C₁₇H₁₆N₂O₂S.C₂H₂O₄ (402,4)

| | | | | | | | |
|---|---|---|---|---|---|---|---|
| Analyse CHN | Calculé | C | 56,7 | H | 4,51 | N | 6,96 |
| | Trouvé | C | 56,8. | H | 4,57 | N | 6,97 |
| Rendement : 10 % | | | | P.F. : 174°C | | | |

### Exemple 138

### (4-(3-(1H-imidazol-4-yl)propyloxy)phényl)carbaldéhyde-oxime

1,2 mmol de (4-(3-(1H-imidazol-4-yl)propyloxy)phényl) carbaldéhyde (exemple 57) et 2,4 mmol de chlorhydrate d'hydroxylamine dans 20 ml d'éthanol anhydre sont chauffées à reflux pendant 3 heures. Le mélange est concentré sous pression réduite, alcalinisé avec une solution saturée de K₂CO₃ et le produit brut est isolé et lavé à l'eau. Le produit est cristallisé sous forme de l'hydrogénomaléate à partir d'éther diéthylique et d'éthanol.
C₁₃H₁₅N₃O₂.C₄H₄O₄. 0,5H₂O (370,4)

| | | | | | | | |
|---|---|---|---|---|---|---|---|
| Analyse CHN | Calculé | C | 55,1 | H | 5,44 | N | 11,4 |
| | Trouvé | C | 54,8 | H | 5,27 | N | 11,0 |
| Rendement : 70 % | | | | P.F. : 154°C | | | |

### Exemple 139

### (4-(3-(1H-imidazol-4-yl)propyloxy)phényl)propanone-oxime

1,2 mmol de (4-(3-(1-imidazol-4-yl)propyloxy)phényl) propanone (exemple 59) est traitée comme décrit dans l'exemple 138.
C₁₅H₁₉N₃O.C₄H₄O₄.0,5H₂O (398,4)

| | | | | | | | |
|---|---|---|---|---|---|---|---|
| Analyse CHN | Calculé | C | 57,3 | H | 6,07 | N | 10,6 |
| | Trouvé | C | 57,3 | H | 6,25 | N | 10,7 |
| Rendement : 80 % | | | | P.F. : 114°C | | | |

### Exemple 140

### (4-(3-(1H-imidazol-4-yl)propyloxy)phényl)butanone-oxime

1,2 mmol de (4-(3-(1H-imidazol-4-yl)propyloxy)phényl)butanone (exemple 71) est traitée comme décrit dans l'exemple 138.
C₁₆H₂₁N₃O₂.C₄H₄O₄.0,25H₂O (407,9)

| | | | | | | | |
|---|---|---|---|---|---|---|---|
| Analyse CHN | Calculé | C | 58,9 | H | 6,30 | N | 10,3 |
| | Trouvé | C | 58,7 | H | 6,61 | N | 10,4 |
| Rendement : 80 % | | | | P.F. : 129°C | | | |

### Exemple 141

### (4-(3-(1H-imidazol-4-yl)propyloxy)phényl)pentanone-oxime

1,2 mmol de (4-(3-(1H-imidazol-4-yl)propyloxy)phényl) pentanone (exemple 127) est traitée comme décrit dans l'exemple 138, mais isolée sous forme de la base libre.
C₁₇H₂₃N₃O₂.0,25H₂O (305,9)

| | | | | | | | |
|---|---|---|---|---|---|---|---|
| Analyse CHN | Calculé | C | 66,8 | H | 7,74 | N | 13,7 |
| | Trouvé | C | 66,6 | H | 7,82 | N | 13,7 |
| Rendement : 80 % | | | | P.F. : 131°C | | | |

### Exemple 142

### (4-(3-(1H-imidazol-4-yl)propyloxy)phényl)hexanone-oxime

1,2 mmol de (4-(3-(1H-imidazol-4-yl)propyloxy)phényl)hexanone (exemple 128) est traitée comme décrit dans l'exemple 138, mais isolée sous forme de la base libre.
C₁₈H₂₅N₃O₂.0,5H₂O (324,4)

| | | | | | | | |
|---|---|---|---|---|---|---|---|
| Analyse CHN | Calculé | C | 66,6 | H | 8,08 | N | 13,0 |
| | Trouvé | C | 66,4 | H | 7,81 | N | 12,8 |
| Rendement : 80 % | | | | P.F. : 138°C | | | |

### Exemple 143

### Cyclopropyl-(4-(3-(1H-imidazol-4-yl)propyloxy)phényl)cétone-oxime

1,2 mmol de cyclopropyl(4-(3-(1H-imidazol-4-yl)propyloxy) phényl)cétone (exemple 61) et 2,4 mmol de chlorhydrate d'hydroxylamine dans 20 ml d'éthanol anhydre sont chauffées à 60°C pendant 5 heures. Le mélange est traité comme décrit dans l'exemple 138, mais isolé sous forme de la base libre.
C₁₆H₁₉N₃O₂.0,25H₂O (289,9)

| | | | | | | | |
|---|---|---|---|---|---|---|---|
| Analyse CHN | Calculé | C | 66,3 | H | 6,78 | N | 14,5 |
| | Trouvé | C | 66,6 | H | 6,62 | N | 14,6 |
| Rendement : 55 % | | | | P.F. : 213°C | | | |

### Exemple 144

### (4-(3-(1H-imidazol-4-yl)propyloxy)-2-méthylphényl)éthanone-oxime

1,2 mmol de (4-(3-(1H-imidazol-4-yl)propyloxy)-2-méthylphényl)éthanone (exemple 134) est traitée comme décrit dans l'exemple 138, mais isolée sous forme de la base libre.
C₁₅H₁₉N₃O₂ (273,3)

| | | | | | | | |
|---|---|---|---|---|---|---|---|
| Analyse CHN | Calculé | C | 65,9 | H | 7,01 | N | 15,4 |
| | Trouvé | C | 65,6 | H | 7,06 | N | 15,2 |
| Rendement : 80 % | | | | P.F. : 190°C | | | |

### Exemple 145

### (2-fluoro-4-(3-(1H-imidazol-4-yl)propyloxy)phényl)éthanone-oxime

1,2 mmol de (2-fluoro-4-(3-(1H-imidazol-4-yl)propyloxy) phényl)éthanone (exemple 135) est traitée comme décrit dans l'exemple 138.
C₁₄H₁₆N₃O₂F.C₄H₄O₄ (393,4)

| | | | | | | | |
|---|---|---|---|---|---|---|---|
| Analyse CHN | Calculé | C | 55,0 | H | 5,12 | N | 10,7 |
| | Trouvé | C | 54,9 | H | 5,40 | N | 10,7 |
| Rendement : 70 % | | | | P.F. : 134°C | | | |

### Exemple 146

### (4-(3-(1H-imidazol-4-yl)propyloxy)phényl)carbaldéhyde-O-méthyloxime

1,2 mmol de (4-(3-(1H-imidazol-4-yl)propyloxy)phényl) carbaldéhyde (exemple 57) et 2,4 mmol de chlorhydrate d'O-méthylhydroxylamine sont traitées comme décrit dans l'exemple 138.
C₁₄H₁₇N₃O₂H₄C₄H₄O₄.0,25H₂O (379,9)

| | | | | | | | |
|---|---|---|---|---|---|---|---|
| Analyse CHN | Calculé | C | 56,9 | H | 5,70 | N | 11,1. |
| | Trouvé | C | 56,7 | H | 5,54 | N | 11,0 |
| Rendement : 85 | % | | | P.F. : 131-132°C | | | |

### Exemple 147

### (4-(3-(1H-imidazol-4-yl)propyloxy)phényl)éthanone-semicarbazone

1,2 mmol de (4-(3-(1H-imidazol-4-yl)propyloxy)phényl)éthanone (exemple 58) et 2,4 mmol de chlorhydrate de semicarbazide sont traitées comme décrit dans l'exemple 138.
C₁₅H₁₉N₅O₂.C₄H₄O₄.0,5H₂O (426,4)

| | | | | | | | |
|---|---|---|---|---|---|---|---|
| Analyse CHN | Calculé | C | 53,5 | H | 5,67 | N | 16,4 |
| | Trouvé | C | 53,5 | H | 5,62 | N | 16,4 |
| Rendement : 85 % | | | | P.F. : 159°C | | | |

### Exemple 148

### 6-(3-(1H-imidazol-4-yl)propyloxy)-2H-1,3-benzoxathiol-2-one

5 mmol de 3-(1-triphénylméthyl-1H-imidazol-4-yl)propanol et 6 mmol de 6-hydroxy-2H-1,3-benzoxathiol-2-one sont traitées comme décrit dans l'exemple 56.
C₁₃H₁₂N₂O₃S.C₄H₄O₄.1,75H₂O (423,9)

| | | | | | | | |
|---|---|---|---|---|---|---|---|
| Analyse CHN | Calculé | C | 48,2 | H | 4,64 | N | 6,61 |
| | Trouvé | C | 48,0 | H | 4,48 | N | 6,38 |
| Rendement : 40 % | | | | P.F. : 147°C | | | |

### Exemple 149

### 3-(1H-imidazol-4-yl)propyl-(4-(5-méthyl-1,2,4-oxadiazol-3-yl)phényl) éther

5 mmol de 3-(1-triphénylméthyl-1H-imidazol-4-yl)propanol et 6 mmol de (5-méthyl-1,2,4-oxadiazol-3-yl)phénol (préparé selon Swain, C. J. et coll. J. Med. Chem. 1991, 34, 140) sont traitées comme décrit dans l'exemple 56, mais isolées sous forme de la base libre.
C₁₅H₁₆N₄O₂.H₂O (302,3)

| | | | | | | | |
|---|---|---|---|---|---|---|---|
| Analyse CHN | Calculé | C | 59,6 | H | 6,00 | N | 18,5 |
| | Trouvé | C | 59,8 | H | 5,84 | N | 18,2 |
| Rendement : 60 % | | | | P.F. : 146°C | | | |

### Exemple 150

### (4-fluorophényl)(4-(3-(1H-imidazol-4-yl)propyloxy)phényl)sulfone

5 mmol de 3-(1-triphénylméthyl-1H-imidazol-4-yl)propanolate de sodium et 10 mmol de bis(4-fluorophényl)sulfone sont traitées comme décrit dans l'exemple 61 (procédé A).
C₁₈H₁₇N₂O₃FS.HCl (396,9)

| | | | | | | | |
|---|---|---|---|---|---|---|---|
| Analyse CHN | Calculé | C | 54,5 | H | 4,57 | N | 7,06 |
| | Trouvé | C | 54,7 | H | 4,67 | N | 6,69 |
| Rendement : 40 % | | | | P.F. : 238°C | | | |

### Exemple 151

### 4-(3-(1H-imidazol-4-yl)propyloxy)phényl-3-phényl prop-2-énone

5 mmol de 3-(1-triphénylméthyl-1H-imidazol-4-yl)propanol et 6 mmol de (4-hydroxyphényl)-3-phényl prop-2-énone (préparée selon Shriner, R.L. et coll., J. Am. Chem. Soc., 1930, 52, 2538) sont traitées comme décrit dans l'exemple 56, mais cristallisées sous forme d'hydrogénooxalate à partir d'éther diéthylique et d'éthanol.
C₂₁H₂₀N₂O₂.C₂H₂O₄.0,75H₂O (435,9)

| | | | | | | | |
|---|---|---|---|---|---|---|---|
| Analyse CHN | Calculé | C | 63,4 | H | 5,43 | N | 6,43 |
| | Trouvé | C | 63,6 | H | 5,83 | N | 6,54 |
| Rendement : 70 % | | | | P.F. : 170°C | | | |

### Exemple 152

### 4-(3-(1H-imidazol-4-yl)propyloxy)phényl)heptanone

5 mmol de 3-(1-triphénylméthyl-1H-imidazol-4-yl)propanol et 6 mmol de (4-hydroxyphényl)heptanone (préparée à partir d'acide heptanoïque selon des procédés standards (acylation de Friedel-Crafts)) sont traitées comme décrit dans l'exemple 56.
C₁₉H₂₆N₂O₂.C₄H₄O₄.0,25H₂O (435,0)

| | | | | | | | |
|---|---|---|---|---|---|---|---|
| Analyse CHN | Calculé | C | 63,5 | H | 7,06 | N | 6,44 |
| | Trouvé | C | 63,3 | H | 7,04 | N | 6,30 |
| Rendement : 70 % | | | | P.F. : 119°C | | | |

### Exemple 153

### 4-(3-(1H-imidazol-4-yl)propyloxy)phényl-2-phényl éthanone

5 mmol de 3-(1-triphénylméthyl-1H-imidazol-4-yl)propanol et 6 mmol de (4-hydroxyphényl)-2-phényl éthanone sont traitées comme décrit dans l'exemple 56, mais cristallisées sous forme d'hydrogéno-oxalate à partir d'éther diéthylique et d'éthanol.
C₂₀H₂₀N₂O₂.C₂H₂O₄.0,75H₂O (423,9)

| | | | | | | | |
|---|---|---|---|---|---|---|---|
| Analyse CHN | Calculé | C | 62,3 | H | 5,59 | N | 6,61 |
| | Trouvé | C | 62,3 | H | 5,45 | N | 6,48 |
| Rendement : 65 % | | | | P.F. : 185°C | | | |

### Exemple 154

### Oxalate de 4-[3-(3-trifluorométhylphénylamino)propyl]-1H-imidazole

Un mélange de 1 g (2,72 mmol) de 1-(triphénylméthyl)-4-[3-hydroxypropyl]imidazole, 0,55 g (4,07 mmol ; 1,5 équivalent) d'oxyde de morpholine et 1,36 g de tamis moléculaire de 4 Å en poudre dans un mélange anhydre d'acétonitrile et de dichlorométhane (10/4) est agité à la température ordinaire sous azote. 0,047 g (0,135 mmol ; 5 % molaires) de perruthénate(VII) de tétrapropylammonium est ajouté en une seule portion et le mélange est agité à la température ordinaire pendant 48 heures. Le mélange réactionnel est filtré sur gel de silice (préchargé avec de l'acétate d'éthyle) et le filtrat est évaporé sous pression réduite. L'huile obtenue est purifiée par chromatographie sur une colonne de gel de silice avec de l'éther diéthylique comme éluant pour fournir le 3-(1-triphénylméthylimidazol-4-yl)-propionaldéhyde.

0,5 g (1,36 mmol) de l'aldéhyde ci-dessus est chauffé avec 0,22 g (1,36 mmol) de 3-trifluorométhylaniline dans 50 ml de toluène anhydre à 50°C pendant 30 minutes. Le solvant est chassé sous pression réduite pour laisser 0,6 g (86 %) d'une huile qui est dissoute dans le méthanol, refroidie à 0°C, puis traitée avec 1,06 g (0,027 mole ; 20 équivalents) de borohydrure de sodium ajouté lentement à 0°C. Le mélange est agité à la température ordinaire pendant une nuit, puis le solvant est chassé sous pression réduite, 20 ml d'eau sont ajoutés et le mélange est extrait avec du chloroforme. Les extraits chloroformiques sont séchés (MgSO₄) et le solvant est évaporé sous pression réduite pour laisser une huile qui est purifiée par chromatographie sur une colonne de gel de silice (éluant : éther diéthylique) pour fournir 0,4 g de 1-triphénylméthyl-4-[3-(3-trifluorométhylphénylamino)propyl]imidazole sous forme d'une huile incolore. Cette dernière (0,35 g ; 6,85 mmol) dans 8 ml de tétrahydrofuranne et 12 ml d'HCl 2 M est chauffée à 80°C pendant 5 heures. Le tétrahydrofuranne est évaporé sous pression réduite et le Ph₃COH est extrait avec de l'éther diéthylique. La couche aqueuse est neutralisée avec du carbonate de potassium et le produit est extrait dans du chloroforme. La solution chloroformique est séchée et évaporée pour fournir une huile brune qui est purifiée par chromatographie sur une colonne de gel de silice avec comme éluant un mélange d'acétate d'éthyle/méthanol (5/1). L'huile obtenue est dissoute dans 4 ml de 2-propanol, traitée avec une solution de 1,5 équivalent d'acide oxalique dans 3 ml de 2-propanol et le mélange est refroidi pendant 4 heures. Le précipité qui est formé par addition d'éther diéthylique est recueilli et lavé avec de l'éther pour fournir l'oxalate désiré sous forme d'un solide blanc,P.F. 150-151°C.
C₁₃H₁₄F₃H₃.1,5C₂H₂O₄ :

| | | | | | | | |
|---|---|---|---|---|---|---|---|
| Analyse CHN | Calculé | C | 47,18 | H | 3,76 | N | 9,96 |
| | Trouvé | C | 47,08 | H | 4,15 | N | 10,17 |

### Exemple 155

### Oxalate de 4-[3-(3-éthanoylphénylamino)propyl]-1H-imidazole

Un mélange de 4 g (0,029 mole) de 3-aminoacétophénone et 2,47 ml (0,044 mole ; 1,5 équivalent) d'éthylèneglycol et une petite quantité d'acide toluène-4-sulfonique dans 60 ml de benzène anhydre sont chauffés à reflux avec élimination azéotropique de l'eau pendant 4 heures. La solution est mise à refroidir, lavée successivement avec du carbonate de sodium aqueux à 20 % et de l'eau, puis séchée (MgSO₄). Le solvant est évaporé sous pression réduite pour laisser une huile jaune qui est purifiée par chromatographie sur une colonne de gel de silice en utilisant comme éluant un mélange d'éther diéthylique/éther de pétrole (1/1), puis cristallisé dans l'hexane pour fournir la 3-(2-méthyl-1,3-dioxolan-2-yl)aniline, P.F. 75-77°C.

Cette dernière (0,096 g ; 1,64 mmol) est chauffée avec 0,6 g (1,64 mmol) de 3-(1-(triphénylméthyl)imidazol-4-yl]propionaldéhyde dans 50 ml de toluène anhydre à 50°C pendant 30 minutes. Le solvant est éliminé sous pression réduite pour laisser une huile qui est réduite avec 0,064 g (0,016 mol) de borohydrure de sodium dans le méthanol comme décrit pour l'exemple 154 pour fournir la N-{3-[1-(triphénylméthyl)imidazol-4-yl]propyl}-3-(2-méthyl-1,3-dioxolan-2-yl)aniline sous forme d'une huile incolore. Cette dernière (0,5 g ; 0,95 mmol) est chauffée avec 12 ml d'HCl 2 M dans 8 ml de tétrahydrofuranne à 80°C pendant 5 heures. Le tétrahydrofuranne est évaporé sous pression réduite et le Ph₃COH est extrait dans l'éther diéthylique. La couche aqueuse est neutralisée avec du carbonate de potassium et le produit est extrait dans le chloroforme. La solution chloroformique est séchée et évaporée pour fournir une huile brune qui est purifiée par chromatographie sur une colonne de gel de silice (éluant : acétate d'éthyle/méthanol : 5/1) puis traitée avec de l'acide oxalique dans le 2-propanol comme décrit pour l'exemple 154 pour fournir l'oxalate désiré, P.F. 152-154°C, après recristallisation dans l'éthanol.
C₁₄H₁₇N₃O.C₂H₂O₄.0,1H₂O :

| | | | | | | | |
|---|---|---|---|---|---|---|---|
| Analyse CHN | Calculé | C | 57,34 | H | 5,77 | N | 12,54 |
| | Trouvé | C | 57,18 | H | 5,78 | N | 12,39 |

### Exemple 156

### Oxalate de 4-[3-(3-éthylphénylamino)propyl)-1H-imidazole

0,4 g (1,10 mmol) de 3-(1-triphénylméthylimidazol-4-yl) propionaldéhyde est traité avec 0,13 g (1,10 mmol) de 3-éthylaniline dans le toluène anhydre, puis réduit comme décrit pour l'exemple 154 pour fournir le 1-triphénylméthyl-4-[3-(3-éthylphénylamino)propyl] imidazole sous forme d'une huile incolore. Ce dernier est déprotégé avec HCl dans le tétrahydrofuranne et l'huile obtenue est transformée en oxalate comme décrit pour l'exemple 154 pour fournir le produit, P.F. 189-191°C après recristallisation dans un mélange de 2-propanol et d'éther diéthylique.
C₁₄H₁₉N₃.1,5C₂H₂O₄.0,4H₂O :

| | | | | | | | |
|---|---|---|---|---|---|---|---|
| Analyse CHN | Calculé | C | 54,95 | H | 6,18 | N | 11,13 |
| | Trouvé | C | 55,03 | H | 5,80 | N | 10,77 |

### Exemple 157

### Oxalate de 4-[2-(3-éthanoylphénylthio)éthyl]-1H-imidazole

0,223 g (1,53 mmol) de 3-éthanoylthiophénol est dissous dans 20 ml de diméthylformamide anhydre et refroidi dans la glace au-dessous de 4°C sous une atmosphère d'azote. 0,153 g (3,83 mmol) d'hydrure de sodium est ajouté en petites portions au-dessous de 5°C. Après 10 minutes, le mélange est réchauffé à la température ordinaire. 0.128 g (0.76 mmol) de chlorhydrate de 4-(2-chloroéthyl)-1H-imidazole et 10 mg de catalyseur constitué d'iodure de tétra-n-butylammonium sont ajoutés et le mélange est chauffé à 80°C pendant 3 jours. Le solvant est éliminé sous pression réduite pour fournir une gomme brune qui est dissoute dans 50-60 ml d'HCl à 10 %, lavée 4 fois avec 40 ml d'éther, alcalinisée avec du carbonate de potassium solide à pH 7-8, puis extraite 3 fois avec 40 ml de dichlorométhane. Les extraits combinés sont évaporés et l'huile obtenue est purifiée par chromatographie sur une colonne de gel de silice avec élution avec un mélange d'acétate d'éthyle/méthanol (95/5), puis, après avoir été extraite dans le 2-propanol froid, est transformée en l'oxalate dans l'éthanol pour fournir un solide cristallin blanc, P.F. 138-140°C.
C₁₃H₁₄N₂OS.0,95C₂H₂O₄.0,1C₃H₇OH :

| | | | | | | | |
|---|---|---|---|---|---|---|---|
| Analyse CHN | Calculé | C | 54,03 | H | 4,98 | N | 8,29 |
| | Trouvé | C | 54,25 | H | 4,99 | N | 7,83 |

### Exemple 158

### Oxalate de 4-[3-(3-(1-hydroxyiminoéthyl)phénoxy)propyl]-1H-imidamole

Une solution de 0,125 g (1,81 mmol) de chlorhydrate d'hydroxylamine et 0,5 g (0,006 mole) d'acétate de sodium dans 10 ml d'eau est agitée pendant 10 minutes, puis une solution de 0,125 g (0,512 mmol) de 4-[3-(3-éthanoylphénoxy)propyl]-1H-imidazole dans 3 ml d'éthanol est ajoutée lentement. Le mélange est agité à la température ordinaire pendant 1 heure, puis chauffé à 80°C pendant 2 heures. Après refroidissement, le solvant est éliminé sous pression réduite et le résidu blanc obtenu est extrait avec du chloroforme. Le chloroforme est séché (MgSO₄), puis évaporé. L'huile obtenue est dissoute dans 4 ml de 2-propanol et traitée avec 1,3 équivalent d'acide oxalique dans 3 ml de 2-propanol. L'addition d'éther diéthylique fournit le produit qui est séparé par filtration, lavé à l'éther et recristallisé dans un mélange de 2-propanol/éther diéthylique, P.F. 149-151°C.
C₁₄H₁₇N₃O₂.C₂H₂O₄:

| | | | | | | | |
|---|---|---|---|---|---|---|---|
| Analyse CHN | Calculé | C | 55,01 | H | 5,45 | N | 12,03 |
| | Trouvé | C | 54,87 | H | 5,01 | N | 12,16 |

### Exemple 159

### Oxalate de 4-[2-(3-trifluorométhylphénylthio)propyl]-1H-imidazole

5,426 g (18,7 mmol) de 1-(N,N-diméthylsulfamoyl)-2-tert-butyldiméthylsilyl-imidazole sont dissous dans 100 ml de THF fraîchement distillé sous azote, refroidis à -78°C et une solution de n-butyllithium dans l'hexane (2,5 M ; 15 ml ; 37,5 mmol) est ajoutée goutte à goutte en une période de 10 min. Le mélange est agité pendant 30 min à -78°C. La solution est réchauffée à O°C avec agitation rapide et une solution de 3,0 ml (2,49 g ; 42,9 mmol) d'oxyde de propylène dans 20 ml de THF fraîchement distillé est ajoutée goutte à goutte en une période de 15 min. Le mélange est agité pendant 18 heures avec chauffage à 20°C, puis le mélange est hydrolysé par addition de 100 ml d'une solution saturée de NH₄Cl. Le THF est éliminé sous pression réduite et le mélange obtenu est extrait trois fois avec 100 ml de dichlorométhane. Les couches organiques sont combinées, séchées (MgSO₄) et évaporées sous pression réduite pour fournir une huile qui est soumise à une chromatographie sur colonne avec de l'éther diéthylique comme éluant pour fournir le 1-(N,N-diméthylsulfamoyl)-2-tert-butyldiméthylsilyl-5-(2-hydroxypropyl)imidazole sous forme d'une huile jaune visqueuse.

L'huile ci-dessus (11,28 g ; 32,5 mmol) est dissoute dans 50 ml de tétrachlorure de carbone anhydre et 9,18 g (35,0 mmol) de triphénylphosphine anhydre dans 50 ml de tétrachlorure de carbone anhydre sont ajoutés. Le mélange est agité sous une atmosphère d'azote à 50°C, puis porté à reflux pendant 16 heures. Le solvant est évaporé sous vide et le solide obtenu est soumis à une chromatographie sur colonne avec du dichlorométhane sur du gel de silice pour fournir, le 1-(N,N-diméthylsulfamoyl)-2-tert-butyl-diméthylsilyl-5-(2-chloropropyl)imidazole sous forme d'une huile jaune pâle qui se solidifie, P.F. 51-53°C.

Du 3-trifluorométhyl-thiophénol (0,298 g ; 1,67 mmol) est dissous dans 20 ml de DMF anhydre et refroidi à 0°C sous une atmosphère d'azote et du NaH (dispersion à 60 % dans l'huile minérale ; 0,0393 g ; 1,638 mmol) est ajouté par petites portions. Le mélange réactionnel est agité à 0°C pendant 15 min, puis à 20°C pendant encore 1,5 h et 0,293 g (0,80 mmol) de 1-(N,N-diméthylsulfamoyl)-2-tert-butyldiméthylsilyl-5-(2-chloropropyl)imidazole dissous dans 5 ml de DMF et 10 mg de n-Bu₄NI sont ajoutés et le mélange est chauffé à 80°C pendant 3 jours. Le solvant est chassé sous pression réduite pour fournir une huile brune qui est traitée avec 100 ml d'eau et extraite 3 fois avec 40 ml de dichlorométhane. Les extraits sont séchés (MgSO₄) et concentrés et l'huile obtenue est soumise à une chromatographie sur colonne en utilisant du white-spirit/acétate d'éthyle 2/1 et 1/1 puis dissoute dans 10 ml d'HCl 2 M et chauffée à 100°C à reflux pendant 3 heures. Le mélange réactionnel est ensuite alcalinisé par addition de NaOH à 10 % (pH environ 11) et est extrait 3 fois avec 40 ml de dichlorométhane. Les extraits sont séchés (MgSO₄) et évaporés pour former une huile limpide qui est soumise à une chromatographie sur colonne avec de l'acétate d'éthyle comme éluant et transformée en l'oxalate du produit désiré dans le 2-propanol, P.F. 166-168°C.
C₁₃H₁₃F₃N₂S.C₂H₂O₄:

| | | | | | | | |
|---|---|---|---|---|---|---|---|
| Analyse CHN | Calculé | C | 47,87 | H | 4,02 | N | 7,44 |
| | Trouvé | C | 47,43 | H | 4,04 | N | 7,22 |

### Exemple 160

### Oxalate de 4-[2-(4-méthylphénoxy)éthyl]-1H-imidazole

### (Semblable au procédé A)

Un mélange de 0,18 g (1,68 mmol) de p-crésol, 0,60 g (1,69 mmol) de 1-(triphénylméthyl)-4-(2-hydroxyéthyl)imidazole et 0,44 g (1,69 mmol) de triphénylphosphine dans 20 ml de tétrahydrofuranne anhydre est refroidi et agité pendant 10 minutes sous azote. 0,66 g (3,44 mmol) d'azodicarboxylate de diéthyle dissous dans 10 ml de THF fraîchement distillé est ajouté lentement au mélange réactionnel et l'agitation est poursuivie à la température ordinaire pendant 16 heures. Après élimination du solvant sous vide, la chromatographie sur colonne du mélange réactionnel brut sur gel de silice (éluant : hexane/acétate d'éthyle 2/1) fournit 0,45 g (60 %) de produit sous forme d'une huile incolore. On chauffe cette dernière à 80°C pendant 5 heures dans 8 ml de THF et 1,3 équivalent d'acide chlorhydrique 2 M. Après refroidissement, le THF est chassé sous pression réduite et le Ph₃ COH est extrait avec 3 portions de 30 ml d'éther diéthylique. La couche aqueuse est neutralisée avec du carbonate de potassium et le produit est extrait dans 3 portions de 30 ml de chloroforme. Les couches chloroformiques combinées sont séchées (MgSO₄) et évaporées sous pression réduite pour fournir une huile qui est traitée avec une solution propanolique d'acide oxalique pour fournir le produit, P.F. 188-189°C qui est séché sous vide. C₁₂H₁₄N₂O.0,8C₂.H₂O₄ :

| | | | | | | | |
|---|---|---|---|---|---|---|---|
| Analyse CHN | Calculé | C | 59,56 | H | 5,73 | N | 10,21 |
| | Trouvé | C | 59,80 | H | 5,77 | N | 10,17 |

### Exemple 161

### Oxalate de 4-[2-(4-propionylphénoxy)éthyl]-1H-imidazole

D'une façon semblable à l'exemple 160, 0,254 g (1,69 mmol) de 4-propionylphénol est transformé en l'oxalate indiqué ci-dessus,
P.F. 185-187°C.
C₁₄H₁₆N₂C₂.0,9C₂H₂O₄:

| | | | | | | | |
|---|---|---|---|---|---|---|---|
| Analyse CHN | Calculé | C | 58,33 | H | 5,52 | N | 8,61 |
| | Trouvé | C | 58,30 | H | 5,45 | N | 8,55 |

### Exemple 162

### Oxalate de 4-[3-(4-sec-butylphénoxy)propyl]-1H-imidazole

D'une façon semblable à l'exemple 160, 0,6 g (1,63 mmol) de 1-(triphénylméthyl)-4-(3-hydroxypropyl)imidazole est traité avec 0,245 g (1,63 mmol) de 4-sec-butylphénol pour fournir l'oxalate indiqué ci-dessus, P.F. 202-203°C.
C₁₆H₂₂N₂O.0,8C₂H₂O₄ :

| | | | | | | | |
|---|---|---|---|---|---|---|---|
| Analyse CHN | Calculé | C | 63,98 | H | 7,20 | N | 8,48 |
| | Trouvé | C | 63,85 | H | 7,24 | N | 8,52 |

### Exemple 163

### Oxalate de 4-[3-(4-éthylphénoxy)propyl)-1H-imidazole

D'une façon semblable à l'exemple 160, 0,80 g (2,17 mmol) de 1-(triphénylméthyl)-4-(3-hydroxypropyl)imidazole est traité avec 0,33 g (2,70 mmol) de 4-éthylphénol pour fournir l'oxalate indiqué ci-dessus, P.F. 199-200°C après recristallisation dans l'éthanol. C₁₄H₁₈N₂O.0,8C₂H₂O₄ :

| | | | | | | | |
|---|---|---|---|---|---|---|---|
| Analyse CHN | Calculé | C | 61,97 | H | 6,53 | N | 9,27 |
| | Trouvé | C | 62,16 | H | 6,39 | N | 9,38 |

### Exemple 164

### Trifluoroacétate de 4-[3-(4-imidazol-1-yl-phénoxy)propyl]-1H-imidazole

D'une façon semblable à l'exemple 160, 0,5 g (1,36 mmol) de 1-(triphénylméthyl)-4-(3-hydroxypropyl)imidazole est traité avec 0,27 g (1,70 mmol) de 4-(imidazol-1-yl)phénol pour fournir le composé indiqué ci-dessus sous forme du sel oxalate. Ce dernier est impur et il est donc soumis à une chromatographie liquide préparative à haute pression sur du Kromasil C₁₈ en utilisant de l'acide trifluoacétique à 0,1 % et de l'acide trifluoroacétique à 0,1 % dans le méthanol dans un rapport de 4/1. Le produit obtenu est le trifluoroacétate hydraté, P.F. 259°C (décomposition).
C₁₅H₁₆N₄O.0,6CF₃CO₂H.2,1H₂O :

| | | | | | | | |
|---|---|---|---|---|---|---|---|
| Analyse CHN | Calculé | C | 51,95 | H | 5,60 | N | 14,96 |
| | Trouvé | C | 51,92 | H | 5,66 | N | 14,76 |

### Exemple 165

### Oxalate de 4-[3-(4-(N,N-diméthylsulfamoyl)phénoxy)propyl]-1H-imidazole

D'une façon semblable à l'exemple 160, 0,60 g (1,63 mmol) de 1-(triphénylméthyl)-4-(3-hydroxypropyl)imidazole est traité avec 0,39 g (1,95 mmol) de 4-(N,N-diméthylsulfamoyl)phénol (P.F. 94-96°C ; Steinkopf, J. prakt. Chem. [2] 1927, 117, 59) pour fournir l'oxalate indiqué ci-dessus, P.F. 178-180°C.
C₁₄H₁₉N₃C₃S.0,85C₂H₂O₄ :

| | | | | | | | |
|---|---|---|---|---|---|---|---|
| Analyse CHN | Calculé | C | 48,86 | H | 5,41 | N | 10,89 |
| | Trouvé | C | 48,81 | H | 5,67 | N | 10,81 |

### Exemple 166

### Oxalate de 4-[3-(4-thiométhylphénoxy)propyl]-1H-imidazole

D'une façon semblable à l'exemple 160, 0,50 g (1,35 mmol) de 1-(triphénylméthyl)-4-(3-hydroxypropyl)imidazole est traité avec 0,19 g (1,35 mmol) de 4-thiométhylphénol pour fournir l'oxalate indiqué ci-dessus qui a un P.F. de 202-204°C, après recristallisation dans l'éthanol.
C₁₃H₁₆N₂OS.C₂H₂O₄.0,1H₂O :

| | | | | | | | | | |
|---|---|---|---|---|---|---|---|---|---|
| Analyse CHNS | Calculé | C | 52,96 | H | 5,39 | N | 8,23 | S | 9,42 |
| | Trouvé | C | 52,73 | H | 5,38 | N | 8,12 | S | 9,66 |

### Exemple 167

### Chlorhydrate de 4-[3-(4-thiobenaylphénoxy)propyl]-13-imidazole

D'une façon semblable à l'exemple 160, 0,70 g (1,9 mmol) de 1-(triphénylméthyl)-4-(3-hydroxypropyl)imidazole est traité avec 0,41 g (1,9 mmol) de 4-thiobenzylphénol, puis déprotégé avec de l'HCl 2 M dans le tétrahydrofuranne. Après élimination du solvant, il demeure un solide qui est lavé avec de l'éther diéthylique et cristallisé dans le 2-propanol et l'éther diéthylique pour fournir le chlorhydrate indiqué ci-dessus qui a un P.F. de 166-168°C.
C₁₉H₂₀N₂OS.HCl :

| | | | | | | | |
|---|---|---|---|---|---|---|---|
| Analyse CHN | Calculé | C | 63,32 | H | 5,87 | N | 7,77 |
| | Trouvé | C | 63,73 | H | 5,77 | N | 8,13 |

### Exemple 168

### Oxalate de 4-[3-(3-acétylphénylthio)propyl]-1H-imidazole

### (Semblable au procédé C)

Du 3-acétylthiophénol (0,256 g ; 1,68 mmol) est dissous dans 20 ml de diméthylformamide anhydre et refroidi au-dessous de 4°C sous une atmosphère d'azote et 0,067 g (1,68 mmol) d'hydrure de sodium est ajouté en petites portions au-dessous de 4°C. Le mélange est agité à 4°C pendant 15 min, puis à 20°C pendant 1,5 h. Du 2-tert-butyldiméthylsilyl-5-(3-chloropropyl)-1-(N,N-diméthylsulfamoyl)imidazole (Vollinga, R.C., Menge, W.M.P.B. et Timmerman, H. Rec. trav. chim. Pays-Bas. 1993, 112, 123-125) (0,283 g ; 0,84 mmol) dans 10 ml de diméthylformamide et 10 mg d'iodure de tétra-n-butylammonium comme catalyseur sont ajoutés et le mélange est chauffé à 80°C pendant 3 jours. Le solvant est éliminé sous pression réduite et le mélange réactionnel est arrêté avec 100 ml d'eau et extrait 3 fois avec 40 ml de dichlorométhane ; les extraits sont séchés (MgSO₄) et concentrés en une huile qui est purifiée par chromatographie sur colonne par utilisation d'un mélange de white-spirit/acétate d'éthyle (60/40) pour fournir le 1-(N,N-diméthylsulfamoyl)-4-[3-(3-acétylphénylthio) propyl]imidazole. Ce dernier est chauffé dans 15 ml d'HCl 2 M à reflux pendant 5 h. La réaction est mise à refroidir, puis alcalinisée avec de l'hydroxyde de sodium aqueux à 10 % jusqu'à pH 10, et extraite 3 fois avec 50 ml de dichlorométhane. Les extraits sont séchés (MgSO₄) et concentrés en une huile qui est purifiée par chromatographie sur colonne avec un mélange d'acétate d'éthyle/méthanol 95/5. Le produit huileux est transformé en l'oxalate dans l'éthanol pour fournir un solide cristallin blanc, P.F. 122-124°C.
C₁₄H₁₆N₂OS.1,2C₂H₂O₄ :

| | | | | | | | |
|---|---|---|---|---|---|---|---|
| Analyse CHN | Calculé | C | 54,14 | H | 5,10 | N | 7,79 |
| | Trouvé | C | 54,57 | H | 5,21 | N | 7,41 |

### Exemple 169

### Oxalate de 4-[3-(4-éthylphénylamino)propyl]-1H-imidazole

D'une façon semblable à l'exemple 154, de la 4-éthylaniline est transformée en le composé indiqué ci-dessus qui a un P.F. de 154-155°C après cristallisation dans l'éthanol.
C₁₄H₁₉N₃.1,85C₂H₂O₄ :

| | | | | | | | |
|---|---|---|---|---|---|---|---|
| Analyse CHN | Calculé | C | 53,70 | H | 5.74 | N | 10,61 |
| | Trouvé | C | 53,74 | H | 5,98 | N | 10,56 |

### Exemple 170

### Oxalate de 4-[3-(4-chlorophénylamino)propyl]-1H-imidazole

D'une façon semblable à l'exemple 154, la 4-chloroaniline est transformée en le composé indiqué ci-dessus qui a un P.F. de 130-134°C après cristallisation dans l'éthanol.
C₁₂H₁₄ClN_{3.}2,2C₂H₂O₄ :

| | | | | | | | |
|---|---|---|---|---|---|---|---|
| Analyse CHN | Calculé | C | 45,41 | H | 4,28 | N | 9,69 |
| | Trouvé | C | 45,48 | H | 4,40 | N | 9,80 |

### Exemple 171

### 3-(1H-imidazol-4-yl)propyl-(4-(2-(triméthylsilyl)éth-1-ynyl)phényl)éther

5 mmol d'ester tert-butylique de l'acide 4-(3-hydroxypropyl)-1H-imidazole-1-carboxylique et 5 mmol de 4-(2-(triméthylsilyl)éth-1-ynyl)phénol (pour la préparation voir la littérature indiquée dans l'exemple 122) sont traitées comme décrit dans l'exemple 122.
C₁₇H₂₂N₂OSi.C₄H₄O₄.0.75 H₂O (428.1)

| | | | | | | | |
|---|---|---|---|---|---|---|---|
| Analyse CHN | Calculé | C | 58,9 | H | 6,48 | N | 6,54 |
| | Trouvé | C | 58,7 | H | 6,04 | N | 6,89 |
| Rendement : 30 % | | | | | P.F. : 126°C | | |

### Exemple 172

### 3-(1H-imidazol-4-yl)propyl-(4-prop-1-ynylphényl)éther

5 mmol d'ester tert-butylique de l'acide 4-(3-hydroxy-propyl)-1H-imidazole-1-carboxylique et 5 mmol de 4-prop-1-ynylphénol sont traitées comme décrit dans l'exemple 122.
C₁₅H₁₆N₂O.C₄H₄O₄.0,5H₂O (365,4)

| | | | | | | | |
|---|---|---|---|---|---|---|---|
| Analyse CHN | Calculé | C | 62,5 | H | 5,79 | N | 7,67 |
| | Trouvé | C | 62,3 | H | 5,83 | N | 7,53 |
| Rendement : 87 % | | | | | P.F. : 137°C | | |

### Exemple 173

### 3-(1H-imidazol-4-yl)propyl-4-isopropylphényl éther

5 mmol de 3-(1-triphénylméthyl-1H-imidazol-4-yl)propanol et 6 mmol de 4-isopropylphénol sont traitées comme décrit dans l'exemple 56.
C₁₅H₂₀N₂O₂.C₄H₄O₄.0,25H₂O (364,9)

| | | | | | | |
|---|---|---|---|---|---|---|
| Analyse CHN | Calculé | C | 62,5 | H | 6,77 | N 7,68 |
| | Trouvé | C | 62,5 | H | 6,70 | N 7,79 |
| Rendement : 70 % | | | | | P.F. : 110°C | |

### Exemple 174

### 3-(1H-imidazol-4-yl)propyl-méthyl éther

A une solution de 22 mmol de sodium dans 50 ml de méthanol sont ajoutées 2,5 mmol de chlorure de 3-(1-triphénylméthyl-1H-imidazol-4-yl)propyle.HCl (préparé à partir de 3-(1-triphénylméthyl-1H-imidazol-4-yl)propanol avec du chlorure de thionyle dans le THF). Le mélange réactionnel est ensuite porté à reflux pendant 100 h, puis purifié comme décrit dans l'exemple 5. Le composé du titre est cristallisé sous forme de l'hydrogénooxalate à partir l'éthanol et de l'éther diéthylique.
C₇H₁₂N₂O.C₂H₂O₄ (230,7)

| | | | | | | | |
|---|---|---|---|---|---|---|---|
| Analyse CHN | Calculé | C | 47,0 | H | 6,13 | N | 12,2 |
| | Trouvé | C | 47,1 | H | 6,01 | N | 12,1 |
| Rendement : 40 % | | | | | P.F. : 139°C | | |

### Exemple 175

### Ethyl-3-(1H-imidazol-4-yl)propyl éther

5 mmol de 3-(1-triphénylméthyl-1H-imidazol-4-yl)propanal et 5 mmol de bromoéthane sont traitées comme décrit dans l'exemple 5. Le composé du titre est cristallisé sous forme d'hydrogénooxalate à partir d'éthanol et d'éther diéthylique.
C₈H₁₄N₂O.0,75C₂H₂O₄ (221,7)

| | | | | | | | |
|---|---|---|---|---|---|---|---|
| Analyse CHN | Calculé | C | 51,5 | H | 7,05 | N | 12,6 |
| | Trouvé | C | 51,4 | H | 6,85 | N | 12,7 |
| Rendement : 20 % | | | | P.F. : 167°C | | | |

### Exemple 176

### 3-(1H-imidazol-4-yl)propyl-propyl éther

5 mmol de 3-(1-triphénylméthyl-1H-imidazol-4-yl)propanol et 5 mmol de bromopropane sont traitées comme décrit dans l'exemple 5. Le composé du titre est cristallisé sous forme d'hydrogénooxalate à partir d'éthanol et d'éther diéthylique.
C₉H₁₆N₂O.0,75C₂H₂O₄ (235,8)

| | | | | | | | |
|---|---|---|---|---|---|---|---|
| Analyse CHN | Calculé | C | 53,5 | H | 7,48 | N | 11,9 |
| | Trouvé | C | 53,1 | H | 7,26 | N | 11,8 |
| Rendement : 20% | | | | | P.F. : 169°C | | |

### Exemple 177

### Cyclopropyl-3-(1H-imidazol-4-yl)propyl éther

5 mmol de chlorure de 3-(1-triphénylméthyl-1H-imidazol-4-yl) propyl.HCl (voir l'exemple 174) et 30 mmol de cyclopropanolate de sodium (préparé avec du sodium dans le cyclopropanol) dans 20 ml de cyclopropanol sont traitées comme décrit dans l'exemple 174. Le composé du titre est cristallisé sous forme d'hydrogénooxalate à partir d'éthanol et d'éther diéthylique.
C₉H₁₄N₂O.C₂H₂O₄ (256,3)

| | | | | | | | |
|---|---|---|---|---|---|---|---|
| Analyse CHN | Calculé | C | 51,6 | H | 6,29 | N | 10,9 |
| | Trouvé | C | 51,3 | H | 5,98 | N | 10,7 |
| Rendement : 10 % | | | | | P.F. : 158°C | | |

### Exemple 178

### Cyclopropylméthyl-3-(1H-imidazol-4-yl)propyl éther

5 mmol de 3-(1-triphénylméthyl-1H-imidazol-4-yl)prapanol et 10 mmol de chlorure de cyclopropylméthyl-sodium sont traitées comme décrit dans l'exemple 5. Le composé du titre est cristallisé sous forme d'hydrogénomaléate à partir d'éthanol et d'éther diéthylique.
C₁₀H₁₆N₂O.C₄H₄O₄ (296.3)

| | | | | | | | |
|---|---|---|---|---|---|---|---|
| Analyse CHN | Calculé | C | 56,7 | H | 6.80 | N | 9,45 |
| | Trouvé | C | 56,7 | H | 6,70 | N | 9,38 |
| Rendement : 10 % | | | | | P.F. : 85°C | | |

### Exemple 179

### 1-(1H-imidazol-4-yl)-6-phényl hexane

5 mmol de 3-(1-triphénylméthyl-1H-imidazol-4-yl)propanal (préparé selon des procédés standards (oxydation de Swern) avec du 3-(1-triphénylméthyl-1H-imidazal-4-yl)propanal et du chlorure d'oxalyle dans le DMSO à -45°C) et 5 mmol de bromure de 3-phénylpropyl-triphényl-phosphonium (préparé à partir de triphénylphosphine et de bromure de 3-phénylpropyle dans le toluène à reflux pendant 12 h) sont traitées comme décrit dans l'exemple 51. Le 1-(1H-imidazol-4-yl)-6-phényl-3-hexène est hydogéné comme décrit dans l'exemple 52. Le composé du titre est cristallisé sous forme d'hydrogénooxalate dans l'éthanol et l'éther diéthylique.
C₁₅H₂₀N₂.C₂H₂O₄ (318,4)

| | | | | | | | |
|---|---|---|---|---|---|---|---|
| Analyse CHN | Calculé | C | 64,1 | H | 6,97 | N | 8,80 |
| | Trouvé | C | 64,1 | H | 7,19 | N | 9,09 |
| Rendement : 25 % | | | | | P.F. : 175°C | | |

### Exemple 180

### N-[3,5-di(trifluorométhyl)phényl]-3-(1H-imidazol-4-yl)propyl carbamate

5 mmol d'acide 3,5-di(trifluorométhyl)benzoïque, 5 mmol de triéthylamine et 5 mmol,de phosphorazidate de diphényle sont traitées comme décrit dans l'exemple 110.
C₁₅H₁₃N₃O₂F₆·C₂H₂O₄ (471,1) Rendement : 16 %
P.F. : 215°C (décomposition)
Spectre de masse à haute résolution, appareil MAT 711/19.944, procédé d'appariement des pics (80 eV, 0,8 mA), température 150°C :
Théorique : 381,091210
Trouvé 381,091180

### Exemple 181

### N-(1-tert-butyl-2-phényl)éthyl-3-(1H-imidazol-4-yl)propyl carbamate

5 mmol d'acide 2-tert-butyl-3-phénylpropionique, 5 mmol de triéthylamine et 5 mmol de phosphorazidate de diphényle sont traitées comme décrit dans l'exemple 110.
C₁₉H₂₇N₃O₂.C₂H₂O.0,25H₂O (424,0)

| | | | | | | | |
|---|---|---|---|---|---|---|---|
| Analyse CHN | Calculé | C | 59,5 | H | 7,01 | N | 9,91 |
| | Trouvé | C | 59,3 | H | 6,71 | N | 9,82 |
| Rendement : 15 % | | | | P.F. : 158°C | | | |

### Exemple 182

### N-(1-éthylpropyl)-3-(1H-imidazol-4-yl)propyl carbamate

5 mmol d'acide 2-éthylbutyrique, 5 mmol de triéthylamine et 5 mmol de phosphorazidate de diphényle sont traitées comme décrit dans l'exemple 110. Le composé du titre est cristallisé sous forme d'hydrogénomaléate à partir d'éther diéthylique et d'éthanol.
C₁₂H₂₁N₃O₂.C₄H₄O₄ (355,4)

| | | | | | | | |
|---|---|---|---|---|---|---|---|
| Analyse CHN | Calculé | C | 54,1 | H | 7,09 | N 11,8 | |
| | Trouvé | C | 53,7 | H | 6,92 | N 11,6 | |
| Rendement : 22 % | | | | | P.F. : 108°C | | |

### Exemple 183

### 1-(1H-Imidazol-4-yl)-6-phényl 3-hexèné

5 mmol de 3-(1-triphénylméthyl-1H-imidazol-4-yl)propanal (préparé selon des procédés standards (oxydation de Swem) avec du 3-(1-triphénylméthyl-1H-imidazol-4-yl)propanol et du chlorure d'oxalyle dans le DMSO à -45° C; voir les exemples 179 et 154) et 5 mmol de bromure de 3-phényl-propyl-triphényl-phosphonium (voir exemple 179) sont traitées comme décrit dans l'exemple 51. Le composé du titre est cristallisé sous forme d'hydrogénooxalate dans l'éthanol et l'éther diéthylique.
C₁₅H₁₈N₂.0,75C₂H₂O₄ (293.9)

| Analyse CHN | | | | | | | |
|---|---|---|---|---|---|---|---|
| | calculé : | C | 67,4 | H | 6.69 | N | 9.53 |
| | trouvé : | C | 67,3 | H | 6,92 | N | 9,69 |
| Rendement: 30 % | | | | P.F. : 140° C. | | | |

### Etude Pharmacologique

### . Composés antagonistes

L'interaction des composés avec le récepteur H₃ est mise en évidence in vitro par la mesure de la libération d'histamine-³H synthétisée à partir d'histidine-³H par des synaptosomes de cortex cérébral de rat (Garbarg et al., J. Pharmacol. Exp. Ther. 1992, 263: 304).

Le pouvoir antagoniste H₃ des composés est évalué par la réversion progressive de l'inhibition de la libération d'histamine-³H par la (R)- -méthylhistamine, un agoniste H₃ sélectif (Arrang et coll., Nature, 1987, 327: 117-123).

Les effets antagonistes des composés in vivo sont évalués par la mesure des variations des taux de téléméthylhistamine cérébrale chez la souris (Garbarg et coll., J. Neurochem. 1989, 53: 1724). Après un délai variable après administration du composé, l'effet d'un antagoniste H₃ est mis en évidence par l'élévation du taux de téléméthylhistamine cérébral qu'il induit. Les résultats sont rassemblés dans les tableaux II et III suivants:

**TABLEAU II : CONSTANTES APPARENTES DE DISSOCIATION (Ki) DE DIVERS DERIVES DE L'INVENTION COMME ANTAGONISTES DE L'HISTAMINE SUR LES RECEPTEURS H₃.**

| Exemple N° | Ki(nM) |
|---|---|
| 21 | 45 |
| 64 | 16 |
| 56 | 22 |
| 30 | 8 |
| 81 | 0,5 |
| 93 | 25 |
| 115 | 44 |
| 149 | 44 |
| 166 | 3 |

**TABLEAU III : EFFETS DES COMPOSES ANTAGONISTES SUR LE TAUX DE TELE-METHYLHISTAMINE CEREBRALE.**

| Antagonistes H₃ | Variation du taux de téléméthylhistamine cérébrale (par rapport aux témoins) |
|---|---|
| 81 | +72% |
| 21 | +79% |
| 67 | +73% |
| 78 | +68% |
| 30 | +67% |
| 39 | +84% |
| 112 | +98% |
| | |
| 140 | +84% |
| 160 | +95% |

Ces divers composés ont été administrés à la dose de 10 mg/kg p.o. et les souris sacrifiées 90 min plus tard. Le composé de référence a été le thiopéramide qui, dans les mêmes conditions, induit des variations de +75% à +100% en moyenne.

Cette propriété d'antagonistes H₃ actifs par voie générale fait des composés de l'invention des dérivés utiles en médecine humaine et vétérinaire. Leurs applications thérapeutiques concernent notamment le système nerveux central (y compris comme psychostimulants). Les composés antagonistes des récepteurs H₃ de l'histamine de formule Ia ou Ib sont avantageusement utilisés comme principe actif de médicaments à actions psychotropes, activatrices de l'éveil, de l'attention, de la mémoire, et de l'humeur, dans le traitement d'affecticns telles que la maladie d'Alzheimer et autres troubles cognitifs des personnes âgées, des états dépressifs ou même simplement asthéniques. Leurs effets nootropes pourront être mis à profit pour stimuler la vigilance ou la capacité d'apprentissage de sujet sains. Leurs effets positifs sur la régulation de l'activité des centres ce l'équilibre seront mis à profit dans le traitement de vertiges. Ils pourront aussi être utilisés comme principe actif de médicaments destinés au traitement des troubles de l'équilibration et des vertiges, notamment vertige de Ménières, en particulier chez les personnes âgées. Ils pourront être utilement associés à des traitements par d'autres agents psychiatriques tels que les neuroleptiques pour en augmenter l'activité et diminuer les effets secondaires. Les applications thérapeutiques concernent également les organes périphériques notamment comme stimulants des sécrétions et de la motricité gastrointestinale.

La présente invention concerne donc également les compositions pharmaceutiques qui contiennent, à titre de principe actif, une quantité thérapeutiquement efficace d'un des composés antagonistes de formule Ia ou Ib.

La composition pharmaceutique conforme à l'invention est administrable à l'homme par voie orale, perlinguale, nasale, dermique, transdermique, ophtalmique, vaginale, percutanée, topique, rectale et parentérale, le principe actif étant associé à un excipient ou véhicule thérapeutiquement convenable.

Chaque dose unitaire contient avantageusement de 0,03 à 3 mg/kg.

L'invention a aussi pour objet l'utilisation des dérivés conformes à l'invention pour la préparation de médicaments antagonistes H₃ selon les modalités précitées.

### . Composés agonistes ou agonistes partiels

L'interaction des composés avec le récepteur H₃ est mise en: évidence in vitro par la mesure de la libération d'histamine-³H synthétisée à partir d'histidine-³H par des synaptosomes de cortex cérébral de rat.

L'effet agoniste H₃ des composés est mis en évidence par l'inhibition de libération d' histamine--³H qu'ils induisent de manière réversible en présence d'un antagoniste H₃ comme le thiopéramide. L'effet agoniste partiel des composés est évalué par comparaison de l'inhibition maximale qu'ils induisent par rapport à l'inhibition maximale induite par l'histamine exogène ou la (R) -α-méthylhistamine ; le rapport de ces deux valeurs fournissant l'activité intrinsèque du composé sur le système.

L'effet d'un agoniste partiel in vivo est caractérisé par la réduction maximale du taux de téléméthylhistamine qu'il induit à dose élevée, moins importante que la réduction induite par un agoniste H₃ de référence.

Cependant, pour certains composés agonistes partiels présentant une activité intrinsèque faible ou même modérée (≤ 10% de celle de l'histamine), l'essai in vitro ne permet pas de mettre en évidence aisément leur effet agoniste. Par contre, nous avons découvert que la mesure de l'activité des neurones histaminergiques cérébraux in vivo, reflétée par le taux de téléméthylhistamine, métabolite caractéristique de l'histamine libérée, constitue un essai plus sensible de l'activité agoniste. En effet, des composés présentant une activité intrinsèque ≤ 25% in vitro provoquent in vivo une baisse maximale ou quasi maximale du taux de téléméthylhistamine. Un autre test sensible de l'activité agoniste H₃, portant cette fois sur des organes périphériques, consiste en la mesure de l'extravasation plasmatique induite chez le rat sous l'influence de la capsaïcine (90 µg/kg i.v.) et déterminée par mesure du taux tissulaire de bleu Evans, colorant administré en même temps que la capsaïcine, soit 5 min avant perfusion et sacrifice (Saria et coll., Naunyn-Schmiedeberg's Arch. Pharmacol. 1983, 324: 212). C'est ainsi que sur ces deux essais, le composé de l'exemple 2 induit une réponse voisine de celle de la (R) -α-méthyl-, histamine tandis que in vitro, son activité agoniste intrinsèque est voisine de 20 % et qu'il se comporte donc essentiellement comme un antagoniste.

Les résultats sont rassemblés dans les tableaux IV et V suivants :

**TABLEAU IV**

| CONCENTRATIONS EFFICACES 50% (CE₅₀) D'AGONISTES PARTIELS | | |
|---|---|---|
| Exemple n° | CE₅₀ (nM) | Activité intrinsèque |
| 8 | 100 | 40% |
| 5 | 130 | 20% |
| 2 | 100 | 20% |

**TABLEAU V**

| EFFETS DES COMPOSES AGONISTES PARTIELS SUR LE TAUX DE TELE-METHYLHISTAMINE CEREBRALE | |
|---|---|
| Agonistes H₃ | Variation du taux de télé-méthylhistamine cérébrale (par rapport aux témoins) |
| 8 | -41% |
| 5 | -52% |
| 2 | -30% |
| 111 | -34% |
| 157 | -38% |

Ces divers composés ont été administrés à la dose de 10 mg/kg p.o. et les souris sacrifiées 90 min plus tard. Le composé de référence a été l'Imétit qui, dans les mêmes conditions, induit des variations de -45% à -60% en moyenne

Cette propriété d'agonistes H₃ actifs par voie générale fait des composés de l'invention des dérivés utiles en médecine humaine et vétérinaire.

Les agonistes et agonistes partiels des récepteurs H₃, par leurs effets cérébraux, exercent principalement une activité sédative, tranquilisante, anti-stress et analgésique indiquant leur utilisation comme psychotropes sédatifs légers notamment dans divers troubles psychosomatiques.

Les agonistes et agonistes partiels H₃ sont également indiqués dans le traitement des états migraineux et autres céphalées.

Par leurs effets périphériques, les agonistes et agonistes partiels des récepteurs H₃ seront principalement indiqués dans le traitement d'affections respiratoires, allergiques ou inflammatoires (asthme, bronchites, rhinite, trachéites, etc.), cardiaques (dysfonctionnement et infarctus du myocarde), gastrointestinales grâce à leurs actions antisécrétoires et anti-inflammatoires (ulcères gastriques ou duodénaux, colite ulcérative, maladie de Crohn, colon irritable, incontinence fécale, etc.), de l'appareil urogénital (cystites, métrites, syndrome prémenstruel, inflammations prostatiques, incontinence urinaire, troubles génitaux), de l'appareil cutané (urticaire, démangeaisons). L'effet anti-inflammatoire et analgésique peut utilement être mis à profit dans le traitement des arthrites et autres affections rhumatismales, les conjonctivites et autres inflammations oculaires, de la sialorrhée.

Les composés agonistes ou agonistes partiels des récepteurs H₃ de l'histamine sont avantageusement utilisés comme principe actif de médicaments, notamment à effets sédatifs léger, antisécrétoire, anti-inflammatoire, régulateur de sommeil, anti-convulsivant, régulateur de sécrétion hypothalamo-hypophysaire, antidépresseur; modulateur de circulation cérébrale, modulateur du système immunitaire, anti-allergique, antimigraineux.

La présente invention concerne donc également les compositions pharmaceutiques qui contiennent, à titre de principe actif, une quantité thérapeutiquement efficace d'un des composés agonistes ou agonistes partiels de formule (Ib).

La composition pharmaceutique conforme à l'invention est administrable à l'homme par voie orale, perlinguale, dermique, transdermique, ophthalmique, vaginale, percutanée, topique, nasale, rectale et parentérale, le principe actif étant associé à un excipient ou véhicule thérapeutiquemert convenable.

Les composés agonistes ou agonistes partiels de la présente invention sont actifs à des doses unitaires comprises entre 0,1 et 10 mg/kg par voie orale chez le rongeur, correspondant à des doses comprises entre 0,03 et 3 mg/kg chez l'homme. Pour des applications locales, par exemple sous forme de pommades ou collyres, les concentrations actives seront comprises entre 10⁻⁸M et 10⁻⁵M**.**

L'invention a aussi pour objet l'utilisation des dérivés conformes à l'invention pour la préparation de médicaments agonistes H₃ selon les modalités précitées.

## Revendications

1. Composés répondant à la formule générale: 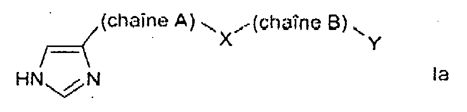 ou 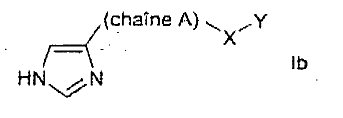 dans laquelle:
- la chaîne A représente un groupement alkylène droit ou ramifié comprenant 1 à 8 atomes de carbone ; un groupement alcényle droit ou ramifié comprenant jusqu'à 8 atomes de carbone ; un groupement alcynyle droit ou ramifié comprenant jusqu'à 4 atomes de carbone ;
- le groupement X représente -O- ; -OCONH-, -OCON(alcène)-, -OCSNH- ; -CH²- ;
- la chaîne B représente un groupement alkylène droit ou ramifié comprenant 1 à 8 atomes de carbone ; un groupement alcényle comportant jusqu'à 8 atomes de carbone ; un groupement (CH₂)ₙO et n est un entier pouvant varier entre 0 et 4 ;
- le groupement Y représente un groupement phényle, mono ou polysubstitué par un ou plusieurs substituants, identiques ou différents, choisis parmi -OCF_{3,} CHO, SO₂N(alkyle)₂, SO₂N(CH₃)₂, S(alkyle), S(aryle), SC_{H2}(phényle), un alcène droit ou ramifié comprenant jusqu'à 8 atomes de carbone, un alcyne droit ou ramifié comprenant jusqu'à 8 atomes de carbone éventuellement substitué par un radical trialkylsilyle, O(aryle), -CH₂CN, un aldéhyde, une sulfone, un acétal, un alcohol, -CH=CH-CHO, -C(alkyle)=N-OH, -C(alkyle)=N-O(alkyle),-CH=NOH, -CH=NO(alkyle), -C(alkyle)=NH-NH-CONH₂, un groupement -O-phényle-OCH₂(phényle), -C(cycloalkyle)=NOH,-C(cycloalkyle)=N-O(alkyle), un hétérocycle éventuellement substitué ; un cycle phényle accolé à un carbocycle non aromatique ou un hétérocycle portant une fonction cétone; un alcyne droit ou ramifié comprenant jusqu'à 8 atomes de carbone et de préférence jusqu'à 5 atomes de carbone ; un alkyle linéaire ou ramifié, mono ou polyphénylique où les groupements phényles sont soit non substitués soit mono ou polysubstitués ; une phénylalkylcétone où le groupement alkyle est ramifié ou non, ou cyclique; un phénylalcool substitué ou non, dont le motif alcool est droit ou ramifié ou cyclique; un alcène droit ou ramifié comprenant jusqu'à 8 atomes de carbone ; un groupement phénylcycloalkyle; un groupement fluorenyle, un groupement indanyle; un groupement phénol; un groupement diphényle; un groupement phénoxyphényle; un groupement benzyloxyphényle;
ainsi que leurs sels pharmaceutiquement acceptables, leurs hydrates, leurs sels hydratés, les structures cristallines polymorphiques et les formes tautomères de ces composés; et leurs isomères optiques, les mélanges racémiques desdits isomères et les diastéréoisomères correspondants,
à l'exception des composés pour lesquels A représente un groupement alcényle droit ou ramifié comprenant jusqu'à 8 atomes de carbone et X= -CH₂-,

2. Composés répondant à la formule générale: 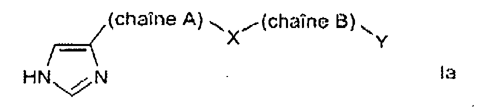 ou 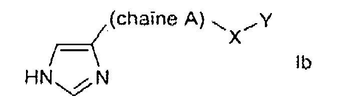 dans laquelle:
- la chaîne A représente un groupement alkylène droit ou ramifié comprenant 1 à 8 atomes de carbone ; un groupement alcényle droit ou ramifié comprenant jusqu'à à 8 atomes de carbone ; un groupement alcynyle droit, ou ramifié comprenant jusqu'à 4 atomes de carbone ;
- le groupement X représente -O- ; -OCONH-, -OCON(alcéne)-, - OCSNH- ; -CH₂- ;
- la chaîne B représente un groupement alkylène droit ou ramifié comprenant 1 à 8 atomes de carbone ; un groupement alcényle comportant jusqu'à 8 atomes de carbone ; un groupement alcynyle comportant jusqu'à 8 atomes de carbone ; un groupement (CH₂)ₙO et n est un entier pouvant varier entre 0 et 4 ;
- le groupement Y représente un groupement phényle, mono ou polysubstitué par un ou plusieurs substituants, identiques ou différents, choisis parmi -OCF₃, CHO, SO₂N(alkyle)₂, SO₂N(CH₃)₂, S(alkyle), S(aryle), SCH₂(phényle), un alcène droit ou ramifié comprenant jusqu'à 8 atomes de carbone, une alcyne droit ou ramifié comprenant jusqu'à 8 atomes de carbone éventuellement substitué par un radical trialkylsilyle, O(aryle), -CH₂CN, un aldéhyde, une sulfone, un acétal, un alcool, -CH=CH-CHO, -C(alkyle)=N-OH, -C(alkyle)=N-O(alkyle), -CH=NOH, -CH=NO(alkyle), -C(alkyle)=NH-NH-CONH₂, un groupement -O-phényle-OCH2(phényle), -C(cycloalkyle)=NOH, -C(cycloalkyle)=N-O(alkyle), un hétérocycle éventuellement substitué ; un cycle phényle accolé à un carbocycle non aromatique ou un hétérocycle portant une fonction cétone; un alcyne droit ou ramifié comprenant jusqu'à 8 atomes de carbone et de préférence jusqu'à 5 atomes de carbone ; un alkyle linéaire ou ramifié, mono ou polyphénylique où les groupements phényles sont soit non substitués soit mono ou polysubstitués ; une phénylalkylcétone ou le groupement alkyle est ramifié ou non, ou cyclique, un phénylalcool substitué ou non, dont le motif alcool est droit ou ramifié ou cyclique ; un alcène droit ou ramifié comprenant **jusqu'à** 8 atomes de carbone ; un groupement phénylcycloalkyle ; un groupement fluorenyle ; un groupement indanyle ; un groupement diphényle; un groupement phénoxyphényle ; un groupement benzyloxyphényle ;
ainsi que leurs sels pharmaceutiquement acceptables, leurs hydrates, leurs sels hydratés, les structures cristallines polymorphiques et les formes tautomères de ces composés ; et leurs isomères optiques, les mélanges racémiques desdits isomères et les diastéréoisomères correspondants.

3. Composés selon la revendication 1 ou 2, **caractérisés en ce que** X représente un oxygène.

4. Composés selon l'une quelconque des revendications 1 à 3, **caractérisés en ce que** A représente -(CH₂)ₙ- n variant de 1 à 4.

5. Composés selon la revendication 4, **caractérisés en ce que** A représente -(CH₂)₃.

6. Composés selon l'une des revendications 1 à 5, **caractérisés en ce que** la chaîne A est un groupement -(CH₂)₃ ; X est un groupement O ou OCONH; la chaîne B est un groupement (CH₂)ₙ où n = 0, 2 ou 3; et Y est un groupement -CH(phényle)₂.

7. Composés selon l'une des revendications 1 à 5, **caractérisés en ce que** la chaîne A est un groupement -(CH₂)ₙ où n est un nombre entier pouvant varier entre 1 et 8 ou CH₂CH(CH₃) -; X est un groupement O ou OCONH; et Y représente un alkyle ramifié ou droit mono- ou polyphénolique.

8. Composés selon l'une des revendications 1 à 7, **caractérisés en ce que** la chaîne A est un groupement -(CH₂)ₙ ou n = 2, 3 ou 4 ; X est un groupement O ou OCONH; et Y représente un groupement -CH(phényle)₂.

9. Composés selon la revendication 1, **caractérisés en ce qu'**ils sont choisis parmi le groupe formé par:
- 3-(1H-Imidazol-4-yl) propyl-N-(diphénylméthyl)carbamate ;
- 3-(1H-Imidazol-4-yl) propyl-N-(2,2-diphényléthyl)carbamate ;
- 3-(1H-Imidazol-4-yl) éthyl-N-(2,2-diphényléthyl)carbamate ;
- 4-[3-(1,2,3,4-tétrahydronapht-6-yloxy) propyl]-1H-imidazole ;
- 4-[3-(Indan-5-yloxy)propyl]-1H-imidazole;
- N,N-Diallyl-3-(1H-imidazol-4-yl) propyl carbamate ;
- 3-(1H-Imidazol-4-yl) propyl-N-trans-(2-phénylcyclopropyl) carbamate ;
- N-Fluoren-9-yl-3-(1H-imidazol-4-yl) propyl carbamat ;
- 3-(1H-Imidazol-4-yl) propyl-(pent-4-inyl) éther;
- 3-(1H-Imidazol-4-yl)propyl-(diphénylméthyl) éther;
- ((4-Fluorophényl) phénylméthyl)-3-(1H-imidazol-4-yl) propyl éther ;
- Bis (4-fluorophényl) méthyl)-3-(1H-imidazol-4-yl) propyl éther ;
- N-(3,3-Diphénylpropyl)-3-(1H-imidazol-4-yl) propyl carbamate.

10. Composés selon la revendication 1 ou 2, **caractérisés en ce qu'**ils sont choisis parmi le groupe formé par:
- 3-(1H-Imidazol-4-yl) propyl-N-prop-2-ényl carbamate ;
- N-(1,1-Diphényléthyl)-3-(1H-imidazol-4-yl) propyl carbamate ;
- 3-(1H-Imidazol-4-yl)propyl-prop-2-ényl éther;
- 3-(1H-Imidazol-4-yl)propyl-pent-4-ényl éther;
- 3-(1H-Imidazol-4-yl)propyl-prop-2-ynyl éther;
- 1-(4-Fluorophényl)-4-(3-(1H-imidazol-4-yl) propyloxy)-butan-1-one ;
- 5-(3-(1H-imidazol-4-yl) propyloxy) indan-1-one;
- 3,4-dihydro-6-(3-(1H-imidazol-4-yl) propyloxy)-2H-naphtalen-1-one.

11. Composition pharmaceutique comprenant une quantité thérapeutiquement efficace d'un composé selon l'une des revendication précédentes dans un excipient pharmaceutiquement acceptable.

12. Médicament agissant comme agoniste, agoniste partiel ou antagoniste des récepteurs H₃ de l'histamine, **caractérisé en ce qu'**il comprend un composé selon l'une des revendications 1 à 10.

13. Médicament selon la revendication 12 **agissant en agoniste ou agoniste partiel des récepteurs H3 de l'histamine**, destiné au traitement du l'asthme, des bronchites, des rhinites, des trachéites, des dysfonctionnement et infarctus du myocarde, des ulcères gastriques ou duodénaux, des colites ulcératives, de la maladie de Crohn, du syndrome du colon irritable, des cystites, des métrites, des incontinences urinaire et fécale, des urticaires, des démangeaisons, des arthrites, des conjonctivites, du syndrome prémenstruel.

14. Médicament selon la revendication 12, **caractérisé en ce qu'**il est administrable par voie générale ou locale notamment sous forme de pommade, de collyres ou de dispositifs pour absorption transcutanée ou transdermique.

15. Médicament selon la revendication 12, **caractérisé en ce que**, notamment lors d'une prise orale, les doses unitaires sont comprises entre 0,03 et 3 mg/kg chez l'homme.

16. Médicament selon la revendication 12 **agissant comme antagoniste des récepteurs 113 de l'histamine,** destiné notamment à exercer une activité psychotrope, activatrice de l'éveil, de l'attention, de la mémoire, de l'humeur, de la vigilance, de la capacité d'apprentissage ou à traiter les états dépressifs, asthéniques, les troubles cognitifs, notamment psychotiques.

17. Médicament selon la revendication 12, **agissant comme antagoniste des récepteurs H3 de l'histamine**, destiné au traitement de la maladie d'Alzheimer.

18. Médicament selon la revendication 12, **agissant comme antagoniste des précepteurs H3 de l'histamine**, destiné au traitement des troubles de l'équilibre est des vertiges.

19. Composé selon l'une quelconque des revendications 1 à 10 **comme agoniste ou agoniste partiel des récepteurs H3 de l'histamine**, pour exercer une activité sédative, tranquillisante, anti-stress, anti-migraineuse, et pour traiter les troubles psychosomatiques, les affections respiratoires, allergiques, rhumatismales, ou inflammatoires de l'oeil, de l'appareil uro-génital, du tractus digestif, de la peau, de l'appareil respiratoire ou des bronches.

## Claims

1. Compounds corresponding to the general formula: 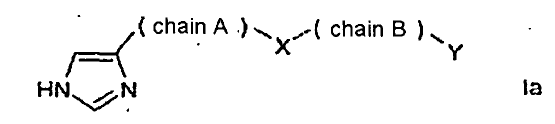 or 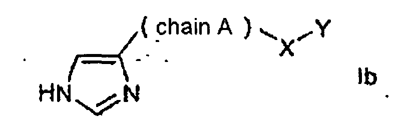 in which:
- chain A represents a straight or branched alkylene group containing from 1 to 8 carbon atoms; a straight or branched alkenyl group containing up to 8 carbon atoms; a straight or branched alkynyl group containing up to 4 carbon atoms;
- the group X represents -O-; -OCONH-, -OCON(alkene)-, -OCSNH-; -CH₂-;
- chain B represents a straight or branched alkylene group containing from 1 to 8 carbon atoms; an alkenyl group containing up to 8 carbon atoms; an alkynyl group containing up to 8 carbon atoms; a group (CH₂)ₙO and n is an integer which can vary from 0 to 4;
- the group Y represents a phenyl group mono- or poly-substituted by one or more identical or different substituents selected from -OCF₃, CHO, SO₂N(alkyl)₂, SO₂N(CH₃)₂, S(alkyl), S(aryl), SCH₂(phenyl), a straight or branched alkene containing up to 8 carbon atoms, a straight or branched alkyne containing up to 8 carbon atoms and optionally substituted by a trialkylsilyl radical, O(aryl), -CH₂CN, an aldehyde, a sulfone, an acetal, an alcohol, -CH=CH-CHO, -C(alkyl)=N-OH, -C(alkyl)=NO(alkyl), -CH=NOH, -CH=NO(alkyl), -C(alkyl)=NH-NH-CONH₂, a group O-phenyl-OCH₂(phenyl), -C(cycloalkyl)=NOH, -C(cycloalkyl)=N-O(alkyl), an optionally substituted heterocycle; a phenyl ring attached to a non-aromatic carbocycle or a heterocycle carrying a ketone function; a straight or branched alkyne containing up to 8 carbon atoms and preferably up to 5 carbon atoms; a linear or branched, mono- or poly-phenylic alkyl, wherein the phenyl groups are either unsubstituted or mono- or poly-substituted; a phenyl alkyl ketone wherein the alkyl group is branched or unbranched, or cyclic; a substituted or unsubstituted phenyl alcohol, the alcohol unit of which is straight or branched or cyclic; a straight or branched alkene containing up to 8 carbon atoms; a phenylcycloalkyl group; a fluorenyl group, an indanyl group; a phenol group; a diphenyl group; a phenoxyphenyl group; a benzyloxyphenyl group;
as well as their pharmaceutically acceptable salts, their hydrates, their hydrated salts, the polymorphic crystalline structures and the tautomeric forms of these compounds; and their optical isomers, the racemic mixtures of said isomers and the corresponding diastereoisomers;
with the exception of compounds in which A represents a straight or branched alkenyl group containing up to 8 carbon atoms and X = -CH₂-.

2. Compounds corresponding to the general formula: 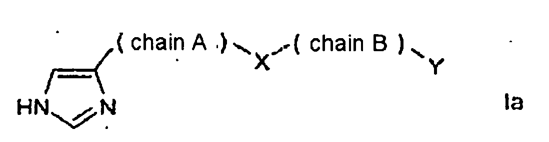 or 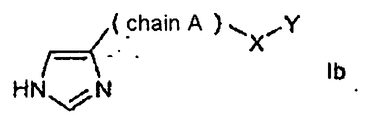 in which:
- chain A represents a straight or branched alkylene group containing from 1 to 8 carbon atoms; a straight or branched alkenyl group containing up to 8 carbon atoms; a straight or branched alkynyl group containing up to 4 carbon atoms;
- the group X represents -O-; -OCONH-, -OCON(alkene)-, -OCSNH- ; -CH₂-;
- chain B represents a straight or branched alkylene group containing from 1 to 8 carbon atoms; an alkenyl group containing up to 8 carbon atoms; an alkynyl group containing up to 8 carbon atoms; a group (CH₂)ₙO and n is an integer which can vary from 0 to 4;
- the group Y represents a phenyl group mono- or poly-substituted by one or more identical or different substituents selected from -OCF₃, CHO, SO₂N(alkyl)₂, SO₂N(CH₃)₂, S(alkyl), S(aryl), SCH₂ (phenyl), a straight or branched alkene containing up to 8 carbon atoms, a straight or branched alkyne containing up to 8 carbon atoms and optionally substituted by a trialkylsilyl radical, O(aryl), -CH₂CN, an aldehyde, a sulfone, an acetal, an alcohol, -CH=CH-CHO, -C(alkyl)=N-OH, -C(alkyl)=NO(alkyl), -CH=NOH, -CH=NO(alkyl), -C(alkyl)=NH-NH-CONH₂, a group -O-phenyl-OCH₂(phenyl), -C(cycloalkyl)=NOH, -C(cycloalkyl)-N-O(alkyl), an optionally substituted heterocycle; a phenyl ring attached to a non-aromatic carbocycle or a heterocycle carrying a ketone function; a straight or branched alkyne containing up to 8 carbon atoms and preferably up to 5 carbon atoms; a linear or branched, mono- or poly-phenylic alkyl, wherein the phenyl groups are either unsubstituted or mono- or poly-substituted; a phenyl alkyl ketone wherein the alkyl group is branched or unbranched, or cyclic; a substituted or unsubstituted phenyl alcohol, the alcohol unit of which is straight or branched or cyclic; a straight or branched alkene containing up to 8 carbon atoms; a phenylcycloalkyl group; a fluorenyl group; an indanyl group; a diphenyl group; a phenoxyphenyl group; a benzyloxyphenyl group;
as well as their pharmaceutically acceptable salts, their hydrates, their hydrated salts, the polymorphic crystalline structures and the tautomeric forms of these compounds; and their optical isomers, the racemic mixtures of said isomers and the corresponding diastereoisomers.

3. Compounds according to claim 1 or 2, **characterised in that** X represents an oxygen.

4. Compounds according to any one of claims 1 to 3, **characterised in that** A represents -(CH₂)ₙ-, n varying from 1 to 4.

5. Compounds according to claim 4, **characterised in that** A represents -(CH₂)₃-.

6. Compounds according to any one of claims 1 to 5, **characterised in that** chain A is a group - (CH₂₎₃; X is a group O or OCONH; chain B is a group (CH₂)ₙ wherein n = 0, 2 or 3; and Y is a group -CH(phenyl)₂.

7. Compounds according to any one of claims 1 to 5, **characterised in that** chain A is a group -(CH₂)ₙ where n is an integer which can vary from 1 to 8, or CH₂CH(CH₃)-; X is a group O or OCONH; and Y represents a branched or straight, mono- or poly-phenolic alkyl.

8. Compounds according to any one of claims 1 to 7, **characterised in that** chain A is a group -(CH₂)ₙ wherein n = 2, 3 or 4; X is a group O or OCONH; and Y represents a group -CH(phenyl)_{2.}

9. Compounds according to claim 1, **characterised in that** they are selected from the group formed by:
- 3-(1H-imidazol-4-yl)propyl-N-(diphenylmethyl) carbamate;
- 3-(1H-imidazol-4-yl)propyl-N-(2,2-diphenylethyl) carbamate;
- 3-(1H-imidazol-4-yl)ethyl-N-(2,2-diphenylethyl) carbamate;
- 4-[3-(1,2,3,4-tetrahydronaphth-6-yloxy)propyl]-1H-imidazole;
- 4-[3-(indan-5-yloxy)propyl]-1H-imidazole;
- N,N-diallyl-3-(1H-imidazol-4-yl)propyl carbamate;
- 3-(1H-imidazol-4-yl)propyl-N-trans-(2-phenylcyclopropyl) carbamate;
- N-fluoren-9-yl-3-(1H-imidazol-4-yl)propyl carbamate;
- 3-(1H-imidazol-4-yl)propyl (pent-4-ynyl) ether;
- 3-(1H-imidazol-4-yl)propyl (diphenylmethyl) ether;
- ((4-fluorophenyl)phenylmethyl) 3-(1H-imidazol-4-yl)propyl ether;
- bis(4-fluorophenyl)methyl) 3-(1H-imidazol-4-yl) propyl ether;
- N-(3,3-diphenylpropyl)-3-(1H-imidazol-4-yl)propyl carbamate.

10. Compounds according to claim 1 to 2, **characterised in that** they are selected from the group formed by:
- 3-(1H-imidazol-4-yl)propyl-N-prop-2-enyl carbamate;
- N-(1,1-diphenylethyl)-3-(1H-imidazol-4-yl)propyl carbamate;
- 3-(1H-imidazol-4-yl)propyl prop-2-enyl ether;
- 3-(1H-imidazol-4-yl)propyl pent-4-enyl ether;
- 3-(1H-imidazol-4-yl)propyl prop-2-ynyl ether;
- 1-(4-fluorophenyl)-4-(3-(1H-imidazol-4-yl)-propyloxy)-butan-1-one;
- 5-(3-(1H-imidazol-4-yl)propyloxy)indan-1-one;
- 3,4-dihydro-6-(3-(1H-imidazol-4-yl)propyloxy)-2H-naphthalen-1-one.

11. Pharmaceutical composition comprising a therapeutically effective amount of a compound according to any one of the preceding claims in a pharmaceutically acceptable carrier.

12. Medicament acting as a histamine H₃ receptor agonist, partial agonist or antagonist, **characterised in that** it comprises a compound according to any one of claims 1 to 10.

13. Medicament according to claim 12 acting as a histamine H₃ receptor agonist or partial agonist, for the treatment of asthma, bronchitis, rhinitis, tracheitis, myocardial dysfunction and infarct, gastric or duodenal ulcers, ulcerative colitis, Crohn's disease, irritable bowel syndrome, cystitis, metritis, urinary and faecal incontinence, urticaria, itching, arthritis, conjunctivitis, premenstrual syndrome.

14. Medicament according to claim 12, **characterised in that** it is administrable by the general or local route, especially in the form of an ointment, a collyrium or a device for transcutaneous or transdermal absorption.

15. Medicament according to claim 12, **characterised in that**, especially when taken orally, the unit doses are from 0.03 to 3 mg/kg in humans.

16. Medicament according to claim 12 acting as a histamine H₃ receptor antagonist, which medicament is especially to exert a psychotropic activity or an activity activating awakening, attention, memory, mood, vigilance or learning ability, or to treat depressive or asthenic states and cognitive, especially psychotic, disorders.

17. Medicament according to claim 12 acting as a histamine H₃ receptor antagonist, for the treatment of Alzheimer's disease.

18. Medicament according to claim 12 acting as a histamine H₃ receptor antagonist, for the treatment of balance disorders and vertigo.

19. Compound according to any one of claims 1 to 10 as a histamine H₃ receptor agonist or partial agonist, for exerting a sedative, tranquillising, anti-stress or anti-migraine activity and for treating psychosomatic disorders, respiratory, allergic, rheumatic or inflammatory disorders of the eye, of the urogenital system, of the digestive tract, of the skin, of the respiratory system or of the bronchi.

## Patentansprüche

1. Verbindungen mit der allgemeinen Formel: 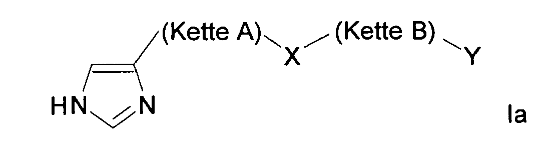 oder 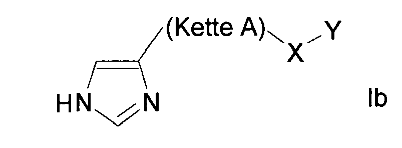 wobei
• die Kette A eine verzweigte oder unverzweigte Alkylgruppe umfassend 1 bis 8 Kohlenstoffatome; eine verzweigte oder unverzweigte Alkenylgruppe umfassend bis zu 8 Kohlenstoffatomen; eine verzweigte oder unverzweigte Alkinylgruppe umfassend bis zu 4 Kohlenstoffatomen darstellt;
• die Gruppe X -O-, -OCONH-, -OCON(Alken)-, -OCSNH-, -CH₂- darstellt;
• die Kette B eine verzweigte oder unverzweigte Alkylgruppe umfassend 1 bis 8 Kohlenstoffatome; eine Alkenylgruppe beinhaltend bis zu 8 Kohlenstoffatomen; eine Alkinylgruppe beinhaltend bis zu 8 Kohlenstoffatome; eine (CH₂)ₙO Gruppe und n ist eine ganze Zahl die zwischen 0 und 4 variieren kann, darstellt;
• die Gruppe Y eine Phenylgruppe darstellt, die einfach oder mehrfach mit einem oder mehreren, identischen oder unterschiedlichen, Substituenten substituiert ist, ausgewählt aus -OCF₃, CHO, SO₂N(alkyl)₂, SO₂N(CH₃)₂, S(alkyl), S(aryl), SCH₂(phenyl), ein verzweigtes oder unverzweigtes Alken umfassend bis zu 8 Kohlenstoffatome, ein verzweigtes oder unverzweigtes Alkin umfassend bis zu 8 Kohlenstoffatome, wahlfrei substituiert durch ein Radikal trialkylsilyl, O(Aryl), -CH₂CN, ein Aldehyd, ein Sulfon, ein Acetal, einen Alkohol, -CH=CH-CHO, -C(alkyl)=N-OH, -C(alkyl)=N-O(alkyl), -CH=NOH, -CH=NO(alkyl), -C(alkyl)=NH-NH-CONH₂, eine -O-phenyl-OCH₂(phenyl) Gruppe, -C(cycloalkyl)=NOH, -C(cycloalkyl)=N-O(alkyl), ein heterogene Ringverbindung wahlfrei substituiert; ein Phenylring gebunden an einen nichtaromatischen Kohlenstoffring oder eine heterogene Ringverbindung mit einer funktionellen Ketogruppe; ein unverzweigtes oder verzweigtes Alkin umfassend bis zu 8 Kohlenstoffatomen und bevorzugt bis 5 Kohlenstoffatome; ein lineares oder verzweigtes Alkyl, mono oder mehrfach phenylisch worin die phenylischen Gruppen entweder nicht substituiert, oder mono oder mehrfach substituiert sind; ein Phenylalkylketon worin die Alkylgruppe verzweigt, nicht verzweigt oder ringförmig ist; ein substituierter oder nicht-substituierter Phenyalkohol deren Grund-Alkohol verzweigt, unverzweigt oder ringförmig ist; ein verzweigtes oder unverzweigtes Alken umfassend bis zu 8 Kohlenstoffatome; eine Phenylcycloalkylgruppe, eine Fluorenylgruppe, eine Indanylgruppe, eine Phenolgruppe, eine Diphenylgruppe, eine Phenoxyphenylgruppe; eine Benzyloxyphenylgruppe;
sowie ihre pharmazeutisch akzeptablen Salze, ihre Hydrate, ihre Hydratsalze, die kristallinen polymorphen Strukturen und die tautomeren Formen von diesen Verbindungen; und ihre optischen Isomeren, die racemischen Gemische besagter Isomere und die entsprechenden Diastereoisomeren,
mit Ausnahme der Verbindungen für welche A eine verzweigte oder unverzweigte Alkinylgruppe umfassend bis zu 8 Kohlenstoffatomen und X = -CH₂- darstellt.

2. Verbindungen mit der allgemeinen Formel 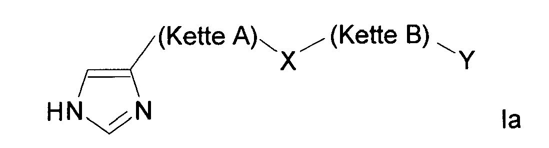 oder 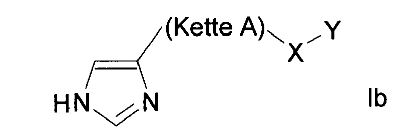 in welcher:
• die Kette A eine verzweigte oder unverzweigte Alkylgruppe umfassend 1 bis 8 Kohlenstoffatome; eine verzweigte oder unverzweigte Alkenylgruppe umfassend bis zu 8 Kohlenstoffatomen; eine verzweigte oder unverzweigte Alkinylgruppe umfassend bis zu 4 Kohlenstoffatomen darstellt;
• die Gruppe X -O-, -OCONH-, -OCON(Alken)-, -OCSNH-, -CH₂- darstellt;
• die Kette B eine verzweigte oder unverzweigte Alkylgruppe umfassend 1 bis 8 Kohlenstoffatome; eine Alkenylgruppe beinhaltend bis zu 8 Kohlenstoffatome; eine Alkinylgruppe beinhaltend bis zu 8 Kohlenstoffatome; eine (CH₂)ₙO Gruppe und n ist eine ganze Zahl die zwischen 0 und 4 variieren kann, darstellt;
• die Gruppe Y eine Phenylgruppe darstellt, die einfach oder mehrfach, mit einem oder mehreren, identischen oder unterschiedlichen, Substituenten substituiert ist, ausgewählt aus -OCF₃, CHO, SO₂N(alkyl)₂, SO₂N(CH₃)₂, S(alkyl), S(aryl), SCH₂(phenyl), ein verzweigtes oder unverzweigtes Alken umfassend bis zu 8 Kohlenstoffatome, ein verzweigtes oder unverzweigtes Alkin umfassend bis zu 8 Kohlenstoffatome, wahlfrei substituiert durch ein Radikal trialkylsilyl, O(Aryl), -CH₂CN, ein Aldehyd, ein Sulfon, ein Acetal, einen Alkohol, -CH=CH-CHO, -C(alkyl)=N-OH, -C(alkyl)=N-O(alkyl), -CH=NOH, -CH=NO(alkyl), -C(alkyl)=NH-NH-CONH₂, eine -O-phenyl-OCH₂(phenyl) Gruppe, -C(cycloalkyl)=NOH, -C(cycloalkyl)=N-O(alkyl), eine heterogene Ringverbindung wahlfrei substituiert; ein Phenylring gebunden an einen nichtaromatischen Kohlenstoffring oder eine heterogene Ringverbindung mit einer funktionellen Ketogruppe; ein unverzweigtes oder verzweigtes Alkin umfassend bis zu 8 Kohlenstoffatomen und bevorzugt bis 5 Kohlenstoffatome; ein lineares oder verzweigtes Alkyl, mono oder mehrfach phenylisch worin die phenylischen Gruppen entweder nicht substituiert, oder mono oder mehrfach substituiert sind; ein Phenylalkylketon worin die Alkylgruppe verzweigt, nicht verzweigt oder ringförmig ist; ein substituierter oder nicht-substituierter Phenyalkohol deren Grund-Alkohol verzweigt, unverzweigt oder ringförmig ist; ein verzweigtes oder unverzweigtes Alken umfassend bis zu 8 Kohlenstoffatome; eine Phenylcycloalkylgruppe, eine Fluorenylgruppe, eine Indanylgruppe, eine Phenolgruppe, eine Diphenylgruppe, eine Phenoxyphenylgruppe; eine Benzyloxyphenylgruppe;
sowie ihre pharmazeutisch akzeptablen Salze, ihre Hydrate, ihre Hydratsalze, die kristallinen polymorphen Strukturen und die tautomeren Formen von diesen Verbindungen; und ihre optischen Isomeren, die racemischen Gemische besagter Isomere und die entsprechenden Diastereoisomeren.

3. Verbindungen gemäß Anspruch 1 oder 2, **dadurch gekennzeichnet, dass** X einen Sauerstoff darstellt.

4. Verbindungen gemäß einen der Ansprüche 1 bis 3, **dadurch gekennzeichnet dass** A -(CH₂)ₙ- darstellt, n variiert von 1 bis 4.

5. Verbindungen gemäß Anspruch 4, **dadurch gekennzeichnet, dass** A -(CH₂)₃- darstellt.

6. Verbindungen gemäß einen der Ansprüche 1 bis 5, **dadurch gekennzeichnet, dass** die Kette A eine -(CH₂)₃ Gruppe ist; X eine O oder OCONH Gruppe ist; die Kette B eine (CH₂)ₙ Gruppe ist, wobei n = 0, 2 oder 3; und Y eine -CH(phenyl)₂ Gruppe ist.

7. Verbindungen gemäß einen der Ansprüche 1 bis 5, **dadurch gekennzeichnet, dass** die Kette A eine -(CH₂)ₙ Gruppe, wobei n eine ganze Zahl variierend zwischen 1 und 8 ist, oder CH₂CH(CH₃) ist; X eine O oder OCONH Gruppe ist; und Y ein verzweigtes oder unverzweigtes, mono oder mehrfach phenolisches Alkan ist.

8. Verbindungen gemäß einen der Ansprüche 1 bis 7, **dadurch gekennzeichnet, dass** die Kette A eine -(CH₂)ₙ- Gruppe ist, wobei n = 2, 3 oder 4; X eine O oder OCONH Gruppe ist; und Y eine -CH(phenyl)₂ Gruppe darstellt.

9. Verbindungen gemäß Anspruch 1, **dadurch gekennzeichnet, dass** sie aus der Gruppe gebildet aus
• 3-(1H-Imidazol-4-yl)propyl-N-(diphenylmethyl)carbamat;
• 3-(1H-Imidazol-4-yl)propyl-N-(2,2-diphenylethyl)carbamat;
• 3-(1H-Imidazol-4-yl)ethyl-N-(2,2-diphenylethyl)carbamat;
• 4-[3-(1,2,3,4-tetrahydronapht-6-yloxy)propyl]-1H-imidazol;
• 4-[3-(Indan-5-yloxy)propyl]-1H-imidazole;
• N,N-Diallyl-3-(1H-imidazol-4-yl)propyl carbamat;
• 3-(1H-imidazol-4-yl)propyl-N-trans-(2-phenylcyclopropyl)carbamat;
• N-Fluoren-9-yl-3-(1 H-imidazol-4-yl)propyl carbamat;
• 3-(1H-imidazol-4-yl)propyl-(pent-4-inyl)ether;
• 3-(1H-imidazol-4-yl)propyl-(diphenylmethyl)ether;
• ((4-Fluorophenyl)phenylmethyl)-3-(1H-imidazol-4-yl)propyl ether;
• Bis(4-fluorophenyl)methyl)-3-(1H-imidazol-4-yl)propyl ether;
• N-(3,3-diphenylpropyl)-3-(1H-imidazol-4-yl)propyl carbamat
ausgewählt sind.

10. Verbindungen gemäß einem der Ansprüche 1 oder 2, **dadurch gekennzeichnet, dass** sie aus der Gruppe gebildet aus
• 3-(1H-Imidazol-4-yl)propyl-N-prop-2-enyl carbamat;
• N-(1,1-diphenylethyl)-3-(1H-imidazol-4-yl) propyl carbamat;
• 3-(1H-Imidazol-4-yl)propyl-prop-2-enyl ether;
• 3-(1H-Imidazol-4-yl)propyl-pent-4-enyl ether;
• 3-(1H-Imidazol-4-yl)propyl-prop-2-inyl ether;
• 1-(4-Fluorophenyl)-4-(3-(1H-imidazol-4-yl)propyloxy)-butan-1-on;
• 5-(3-(1H-imidazol-4-yl)propyloxy)indan-1-on;
• 3,4-dihydro-6-(3-(1H-imidazol-4-yl)propyloxy)-2H-naphtalen-1-on;
ausgewählt sind.

11. Pharmazeutische Zusammensetzung umfassend eine pharmazeutisch wirksame Quantität der Verbindung gemäß einem der vorstehenden Ansprüche in einer pharmazeutisch akzeptablen Grundmasse.

12. Medikament wirkend als Agonist, teilweiser Agonist oder Antagonist der Histamin H3 Rezeptoren, **dadurch gekennzeichnet, dass** es eine Verbindung gemäß einem der Ansprüche 1 bis 10 umfasst.

13. Medikament gemäß Anspruch 12 als Agonist oder teilweiser Agonist der Histamin H3 Rezeptoren, bestimmt zur Behandlung von Asthma, Bronchien, Nasenschleimhautentzündungen, Luftröhrenentzündungen, Funktionsstörungen und Infarkte des Herzens, Magen- oder Zwölffingerdarmgeschwüren, geschwürbildender Kolitis, der Chron Krankheit, des Syndroms des reizbaren Grimmdarms, Blasenentzündungen, Gebärmutterentzündungen, Harn- und Stuhlinkontinenzen, Nesselsucht, Juckreiz, Arthritis, Bindehautentzündung, des prämenstruellen Syndroms.

14. Medikament gemäß Anspruch 12, **dadurch gekennzeichnet, dass** es durch einen allgemeinen oder lokalen Weg verabreichbar ist, insbesondere salbenförmig, in Form von Augentropfen oder in Form von Mitteln für eine transkutane Absorption oder Absorption durch die Haut.

15. Medikament gemäß Anspruch 12, **dadurch gekennzeichnet, dass**, insbesondere bei einer oralen Einnahme, die einheitlichen Dosen einschließlich zwischen 0.03 und 3 mg/kg beim Menschen sind.

16. Medikament gemäß Anspruch 12, wirkend als Antagonist der Histamin H3 Rezeptoren, insbesondere bestimmt zum Ausüben einer psychotropen Wirkung, das Erwachen, die Aufmerksamkeit, die Erinnerung, die Stimmung, die Wachsamkeit, die Lernkapazität aktivierend, oder zum Behandeln depressiver Zustände, asthenischer Zustände, kognitiver Beschwerden, insbesondere psychotischer.

17. Medikament gemäß Anspruch 12, wirkend als Antagonist der Histamin H3 Rezeptoren, bestimmt zur Behandlung der Alzheimer Krankheit.

18. Medikament gemäß Anspruch 12, wirkend als Antagonist der Histamin H3 Rezeptoren, bestimmt zur Behandlung von Gleichgewichtsstörungen und Schwindelanfällen.

19. Verbindung gemäß einem der vorstehenden Ansprüche 1 bis 10 als Agonist oder teilweiser Agonist der Histamin H3 Rezeptoren, zum Ausüben einer sedativen, beruhigenden, anti-stress, anti-migränen Wirkung, und zum Behandeln von psychosomatischen Beschwerden, Atemerkrankungen, allergischen und rheumatischen Erkrankungen, oder Entzündungen des Auges, des Urogenitalorgans, des Verdauungstraktes, der Haut, des Atmungssystem oder der Bronchien.
